(19) Europäisches Patentamt
European Patent Office
Office européen des brevets

(11) EP 2 450 458 B1

(12) EUROPEAN PATENT SPECIFICATION

(45) Date of publication and mention of the grant of the patent:
**02.03.2016 Bulletin 2016/09**

(51) Int Cl.:
***C12R 1/01*** *(2006.01)* ***C12N 9/14*** *(2006.01)*

(21) Application number: **11181265.7**

(22) Date of filing: **14.09.2011**

(54) **Novel microorganisms producing a thermostable lipase and their use**

Neue Mikroorganismen zur Herstellung einer wärmestabilen Lipase und ihre Verwendung

Nouveaux microorganismes produisant une lipase thermostable et leur utilisation

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.09.2010 MY 1070066**

(43) Date of publication of application:
**09.05.2012 Bulletin 2012/19**

(73) Proprietor: **Universiti Putra Malaysia (UPM)**
**Selangor (MY)**

(72) Inventors:
- **Rahman, Raja Noor Zaliha Raja.Abd.**
  **Selangor (MY)**
- **Salleh, Abu Bakar**
  **Selangor (MY)**
- **Ebrahimpour, Afshin**
  **Selangor (MY)**
- **Basri, Mahiran**
  **Selangor (MY)**

(74) Representative: **Krauss, Jan**
**Boehmert & Boehmert**
**Anwaltspartnerschaft mbB**
**Patentanwälte Rechtsanwälte**
**Pettenkoferstrasse 20-22**
**80336 München (DE)**

(56) References cited:


- **DATABASE EMBL [Online] 7 November 2006 (2006-11-07), "Geobacillus stearothermophilus strain ARM1 thermostable lipase precursor, gene, complete cds.", XP002671150, retrieved from EBI accession no. EMBL:EF042975 Database accession no. EF042975**
- **EBRAHIMPOUR AFSHIN ET AL: "High level expression and characterization of a novel thermostable, organic solvent tolerant, 1,3-regioselective lipase from Geobacillus sp. strain ARM", BIORESOURCE TECHNOLOGY, vol. 102, no. 13, July 2011 (2011-07), pages 6972-6981, XP002671151,**
- **LI H ET AL: "Characterization of thermostable lipase from thermophilic Geobacillus sp. TW1", PROTEIN EXPRESSION AND PURIFICATION, ACADEMIC PRESS, SAN DIEGO, CA, vol. 42, no. 1, 1 July 2005 (2005-07-01), pages 153-159, XP004918459, ISSN: 1046-5928, DOI: 10.1016/J.PEP.2005.03.011**
- **SOLIMAN ET AL: "Molecular cloning and characterization of thermostable esterase and lipase from Geobacillus thermoleovorans YN isolated from desert soil in Egypt", PROCESS BIOCHEMISTRY, ELSEVIER, NL, vol. 42, no. 7, 1 July 2007 (2007-07-01), pages 1090-1100, XP022138699, ISSN: 1359-5113, DOI: 10.1016/J.PROCBIO.2007.05.005**
- **MALIHE MASOMIAN ET AL: "A unique thermostable and organic solvent tolerant lipase from newly isolated Aneurinibacillus thermoaerophilus strain HZ: physical factor studies", WORLD JOURNAL OF MICROBIOLOGY AND BIOTECHNOLOGY, KLUWER ACADEMIC PUBLISHERS, DO, vol. 26, no. 9, 23 February 2010 (2010-02-23), pages 1693-1701, XP019832775, ISSN: 1573-0972**


Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

(Cont. next page)

- **RAHMAN RAJA NOOR ZALIHA RAJA ABD ET AL: "Geobacillus zalihae sp. nov., a thermophilic lipolytic bacterium isolated from palm oil mill effluent in Malaysia", BMC MICROBIOLOGY, BIOMED CENTRAL, LONDON, GB, vol. 7, no. 1, 10 August 2007 (2007-08-10) , page 77, XP021028197, ISSN: 1471-2180, DOI: 10.1186/1471-2180-7-77**
- **EBRAHIMPOUR AFSHIN ET AL: "Lipase production and growth modeling of a novel thermophilic bacterium: Aneurinibacillus thermoaerophilus strain AFNA", ELECTRONIC JOURNAL OF BIOTECHNOLOGY, vol. 14, no. 4, July 2011 (2011-07), XP002675459,**
- **DATABASE EMBL [Online] 14 November 2006 (2006-11-14), "Aneurinibacillus thermoaerophilus strain AFNA 1 16S ribosomal RNA gene, partial sequence.", XP002675460, retrieved from EBI accession no. EMBL:EF032876 Database accession no. EF032876**

SEQUENCE LISTING

<110> Universiti Putra Malaysia

<120> NOVEL MICROORGANISMS PRODUCING A THERMOSTABLE LIPASE AND THEIR USE

<130> FB25397

<150> MY2010700066
<151> 2010-09-14

<160> 4

<170> PatentIn version 3.5

<210> 1
<211> 1563
<212> DNA
<213> Geobacillus sp.

<400> 1

```
ttcttttgga gagtttgatc ctggctcagg acgaacgctg gcggcgtgcc taatacatgc    60
aagtcgagcg gactggattg gagcttgctc tgattcggtc agcggcggac gggtgagtaa   120
cacgtgggca acctgcccgc aagaccggga taactccggg aaaccggagc taataccgga   180
taacaccgaa gaccgcatgg tctttggttg aaaggcggcc tttggctgtc acttgcggat   240
gggcccgcgg cgcattagct agttggtgag gtaacggctc accaaggcga cgatgcgtag   300
ccggcctgag agggtgaccg gccacactgg gactgagaca cggcccagac tcctacggga   360
ggcagcagta gggaatcttc cgcaatgggc gaaagcctga cggagcgacg ccgcgtgagc   420
gaagaaggcc ttcgggtcgt aaagctctgt tgtgagggac gaaggagcgc cgttcgaaga   480
gggcggcgcg gtgacggtac ctcacgagga agccccggct aactacgtgc cagcagccgc   540
ggtaatacgt aggggcgag cgttgtccgg aattattggg cgtaaagcgc gcgcaggcgg    600
ttccttaagt ctgatgtgaa agcccacggc tcaaccgtgg agggtcattg gaaactgggg   660
gacttgagtg caggagagga gagcggaatt cccacgtgta gcggtgaaat gcgtagagat   720
gtggaggaac accagtggcg aaggcggctc tctggcctgc aactgacgct gaggcgcgaa   780
agcgtgggga gcaaacagga ttagataccc tggtagtcca cgccgtaaac gatgagtgct   840
aagtgttaga ggggtcacac cctttagtgc tgcagctaac gcgataagca ctccgcctgg   900
ggagtacggc cgcaaggctg aaactcaaag gaattgacgg gggcccgcac aagcggtgga   960
gcatgtggtt taattcgaag caacgcgaag aaccttacca ggtcttgaca tcccctgaca  1020
acccaagaga ttgggcgttc cccttcgggg gggacagggt gacaggtggt gcatggttgt  1080
cgtcagctcg tgtcgtgaga tgttgggtta agtcccgcaa cgagcgcaac cctcgcctct  1140

agttgccagc attcggttgg gcactctaga gggactgccg gcgacaagtc ggaggaaggt  1200
ggggatgacg tcaaatcatc atgcccctta tgacctgggc tacacacgtg ctacaatggg  1260
cggtacaaag ggctgcgaac ccgcgagggg gagcgaatcc caaaaagccg ctctcagttc  1320
ggattgcagg ctgcaactcg cctgcatgaa gccggaatcg ctagtaatcg cggatcagca  1380
tgccgcggtg aatacgttcc cgggccttgt acacaccgcc cgtcacacca cgagagcttg  1440
caacacccga agtcggtgag gcaacccgca agggagccag ccgccgaagg tggggcaagt  1500
gattggggtg aagtcgtaac aaggtagccg taccggaagg tgcggctgga tcacctcctt  1560
tct                                                                1563
```

<210> 2
<211> 1504
<212> DNA
<213> Aneurinibacillus thermoaerophilus

<400> 2

```
gagagtttga tcctggctca ggacgaacgc tggcggcgtg cctaatacat gcaagtcgag      60
cgaaccgatg gagtgcttgc attcctgagg ttagcggcgg acgggtgagt aacacgtagg     120
caacctgcct gtacgaccgg gataactccg ggaaaccgga gctaataccg gataggatgc     180
cgaaccgcat ggttcggcat ggaaaggcct ttgagccgcg tacagatggg cctgcggcgc     240
attagctagt tggtggggta acggcctacc aaggcgacga tgcgtagccg acctgagagg     300
gtgaacggcc acactgggac tgagacacgg cccagactcc tacgggaggc agcagtaggg     360
aatcttccgc aatggacgaa agtctgacgg agcaacgccg cgtgagtgag gaaggtcttc     420
ggatcgtaaa actctgttgt cagggaagaa ccgccgggat gacctcccgg tctgacggta     480
cctgacgaga aagccccggc taactacgtg ccagcagccg cggtaatacg taggggggcaa    540
gcgttgtccg gaattattgg gcgtaaagcg cgcgcaggcg gcttcttaag tcaggtgtga     600
aagcccacgg ctcaaccgtg gagggccatc tgaaactggg gagcttgagt gcaggagagg     660
agagcggaat tccacgtgta gcggtgaaat gcgtagagat gtggaggaac accagtggcg     720
aaggcggctc tctggcctgt aactgacgct gaggcgcgaa agcgtgggga gcaaacagga     780
ttagataccc tggtagtcca cgccgtaaac gatgagtgct aggtgttggg gagtccacct     840
cctcagtgcc gcagctaacg caataagcac tccgcctggg gagtacggcc gcaaggctga     900
aactcaaagg aattgacggg gacccgcaca agcggtggag catgtggttt aattcgaagc     960
aacgcgaaga accttaccag ggcttgacat cccgctgacc cctccagaga tggaggcttc    1020
cttcgggaca gcggtgacag gtggtgcatg gttgtcgtca gctcgtgtcg tgagatgttg    1080
ggttaagtcc cgcaacgagc gcaacccttg tcctttgttg ccagcattca gttgggcact    1140
ctaaggagac tgccgtcgac aagacggagg aaggtgggga tgacgtcaaa tcatcatgcc    1200
ccttatgtcc tgggctacac acgtgctaca atggacggta caacgggcgt gccaacccgc    1260
gagggtgagc caatccctaa aaaccgttct cagttcggat tgcaggctgc aactcgcctg    1320
catgaagccg gaatcgctag taatcgcgga tcagcatgcc gcggtgaata cgttcccggg    1380
tcttgtacac accgcccgtc acaccacgag agtttgcaac acccgaagtc ggtgaggtaa    1440
ccttctggag ccagccgccg aaggtggggc agatgattgg ggtgaagtcg taacaaggta    1500
tccg                                                                  1504
```

<210> 3

<211> 1588
<212> DNA
<213> Geobacillus

<400> 3

```
gggttggaag gggccttggg tcttatgtat aaatattctg gtaattcaga atgaaaagat     60
ttcggcctat tgaaggaaag gagagggaga acatgatgaa atgctgtcgg cgtgttgccc    120
ttgtgttgct cggattatgg tttgtgttct gcatatctgt tctgggaggg cgagctgaag    180
cggcggcttc gcgagccaac gatgcgccga ttgtacttct ccacgggttt accggctggg    240
gaagagaaga aatgtttggg ttcaagtact ggggcggcgt gcgcggcgat atcgaacaat    300
ggctgaacga caacggttat cgaacttata cgctggcggt cggcccgctc tcgagcaact    360
gggatcgggc gtgcgaagcg tatgcccagc ttgtcggcgg gacggtcgat tatggggcag    420
cccatgcggc aaagcacggc catgcgcggt ttggccgcac ctatcccggc ctgttgccgg    480
aattgaaaag gggcggtcgc atccatatca tcgcccacag ccaaggaggg cagacggccc    540
gcatgcttgt atcgctccta gagaatggaa gccaagaaga gcgggagtac gctaaggcgc    600
ataacgtgtc tttgtcgccg ttgtttgaag gcggacatca ttttgtgttg agagtgacaa    660
ccattgccac ccctcatgac gggacgacgc ttgtcaacat ggttgatttc accgatcgct    720
tttttgactt gcaaaaagcg gtgttgaaag cagcggctgt cgccagcaat gtgccgtaca    780
caagtcaagt atacgatttt aagctcgacc aatggggact gcgccgccag ccaggtgaat    840
cgtttgacca atattttgaa cggctcaaac ggtcccctgt ttggacgtcg acagataccg    900
ctcgctatga tttatccgtt cccggggctg agaagttgaa tcaatgggtg aaagcaagcc    960
cgaatacgta ttatttgagc tttgccacag aacggacgta tcgcggagcg ctcacaggca   1020
actattatcc cgaactcgga atgaatgcat tcagcgccgt cgtatgcgcc ccgtttctcg   1080
gttcgtaccg caatgcgacg ctcggcattg acgaccgctg gcttgagaac gatggcatcg   1140

tcaatacgtt ttcgatgaac ggaccaaagc gcggatcgac cgatcggatc gtgccgtatg   1200
acgggacaat aaaaaaaggg gtttggaatg atatgggaac gtacaatgtc gatcatttgg   1260
aagtcatcgg cgttgacccg aatccgttgt ttgatatccg tgccttttat ttgcgccttg   1320
ccgagcagtt ggcgagcttg caaccttaaa actagtattt tgcgaaaaaa gccatctcga   1380
tcggatggcg ctttttttcg tgaacattca tgcgccttgg ttggtcattt tcatgcccgg   1440
gtggagaaca aacgctgttc atgaactttt ctgtgaatat gcacgttcct ttaactttca   1500
gtttcatgtc ggggtgaaaa acgaaaaatg gtttatggat gttttggaga acaagcagct   1560
gattggtatg acggccgcgg aatcccga                                      1588
```

<210> 4
<211> 418
<212> PRT
<213> Geobacillus

<400> 4

```
Met Met Lys Cys Cys Arg Arg Val Ala Leu Val Leu Leu Gly Leu Trp
1               5                   10                  15

Phe Val Phe Cys Ile Ser Val Leu Gly Gly Arg Ala Glu Ala Ala Ala
            20                  25                  30

Ser Arg Ala Asn Asp Ala Pro Ile Val Leu Leu His Gly Phe Thr Gly
        35                  40                  45

Trp Gly Arg Glu Glu Met Phe Gly Phe Lys Tyr Trp Gly Gly Val Arg
    50                  55                  60

Gly Asp Ile Glu Gln Trp Leu Asn Asp Asn Gly Tyr Arg Thr Tyr Thr
65                  70                  75                  80

Leu Ala Val Gly Pro Leu Ser Ser Asn Trp Asp Arg Ala Cys Glu Ala
                85                  90                  95

Tyr Ala Gln Leu Val Gly Gly Thr Val Asp Tyr Gly Ala Ala His Ala
            100                 105                 110

Ala Lys His Gly His Ala Arg Phe Gly Arg Thr Tyr Pro Gly Leu Leu
        115                 120                 125

Pro Glu Leu Lys Arg Gly Gly Arg Ile His Ile Ile Ala His Ser Gln
    130                 135                 140
```

```
Gly Gly Gln Thr Ala Arg Met Leu Val Ser Leu Leu Glu Asn Gly Ser
145                 150                 155                 160

Gln Glu Glu Arg Glu Tyr Ala Lys Ala His Asn Val Ser Leu Ser Pro
                165                 170                 175

Leu Phe Glu Gly Gly His His Phe Val Leu Arg Val Thr Thr Ile Ala
            180                 185                 190

Thr Pro His Asp Gly Thr Thr Leu Val Asn Met Val Asp Phe Thr Asp
        195                 200                 205

Arg Phe Phe Asp Leu Gln Lys Ala Val Leu Lys Ala Ala Ala Val Ala
    210                 215                 220

Ser Asn Val Pro Tyr Thr Ser Gln Val Tyr Asp Phe Lys Leu Asp Gln
225                 230                 235                 240

Trp Gly Leu Arg Arg Gln Pro Gly Glu Ser Phe Asp Gln Tyr Phe Glu
                245                 250                 255

Arg Leu Lys Arg Ser Pro Val Trp Thr Ser Thr Asp Thr Ala Arg Tyr
            260                 265                 270

Asp Leu Ser Val Pro Gly Ala Glu Lys Leu Asn Gln Trp Val Lys Ala
        275                 280                 285

Ser Pro Asn Thr Tyr Tyr Leu Ser Phe Ala Thr Glu Arg Thr Tyr Arg
    290                 295                 300

Gly Ala Leu Thr Gly Asn Tyr Tyr Pro Glu Leu Gly Met Asn Ala Phe
305                 310                 315                 320

Ser Ala Val Val Cys Ala Pro Phe Leu Gly Ser Tyr Arg Asn Ala Thr
                325                 330                 335

Leu Gly Ile Asp Asp Arg Trp Leu Glu Asn Asp Gly Ile Val Asn Thr
            340                 345                 350

Phe Ser Met Asn Gly Pro Lys Arg Gly Ser Thr Asp Arg Ile Val Pro
        355                 360                 365

Tyr Asp Gly Thr Ile Lys Lys Gly Val Trp Asn Asp Met Gly Thr Tyr
    370                 375                 380
```

```
Asn Val Asp His Leu Glu Val Ile Gly Val Asp Pro Asn Pro Leu Phe
385                 390                 395                 400

Asp Ile Arg Ala Phe Tyr Leu Arg Leu Ala Glu Gln Leu Ala Ser Leu
                405                 410                 415

Gln Pro
```

**Description**

## FIELD OF INVENTION

The present invention relates to the field of enzyme technology and microbiology. The present invention is defined by the appended set of claims.

## BACKGROUND OF THE INVENTION

Microbial enzymes are often more useful than enzymes derived from plants or animals because of the great variety of catalytic activities available, the high yields possible, ease of genetic manipulation, regular supply due to absence of seasonal fluctuations and rapid growth of microorganisms on inexpensive media. Microbial enzymes are also more stable than their corresponding plant and animal enzymes and their production is more convenient (Sharma *et al,* 2001a; Hasan *et al.,* 2006).

Nowadays, lipases stand among the most important biocatalysts due to their biotechnological potential. They carry out novel reactions in both aqueous and non-aqueous media. Lipases are used to hydrolyze ester bonds of a variety of nonpolar substrates at high activity, regioselectivity, and stereoselectivity. Moreover, they are used to catalyze the reverse reaction in non-polar solvents. The reaction can be designed and optimized to get a broad range of novel products by changing substrate structure, solvent, additives, water activity, pressure, temperature, immobilization methods, and, as recombinant lipases become available, the biocatalyst itself (Sharma *et al.,* 2001a; Hasan *et al.,* 2006; Svendsen, 2004).

On the other hand, during the last decade, the determination of lipases three-dimensional structure has thrown light into their unique structure-function relationship, and lipases with improved properties are being produced by natural selection and protein engineering to further enhance usefulness of these enzymes. Thus, lipases are today the enzymes of choice for organic chemists, pharmacists, biophysicists, biochemical and process engineers, biotechnologists and microbiologists (Sharma *et al.,* 2001a).

Lipases have been purified from a large number of microbial (bacteria, fungi, yeasts, and actinomyces), plant and animal sources. Lipases isolated from different sources have a wide range of properties with respect to substrate specificity (chemo-, regio- and enantioselectivity), thermo- and solvent- stability, pH optimum, etc. (Sharma *et al.,* 2001a; Hasan *et al.,* 2006). Among lipases of different sources, extracellular microbial thermostable lipases are highly advantageous for biotechnological applications, since they can be produced at low cost and exhibit improved thermo- and solvent- stability and substrate specificity (Hasan *et al.,* 2006; Sharon *et al.,* 1998; Sugihara *et al.,* 1991 a and b).

The temperature stability of lipases has been regarded as the most important characteristic for use in industry. However, low stability is favorable for some purposes. For example, heat-labile enzymes can be easily inactivated by treatment for short periods at relatively low temperatures after being used for processing of food and other materials

Based on the previous research and inventions, Enzymes produced by (hyper)thermophiles are typically thermostable (i.e. resistant to irreversible inactivation at high temperatures) and thermophilic (i.e. optimally active at high temperatures) and are known as thermozymes (thermoenzymes) (Vieille *et al.,* 1996). Thermozymes are gaining wide industrial and biotechnological interest due to the fact that they are better suited for harsh industrial processes (Dandavate *et al.,* 2009; Littlechild *et al.,* 2007; Haki and Rakshit, 2003; Jaeger and Eggert, 2002; Sharma *et al.,* 2001b). Thermozymes offer following important advantages over mesozymes (mesophilic enzymes) (Li *et al.,* 2005; Haki and Rakshit, 2003; Vieille and Zeikus, 2001). They have a higher resistance to chemical denaturants; withstand higher substrate concentrations; reducing the risk of contamination by common mesophiles; often display higher reaction rates than mesozymes catalyzed reactions due to a decrease in viscosity and an increase in diffusion coefficient of substrates; as well as higher process yield due to increased solubility of substrates and products and favorable equilibrium displacement in endothermic reactions. In addition, thermozymes have become model systems to study: enzyme (thermo)stability and (thermo)activity mechanisms; enzyme evolution; protein structure-function relationships; and biocatalysis under extreme conditions.

Recently thermostable lipases from various microorganisms have been purified and characterized such as *Bacillus* spp. (Nawani and Kaur, 2007; Nawani *et al.,* 1998; Becker *et al.,* 1997; Llarch *et al.,* 1997; Handelsman and Shoham, 1994 and Sugihara *et al.,* 1991 a), *Bacillus coagulans* BTS-3 (Kumar *et al.,* 2005), *Bacillus* sp. RSJ-1 (Sharma *et al.,* 2002a), *Bacillus* sp. H-257 (Imamura and Kitaura, 2000), *Geobacillus thermoleovorans* (Abdel-Fattah and Gaballa, 2008), *Geobacillus* sp. TW1 (Li and Zhang, 2005), *Geobacillus zalihae* (Rahman *et al.,* 2007; Rahman *et al.,* 2005a; Leow *et al.,* 2004), *Geotrichum candidum* Y05 (Yan *et al.,* 2007), *Burkholderia cepacia* ATCC 25416 (Wang *et al.,* 2009), *Natronococcus* sp. strain TC6 (Boutaiba *et al.,* 2006) and *Alcaligenes* sp. (Wilson *et al.,* 2006).

The present invention responds specifically to the long-felt need heretofore unmet by the prior art, and especially with a view to overcoming the objective of the present invention which relates to the isolation of thermostable lipase producing bacteria, optimizing thermostable lipase production. Yet other objective of the present invention provides expressing lipase gene encoding thermostable lipase and characterizing purified recombinant thermostable lipase.

## SUMMARY OF INVENTION

The present invention discloses biologically pure cultures of *Geobacillus* sp. strain ARM isolated from soil contaminated with oil; whereby the *Geobacillus sp* strain ARM is deposited under the accession number NCIMB 41583 at The National Collection of Industrial, Marine and Food Bacteria (NCIMB), UK. Moreover, the *Geobacillus sp* strain ARM is capable to produce a novel thermostable enzyme isolated and characterized from *Geobacillus sp* strain ARM.

In addition, the present invention provides the use of *Geobacillus sp* strain ARM producing thermostable lipase, for industrial applications such as in food industry, oil processing, and production of surfactants, oil processing, detergents, pesticides, environmental management and leather industry. In particular the use of ARM lipase for industrial applications such as in food industry, oil processing, production of surfactants, oil processing, detergents, pesticides, environmental management and leather industry.

## BRIEF DESCRIPTION OF THE DRAWINGS

The accompanied drawings constitute part of this specification and include an exemplary or preferred embodiment of the invention, which may be embodied in various forms. It should be understood, however, the disclosed preferred embodiments are merely exemplary of the invention. Therefore, the figures disclosed herein are not to be interpreted as limiting, but merely as the basis for the claims and for teaching one skilled in the art of the invention

**Figure 1** shows Tributyrin diffusion agar method (TBA) and Rhodamine B agar plate assay (RBA) were used for isolation of lipase producing bacteria. Clear zone formations surrounding the colonies on TBA and orange fluorescent halos on RBA were considered as lipase production at 60°C after 24 h.

**Figure 2** shows Phase contrast (A), Gram staining (B), SEM (D) and **TEM** after **negative staining** (C) images of cells and flagella of strains (1) ARM and (2) AFNA.

**Figure 3** shows Phenotypic classification of aerobic spore-forming bacteria, Groups 4 (A) and 3 (B) where strains ARM and AFNA stood, respectively (Reva *et al.,* 2001).

**Figure 4** shows Two-dimensional TLC analysis of polar lipids; strain ARM (A) and strain AFNA (B). The chromatograms were developed from right to left with chloroform-methanol-water-28% aqueous ammonia (130:70:8:0.5) and then in the vertical direction with chloroform-acetone-methanol-acetic acid-water (100:40:20:20:10). DPG: Diphosphatidylglycerol; PG: Phosphattidylglycerol; PE: Phosphatidylethanolamine; PL: Phospholipid; AL: Aminolipid; PC: Phosphatidylcholine; PGL1-PGL3: Phosphoglycolipids.

**Figure 5** shows Dendrograms of fatty acids similarities (Euclidian distance) based on MIDI analysis summarize the relatedness of the whole-cell fatty acid compositions of strain ARM and *Bacillus stearothermophilus* spp. (A) as well as strain AFNA and *Aneurinibacillus thermoaerophilus* DSM 10154$^T$ (B).

**Figure 6** shows 16S rDNA of isolates ARM and AFNA. The PCR product was electrophoresed in 1% agarose gel with 1kb DNA ladder as the standard marker at 90 volt for

~ 40 min. The gel was stained with ethidium bromide (1 μg/ml) for 10 min and DNA was visualized under UV light. 1kb DNA ladder includes fragment sizes of 10000, 8000, 6000, 5000, 4000, 3500, 3000, 2500, 2000, 1500, 1000, 750, 500 and 250 bp.

**Figure 7** shows 16S rDNA sequences of isolates (A) ARM and (B) AFNA.

**Figure 8** shows Phylogenetic position based on neighbour-joining of the 16S rDNA sequences of the strains (A) ARM and (B) AFNA among other *Bacillus* spp.

**Figure 9 Lipase activities in different composition of production media.** Bacterial inoculum (2% v/v; $Ab_{600}$ ~ 0.5 of overnight culture in TSB) of each isolate was individually inoculated into 50 ml of different production media and incubated by agitation under 150 rpm, for 48 h at 60°C.

**Figure 10** shows three dimensional plots showing the effect of: (A) growth temperature, inoculum size; (B) agitation rate, medium volume; (C) initial pH, agitation rate; and (D) initial pH, incubation period, and their mutual effect on the lipase production by strain ARM Other variables are constant: growth temperature (52.3°C), medium volume (50 ml), inoculum size (1%), agitation rate (static condition), incubation period (24 h) and initial pH (5.8)

**Figure 11** shows three dimensional plots showing the effect of: (A) growth temperature, inoculum size; (B) agitation rate, medium volume; and (C) initial pH, incubation period, and their mutual effect on the lipase production by strain AFNA. Other variables are constant: growth temperature (52.7°C), medium volume (133.1 ml), inoculum size (2.9%), agitation rate (103 rpm), incubation period (48 h) and initial pH (6.9)

**Figure 12** shows importance of effective parameters on lipase production by strains (A) ARM and (B) AFNA.

**Figure 13** shows full length of amplified ARM lipase gene. The PCR product was electrophoresed in 1% agarose gel with 1kb DNA ladder as the standard marker at 90 volt for

~ 40 min. The gel was stained with ethidium bromide (1 μg/ml) for 10 min and DNA was visualized under UV light. 1kb DNA ladder includes fragment sizes of 10000, 8000, 6000, 5000, 4000, 3500, 3000, 2500, 2000, 1500, 1000, 750, 500 and 250 bp.

**Figure 14** shows nucleotide and amino acid sequences of the thermostable ARM lipase. The predicted promoter region (-10 and -35), ribosomal binding site (RBS), start and stop codones, signal peptide and stem loop (inverted repeat) have been mentioned. Conserved penta peptide (AHSQG) as well as catalytic triad amino acids **(S, D** and **H)** and lid amino acids sequence **(DFTDRFFDLQKAVLKAA)** have been highlighted.

**Figure 15** shows Phylogenetic position based on neighbour-joining of ARM lipase gene sequence among other thermostable lipase genes

**Figure 16** shows pairwise alignment of amino acid sequence of mature ARM lipase with thermostable lipases from *Geobacillus kaustophilus* HTA426, *Geobacillus stearothermophilus* (TW1) and *Geobacillus zalihae* (T1).

**Figure 17** shows positive transformed colony (A) and its sub culture (B) with clear surrounding zone on tributyrin agar plate. The transformed cells (50 μl) were spread on a prewarmed LB tributryrin agar plate containing 50 μg/ml ampicillin and 0.5% glucose (as selection plate) and incubated overnight at 37°C.

**Figure 18** shows effect of post induction time on recombinant ARM thermostable lipase gene expression in 200 ml of LB broth containing 50 μg/ml ampicillin, after induction by 1.0 mM inducer (IPTG) at $OD_{600\ nm}$ ~0.5 and incubation at 37°C, under 150 rpm agitation

**Figure 19** shows effect of different concentration of inducer (IPTG) on recombinant ARM thermostable lipase gene expression at $OD_{600\ nm}$ ~ 0.5, in 200 ml of LB broth containing 50 μg/ml ampicillin, after 18 h post induction incubation at 37°C, under 150 rpm agitation.

**Figure 20** shows secretory expression. Effect of concentration of inducer (IPTG) on recombinant ARM thermostable lipase gene expression and release at $OD_{600\ nm}$ ~ 0.5, in 200 ml of LB broth containing 50 μg/ml ampicillin and 34 μg/ml chloramphenicol, after 16 h post induction incubation at 37°C, under 150 rpm agitation

**Figure 21** shows secretory expression. Effect of post-induction time on recombinant ARM thermostable lipase gene expression and release in 200 ml of LB broth containing 50 μg/ml ampicillin and 34 μg/ml chloramphenicol, after induction by 50 μM inducer (IPTG) at $OD_{600\ nm}$ ~ 0.5 and incubation at 37°C, under 150 rpm agitation

**Figure 22** shows immobilized metal affinity chromatography (ProBond™ Purification System) chromatogram of **the** His-tagged recombinant ARM thermostable lipase purification.

Ac: lipase activity; [Pr]: protein concentration; Crude: cell lysate; Flow: flow through fraction; w1-w8: washing fractions; No. 1-12: elution fractions.

**Figure 23** shows SDS-PAGE gel of the His-tagged recombinant ARM thermostable lipase purification steps. Lane 1, molecular weight markers, 116, 66.2, 45, 35, 25, 18.4 and 14.4 kDa; Lane 2, cell lysate; Lane 3, flow through; Lanes 4-7, washing fractions and Lanes 8-13, elution fractions

**Figure 24** shows determination of the molecular weight of native purified recombinant ARM thermostable lipase by size exclusion chromatography. The following standards were used in this experiment to calibrate the Sephadex G-75 column: bovine serum albumin (BSA) (67 kDa), ovalbumin (43 kDa), chymotrypsinogen A (25 kDa), and ribonuclease A (13.7 kDa)

**Figure 25** shows effects of pH and temperature on purified recombinant ARM thermostable lipase activity. The activity was determined by emulsifying the substrate (olive oil) in buffers of different pH ranging from 4.4 to 10.6 at different temperatures (40-80°C) under 200 rpm agitation. The used buffers (50 mM) were sodium acetate (Act.; 4.4-5.6), sodium citrate-sodium phosphate (PC; 5.6-6.5), sodium phosphate (PBS; 6.0-8.0), Tris-HCl (Tris; 7.5-8.5), glycine-NaOH (Gly; 8.0-9.5) and sodium carbonate (H; 9.6-10.6). All buffers were prepared at exact testing temperatures.

**Figure 26** shows effect of 50 mM Tris-HCl buffer (pH 7.5 and 8.0) at different temperatures (60-75°C) on purified recombinant ARM thermostable lipase activity, under 200 rpm agitation using olive oil as substrate. Buffers were prepared at exact testing temperatures.

**Figure 27** shows effect of pH on purified recombinant ARM thermostable lipase activity at 70°C, under 200 rpm agitation using olive oil as substrate. The used buffers (50 mM) were sodium acetate (Act.; 4.4-5.6), sodium citrate-sodium phosphate (PC; 5.6-6.5), sodium phosphate (PBS; 6.0-8.0), Tris-HCl (Tris; 7.5-8.5), glycine-NaOH (Gly; 8.0-9.5) and sodium carbonate (H; 9.6-10.6). All buffers were prepared at exact testing temperature.

**Figure 28** shows residual activity of purified recombinant ARM thermostable lipase as a function of pH and pre-incubation time. The enzyme was pre-incubated in different buffers (1:1 v/v) at 50°C. The activity was determined at 65°C in Tris-HCl buffer pH 8.0, under 200 rpm agitation, using olive oil as substrate. The used buffers (50 mM) were sodium acetate (Act.; 4.4-5.6), sodium citrate-sodium phosphate (PC; 5.6-6.5), sodium phosphate (PBS; 6.0-8.0), (Tris; 7.5-8.5), glycine-NaOH (Gly; 8.0-9.5) and sodium carbonate (H; 9.6-10.6). All buffers were prepared at exact testing temperatures.

**Figure 29** shows effect of metal ions on purified recombinant ARM thermostable lipase activity. The enzyme was pre-incubated for 30 min at 30°C with various metal ions. Two concentrations (1 and 5 mM) of each salt were prepared using MOPS buffer (50 mM, pH 8) (Aslamkhan *et al.,* 2002). The control contained no ion. The relative activity was measured at 65°C using olive oil (emulsified, 1:1 v/v, in 50 mM Tris-HCl buffer, pH 8.0) as substrate.

**Figure30** shows effect of surfactants on purified recombinant ARM thermostable lipase activity. The enzyme was pre-incubated for 30 min at 30°C in Tris-HCl buffer (50 mM, pH 8.0) containing 0.1 and 1% (v/v) of each non-ionic surfactant and in the case of SDS (ionic surfactant) concentrations were 1 and 5 mM. The control contained no surfactant. The relative activity was measured at 65°C using olive oil (emulsified, 1:1 v/v, in 50 mM Tris-HCl buffer, pH 8.0) as substrate.

**Figure 31** shows effect of inhibitors on purified recombinant ARM thermostable lipase activity. The enzyme was pre-incubated for 30 min at 30°C in Tris-HCl buffer (50 mM, pH 8.0) containing 1 and 5 mM of following inhibitors: EDTA as di- and tri-valent metal ions chelating agent; β-mercaptoethanol as redox reagent and PMSF as serine hydrolase inhibitor.

The control contained no inhibitor. The relative activity was measured at 65°C using olive oil (emulsified, 1:1 v/v, in 50 mM Tris-HCl buffer,pH 8.0) as substrate.

**Figure 32** shows specificity of purified recombinant ARM thermostable lipase towards natural oils. The oils were emulsified (1:1 v/v) in 50 mM Tris-HCl (pH 8.0). The lipase activity was assayed at 65°C. The relative activity was expressed as a fraction of the activity obtained by olive oil (as the standard substrate).

**Figure 33** shows HPTLC chromatograms for the products analysis of hydrolysed,triolein by ARM lipase, (A) before (0 h) and (B) after (4 h) reaction at 65°C under 200 rpm agitation using pure triolein as substrate that was emulsified (1:1 v/v) in 50 mM Tris-HCl (pH 8.0). n-hexane was added to stop the reaction and extract the products following by mono-dimensional HPTLC analysis using n-hexane/diethyl ether/acetic acid, 54:45:1 (v/v) as mobile phase. Products were visualized with 10% concentrated sulfuric acid in methanol. The quantities of products were determined by densitometry and comparison with standard compounds using the CAMAG TLC scanner 3 under wavelength of 385 nm and winCATS Planar Chromatography Manager software (CAMAG). Pure monoolein, 1,2 (2,3) diolein, 1,3 diolein, oleic acid and triolein were used as the reference glycerides.

**Figure 34** shows effect of organic solvents on purified recombinant ARM thermostable lipase stability. The enzyme was incubated with the solvents (30%, v/v) for 30 min at 30°C with shaking at 150 rpm. The stability was expressed as the remaining lipolytic activity relative to that of the control (without solvent). The relative activity was measured at 65°C using olive oil (emulsified, 1:1 v/v, in 50 mM Tris-HCl buffer, pH 8.0) as substrate.

**Figure 35** shows effect of ionic liquids on purified recombinant ARM thermostable lipase activity. Enzyme was suspended in 90% (v/v) of each ionic liquid (IL) properly. Then, the mixture was used for lipase activity. The selected ILs were [Emim][$BF_{4]}$; [Emim][TfO];

[Bmim][$BF_{4]}$ and [Bmim][$PH_6$]. The activity was expressed as the remaining lipolytic activity relative to that of the control (without ILs). The residual activity was measured at 65°C using olive oil (emulsified, 1:1 v/v, in 50 mM Tris-HCl buffer, pH 8.0) as substrate.

**Figure 36** shows effect of ionic liquids on purified recombinant ARM thermostable lipase thermostability. Enzyme was suspended in 90% (v/v) of each ionic liquid (IL) properly. Then, the mixture of lipase and IL was incubated for 0-90 min at 60°C with shaking at 150 rpm. The selected ILs were [Emim][$BF_4$]; [Emim][TfO]; [Bmim][$BF_{4]}$ and [Bmim][$PH_6$]. The thermostability was expressed as the remaining lipolytic activity relative to that of the control (without ILs) after incubation at high temperature. The residual activity was measured at 65°C using olive oil (emulsified, 1:1 v/v, in 50 mM Tris-HCl buffer, pH 8.0) as substrate.

## DESCRIPTION OF THE INVENTION

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein are incorporated herein by reference to disclose and describe the methods and/or materials in connection with which the publications are cited. It must be noted that as used herein and in the appended claims, the singular forms "a," "an," and "the" include plural referents unless the context clearly dictates otherwise.

The invention discloses 58 different positive lipolytic colonies isolated from oil-contaminated soil. A Gram-negative and Gram-positive spore-forming bacilli shaped isolates were selected as the best lipase producers for further studies. Polyphasic taxonomy studies categorized the selected isolates as: (1) *Geobacillus* sp. strain ARM species novel (Patent deposit: DSM 21496, NCIMB 41583; GenBank accession for 16S rDNA: EF025325); and (2) *Aneurinibacillus thermoaerophilus* strain AFNA subspecies novel (Patent deposit: DSM 21497, NCIMB 41584; GenBank accession for 16S rDNA: EF032876).

Thermostable lipase production was optimized using artificial neural network (ANN) and response surface methodology (RSM) whereupon 4.7-fold (strain ARM) and 1.85-fold (strain AFNA) increases in lipase productions were achieved. The mature ARM thermostable lipase gene (as a new lipase gene; Genbank accession: EF042975) was cloned into the pTrcHis expression vector and over-expressed in *E. coli* TOP10 host. The optimum lipase expression is obtained after 18 h of induction by 1.0 mM IPTG, where the lipase activity was approximately 1623-fold higher than wild-type. ARM lipase was secreted out by bacteriocin release protein gene co-expression using pJL3 vector in *E. coli* TOP10 host. The optimum lipase secretion is obtained after 16 h of induction by 50 μM IPTG, where 72.8% of total lipase was secreted in culture medium. The His-tagged recombinant lipase was purified with 63.2% total recovery and average purification factor of 14.6 using immobilized metal affinity chromatography.

Also, purified ARM lipase is characterize to be as high active (7092 $Umg^{-1}$), serine-hydrolase, thermostable, organic solvent tolerant, regioselective (1,3-specific) lipase with a molecular weight around 44 kDa. The enzyme is a monomer with disulfide bond(s) in its structure, but is not a metalloenzyme. It hydrolysed a broad range of natural oils as substrate. ARM lipase was active in a broad range of temperature (40-80°C) and pH (6.0-9.0) with optimum lipolytic activity at pH 8.0 and 65°C. It was stable for 2 h at pH 5.0-8.0, 1.5 h at pH 9.0 and 50 min at pH 10.0 at 50°C. Lipase activity and thermostability were significantly increased (P < 0.05) by some tested ionic liquids.

Industrial application

Thermostable bacterial lipases occupy a place of prominence among biocatalysts owing to their novel, multifold applications and resistance to high temperature and other operational conditions. Capability of lipases to catalyze a variety of novel reactions in both aqueous and nonaqueous media and a billion dollar business of lipases in commercial use, present a fascinating field for research and creating interest to isolate novel lipase producers, clone and over-express the lipase gene.

**SEQUENCE LISTING**

**16S rDNA sequence of *Geobacillus* sp. strain ARM (EF025325)**

```
   1 ttcttttgga gagtttgatc ctggctcagg acgaacgctg gcggcgtgcc taatacatgc
  61 aagtcgagcg gactggattg gagcttgctc tgattcggtc agcggcggac gggtgagtaa
 121 cacgtgggca acctgcccgc aagaccggga taactccggg aaaccggagc taataccgga
 181 taacaccgaa gaccgcatgg tctttggttg aaaggcggcc tttggctgtc acttgcggat
 241 gggcccgcgg cgcattagct agttggtgag gtaacggctc accaaggcga cgatgcgtag
 301 ccggcctgag agggtgaccg gccacactgg gactgagaca cggcccagac tcctacggga
 361 ggcagcagta gggaatcttc cgcaatgggc gaaagcctga cggagcgacg ccgcgtgagc
 421 gaagaaggcc ttcgggtcgt aaagctctgt tgtgagggac gaaggagcgc cgttcgaaga
 481 gggcggcgcg gtgacggtac ctcacgagga agccccggct aactacgtgc cagcagccgc
 541 ggtaatacgt agggggcgag cgttgtccgg aattattggg cgtaaagcgc gcgcaggcgg
 601 ttccttaagt ctgatgtgaa agcccacggc tcaaccgtgg agggtcattg gaaactgggg
 661 gacttgagtg caggagagga gagcggaatt cccacgtgta gcggtgaaat gcgtagagat
 721 gtggaggaac accagtggcg aaggcggctc tctggcctgc aactgacgct gaggcgcgaa
 781 agcgtgggga gcaaacagga ttagataccc tggtagtcca cgccgtaaac gatgagtgct
 841 aagtgttaga ggggtcacac cctttagtgc tgcagctaac gcgataagca ctccgcctgg
 901 ggagtacggc cgcaaggctg aaactcaaag gaattgacgg gggcccgcac aagcggtgga
 961 gcatgtggtt taattcgaag caacgcgaag aaccttacca ggtcttgaca tcccctgaca
1021 acccaagaga ttgggcgttc cccttcgggg gggacagggt gacaggtggt gcatggttgt
1081 cgtcagctcg tgtcgtgaga tgttgggtta agtcccgcaa cgagcgcaac cctcgcctct
1141 agttgccagc attcggttgg gcactctaga gggactgccg gcgacaagtc ggaggaaggt
1201 ggggatgacg tcaaatcatc atgcccctta tgacctgggc tacacacgtg ctacaatggg
1261 cggtacaaag ggctgcgaac ccgcgagggg gagcgaatcc caaaaagccg ctctcagttc
1321 ggattgcagg ctgcaactcg cctgcatgaa gccggaatcg ctagtaatcg cggatcagca
1381 tgccgcggtg aatacgttcc cgggccttgt acacaccgcc cgtcacacca cgagagcttg
```

1441 caacacccga agtcggtgag gcaacccgca agggagccag ccgccgaagg tggggcaagt

1501 gattggggtg aagtcgtaac aaggtagccg taccggaagg tgcggctgga tcacctcctt

1561 tct

**16S rDNA sequence of *Aneurinibacillus thermoaerophilus* strain AFNA (EF032876)**

1 gagagtttga tcctggctca ggacgaacgc tggcggcgtg cctaatacat gcaagtcgag

61 cgaaccgatg gagtgcttgc attcctgagg ttagcggcgg acgggtgagt aacacgtagg

121 caacctgcct gtacgaccgg gataactccg ggaaaccgga gctaataccg gataggatgc

181 cgaaccgcat ggttcggcat ggaaaggcct ttgagccgcg tacagatggg cctgcggcgc

241 attagctagt tggtggggta acggcctacc aaggcgacga tgcgtagccg acctgagagg

301 gtgaacggcc acactgggac tgagacacgg cccagactcc tacgggaggc agcagtaggg

361 aatcttccgc aatggacgaa agtctgacgg agcaacgccg cgtgagtgag gaaggtcttc

421 ggatcgtaaa actctgttgt cagggaagaa ccgccgggat gacctcccgg tctgacggta

481 cctgacgaga aagccccggc taactacgtg ccagcagccg cggtaatacg taggggggcaa

541 gcgttgtccg gaattattgg gcgtaaagcg cgcgcaggcg gcttcttaag tcaggtgtga

601 aagcccacgg ctcaaccgtg gagggccatc tgaaactggg gagcttgagt gcaggagagg

661 agagcggaat tccacgtgta gcggtgaaat gcgtagagat gtggaggaac accagtggcg

721 aaggcggctc tctggcctgt aactgacgct gaggcgcgaa agcgtgggga gcaaacagga

781 ttagataccc tggtagtcca cgccgtaaac gatgagtgct aggtgttggg gagtccacct

841 cctcagtgcc gcagctaacg caataagcac tccgcctggg gagtacggcc gcaaggctga

901 aactcaaagg aattgacggg gacccgcaca agcggtggag catgtggttt aattcgaagc

961 aacgcgaaga accttaccag ggcttgacat cccgctgacc cctccagaga tggaggcttc

1021 cttcgggaca gcggtgacag gtggtgcatg gttgtcgtca gctcgtgtcg tgagatgttg

1081 ggttaagtcc cgcaacgagc gcaacccttg tcctttgttg ccagcattca gttgggcact

1141 ctaaggagac tgccgtcgac aagacggagg aaggtgggga tgacgtcaaa tcatcatgcc

1201 ccttatgtcc tgggctacac acgtgctaca atggacggta caacgggcgt gccaacccgc

1261 gagggtgagc caatccctaa aaaccgttct cagttcggat tgcaggctgc aactcgcctg

1321 catgaagccg gaatcgctag taatcgcgga tcagcatgcc gcggtgaata cgttcccggg

1381 tcttgtacac accgcccgtc acaccacgag agtttgcaac acccgaagtc ggtgaggtaa

1441 ccttctggag ccagccgccg aaggtggggc agatgattgg ggtgaagtcg taacaaggta

1501 tccg

**Nucleotide sequence of the thermostable ARM lipase (EF042975)**

gggttggaaggggccttgggtcttatgtataaatattctggtaattcagaatgaaaagattt
cggcctattgaaggaaaggagagggagaacatgatgaaatgctgtcggcgtgttgccctt
gtgttgctcggattatggtttgtgttctgcatatctgttctgggagggcgagctgaagcg
gcggcttcgcgagccaacgatgcgccgattgtacttctccacgggtttaccggctgggga
agagaagaaatgtttgggttcaagtactggggcggcgtgcgcggcgatatcgaacaatgg
ctgaacgacaacggttatcgaacttatacgctggcggtcggcccgctctcgagcaactgg
gatcgggcgtgcgaagcgtatgcccagcttgtcggcgggacggtcgattatggggcagcc
catgcggcaaagcacggccatgcgcggtttggccgcacctatcccggcctgttgccggaa
ttgaaaaggggcggtcgcatccatatcatcgcccacagccaaggagggcagacggcccgc
atgcttgtatcgctcctagagaatggaagccaagaagagcgggagtacgctaaggcgcat
aacgtgtctttgtcgccgttgtttgaaggcggacatcattttgtgttgagagtgacaacc
attgccacccctcatgacgggacgacgcttgtcaacatggttgatttcaccgatcgcttt
tttgacttgcaaaaagcggtgttgaaagcagcggctgtcgccagcaatgtgccgtacaca
agtcaagtatacgattttaagctcgaccaatggggactgcgccgccagccaggtgaatcg
tttgaccaatattttgaacggctcaaacggtcccctgtttggacgtcgacagataccgct
cgctatgatttatccgttcccggggctgagaagttgaatcaatgggtgaaagcaagcccg
aatacgtattatttgagctttgccacagaacggacgtatcgcggagcgctcacaggcaac
tattatcccgaactcggaatgaatgcattcagcgccgtcgtatgcgccccgtttctcggt
tcgtaccgcaatgcgacgctcggcattgacgaccgctggcttgagaacgatggcatcgtc
aatacgttttcgatgaacggaccaaagcgcggatcgaccgatcggatcgtgccgtatgac
gggacaataaaaaaaggggtttggaatgatatgggaacgtacaatgtcgatcatttggaa
gtcatcggcgttgacccgaatccgttgtttgatatccgtgccttttatttgcgccttgcc
gagcagttggcgagcttgcaaccttaaaactagtattttgcgaaaaaagccatctcgatc
ggatggcgctttttttcgtgaacattcatgcgccttggttggtcattttcatgcccgggt
ggagaacaaacgctgttcatgaacttttctgtgaatatgcacgttcctttaactttcagt
ttcatgtcggggtgaaaaacgaaaaatggtttatggatgttttggagaacaagcagctga
ttggtatgacggccgcggaatcccga

**Amino acid sequence of the thermostable ARM lipase (EF042975)**

```
M M K C C R R V A L V L L G L W F V F C I S V L G G R A
E A A A S R A N D A P I V L L H G F T G W G R E E M F G F
K Y W G G V R G D I E Q W L N D N G Y R T Y T L A V G P
L S S N W D R A C E A Y A Q L V G G T V D Y G A A H A A K
H G H A R F G R T Y P G L L P E L K R G G R I H I I A H
S Q G G Q T A R M L V S L L E N G S Q E E R E Y A K A H N
V S L S P L F E G G H H F V L R V T T I A T P H D G T T
L V N M V D F T D R F F D L Q K A V L K A A A V A S N V
P Y T S Q V Y D F K L D Q W G L R R Q P G E S F D Q Y F E
R L K R S P V W T S T D T A R Y D L S V P G A E K L N Q
W V K A S P N T Y Y L S F A T E R T Y R G A L T G N Y Y P
E L G M N A F S A V V C A P F L G S Y R N A T L G I D D
R W L E N D G I V N T F S M N G P K R G S T D R I V P Y
D G T I K K G V W N D M G T Y N V D H L E V I G V D P N P
L F D I R A F Y L R L A E Q L A S L Q P
```

nucleotide sequence (16S rDNA) of SEQ ID NO 1 (EF025325).
nucleotide sequence (16S rDNA) of SEQ ID NO 2 (EF032876).
nucleotide sequence of SEQ ID NO 3 (EF042975)
amino acid sequence of SEQ ID NO 4 (EF042975).

## BEST MODE TO CARRY OUT THE INVENTION

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

On the basis of nucleic acids in accordance with SEQ ID NO: 1, 2 and 3 or polypeptides in accordance with SEQ ID NO: 4, the present invention further comprises nucleic acids and polypeptides of a similar structure and the same or similar function as the aforementioned molecules. As modification possibilities in particular those methods are preferred which are known to the person skilled in the art prior art, such as fragmentation, deletion, insertion and fusion with other proteins or protein parts of the mentioned molecules. An optimization can take place for example in the adaptation to temperature influences, pH value variations or redox ratios. Desired modifications are for example the improvement of the oxidation resistance, the stability towards denaturing agents or proteolytic degradation, stability in relation to high temperatures, acidic or strong alkaline conditions, a change of the sensitivity towards calcium ions or other CO factors, a reduction of the immunogenicity or to genes they affect. Further it can be desirable to change the surface charges and/or the isoelectric point of the polypeptides of the invention in order to optimize the interaction with the substrate. An increase of the stability of the polypeptide can take place independent from a coupling to other peptides about also via coupling to polymers.

The person of skill in the relevant art understands that the invention in a further aspect also encompasses any polynucleotide or polypeptide derived from a sequence shown in SEQ ID No: 1 to 4, and/or fragments thereof, under the provision that the derived molecules or fragments are functionally equivalent to the molecules of SEQ ID No 1 to 4. Preferably such derived molecules or fragments comprise the catalytic domain of the enzymes of the present invention.

Another subject matter of the instant invention is therefore a Polynucleotide selected from the group comprsing:

a) Polynucleotide with a nucleic acid sequence in accordance with SEQ ID NO: 1 and SEQ ID NO: 2 and SEQ ID NO:3

b) Polynucleotide coding for a polypeptide with an amino acid sequence in accordance with SEQ ID NO: 4.

Furthermore subject matter of the instant invention is the use of the nucleic acids according to invention, and the production or isolation of nucleic acids according to invention, or the production or isolation of nucleic acids which are homologous to the nucleic acids according to invention. In particular encompassed are such nucleic acids which have a similar structure and the same, similar and/or improved functional properties as the nucleic acids according to the invention, wherein the basic functional properties of the nucleic acids of the invention are present. In particular the hydrolase activity, preferably the proteolytic activity, and basic improved functional properties for example higher specificity and/or higher conversion and/or higher stability of the enzyme in the desired reaction conditions, in particular regarding the pH value or temperature.

The nucleic acids according to invention can be used for example as probes for the identification and/or isolation of homologous nucleic acids from a DNA, one cDNA or a genomic gene bank; here preferably for the identification of nucleic acids, which are coding for hydrolases, in particular proteases, and/or for their coding parts, or as anti scythe nucleic acids. Furthermore they can be used as primers in the polymerase chain reaction (PCR) for the amplification of nucleic acids comprising nucleic acids for hydrolases, preferably proteases, or their parts, for use in an "expression cassette" or "expression vector", which are a nucleic acid construct generated recombinantly or synthetically, with a series of specified nucleic acid elements that permit the transcription of a particular nucleic acid in a target cell. The recombinant expression vector can be incorporated into a plasmid, chromosome, mitochondrial DNA, plastid DNA, virus, or nucleic acid fragment. Typically, the expression vector includes, among other sequences, a nucleic acid sequence to be transcribed and a promoter. Expression cassette may be used interchangeably with DNA construct and its grammatical equivalents. As used herein, the term "vector" refers to a nucleic acid construct designed to transfer nucleic acid sequences into cells. An "expression vector" refers to a vector that has the ability to incorporate and express heterologous DNA fragments in a foreign cell. Many prokaryotic and eukaryotic expression vectors are commercially available. Selection of appropriate expression vectors is within the knowledge of those having skill in the art. As used herein, the term "plasmid" refers to a circular double-stranded (ds) DNA construct used as a cloning vector, and which forms an extrachromosomal self-replicating genetic element in some eukaryotes or integrates into the host chromosomes. The term "nucleic acid molecule" or "nucleic acid sequence" includes RNA, DNA and cDNA molecules. It will be understood that, as a result of the degeneracy of the genetic code, a multitude of nucleotide sequences encoding a given protein may be produced.

Other subject matter of the instant invention is therefore a method for the isolation, preferably of a protease, or parts thereof, and its coding nucleic acid, comprising subsequently the steps of:

a) on the basis of a nucleic acid according to invention primers are prepared,
b) the primers in accordance with (a) are used, in order to be amplified in the PCR nucleic acids, above all cDNAs, unknown nucleic acid sequence,
c) the nucleic acids obtained in accordance with (b) by amplification are then sequenced.

Another subject matter of the instant invention is a method for the production of a nucleic acid according to invention, characterised in that the nucleic acid is chemical synthesized. The nucleic acids according to invention can for example be chemically synthesized on the basis of the sequence shown in SEQ ID NO: 1 and SEQ ID NO: 3 or on the basis of SEQ ID NO: 2 and SEQ ID NO: 4 indicated amino acid sequence in the manner known to the person of skill in the relevant art. Furthermore, subject matter of the instant invention are vectors, in particular cloning and/or expression vectors, comprising one of the nucleic acids according to invention specified before. The vector can be for example a prokaryotic or eukaryotic vector. The nucleic acids according to invention can become in an embodiment with flanking nucleic acids into a vector incorporated in such a way that with expression of the vector according to invention the polypeptides encoded of the nucleic acids as fusion proteins to be present or one day, a marking amino acid sequence, inertial, which can facilitate for example the cleaning and/or detection of the polypeptides. The expression vectors here preferably contain the preferred regulatory sequences suitable for the host cell, lac or the TAC promoter for an expression.

Therefore the invention furthermore pertains to the following aspects:

1. Biologically pure cultures of *Geobacillus* sp. strain ARM and *Aneurinibacillus thermoaerophilus* strain AFNA is isolated from soil; wherein

    a) *Geobacillus sp* strain ARM is deposited under the accession number DSM 21496 at DeutscheSammlung von Mikroorganismen und Zellkulturen (DSMZ) and NCIMB 41583 at The National Collection of Industrial, Marine and Food Bacteria (NCIMB), UK, and
    b) *Aneurinibacillus thermoaerophilus* strain AFNA is deposited under the accession number DSM 21497 at DeutscheSammlung von Mikroorganismen und Zellkulturen (DSMZ) Germany and NCIMB 41584 at The National Collection of Industrial, Marine and Food Bacteria (NCIMB), UK

2. The biologically pure cultures as claimed in claim 1, wherein the soil is contaminated with oil.

3. The biologically pure cultures as claimed in claim 1,wherein the *Geobacillus sp strain* ARM and *Aneurinibacillus thermoaerophilus* strain AFNA having the capability to produce a novel thermostable enzyme isolated and characterized from *Geobacillus sp* strain ARM and *Aneurinibacillus thermoaerophilus* strain AFNA.

4. The novel thermostable enzyme isolated and characterized from *Geobacillus sp* strain ARM and *Aneurinibacillus thermoaerophilus* strain AFNA according to claim 3, wherein the novel thermostable enzyme is a thermostable lipase.

5. The biologically pure cultures as claimed in claim 1(a), wherein the *Geobacillus sp* strain ARM providing characteristics that includes:

a) a gram positive microorganism ;
b) having the capability to grow between 45°C and 70°C with optimum growth 55°C;
c) showing a positive growth in tributyrin agar and Rhodemine B agar and nutrient agar;
d) having the capability to grow at pH between 5.5-9 with optimal pH growth of 7;
e) negative anaerobic growth; and
f) organic solvent tolerant.

6. The biologically pure cultures as claimed in claim 1(a), wherein the *Geobacillus sp* strain ARM showing physiological characteristics that includes:

a) catalase positive and oxidase negative;
b) fermentation of D-glucose (positive), L-arabinose (negative), D-xylose (positive),D-mannitol (positive, D-fructose (positive);
c) hydrolyse Tween 8;
d) hydrolyse natural oils.

7.The biologically pure cultures as claimed in claim 1(a), wherein the *Geobacillus sp* strain ARM encodes nucleotide sequence 16S rDNA of SEQ ID NO 1 (EF025325) and/or nucleotide sequence (EF042975) of SEQ ID NO 3 and/or amino acid sequence (EF042975)of SEQ ID NO 4.

8. The biologically pure cultures as claimed in claim 1(b), wherein the *Aneurinibacillus thermoaerophilus* strain AFNA providing characteristics that includes:

a) a gram negative microorganism ;
b) having the capability to grow between 45°C and 60°C with optimum growth 53°C;
c) showing a positive growth in tributyrin agar and Rhodemine B agar and nutrient agar;
d) having the capability to grow at pH between 5.5-7.5 with optimal pH growth of 6.9;
e) negative anaerobic growth.

9. The biologically pure cultures as claimed in claim 1(b), wherein the *Aneurinibacillus thermoaerophilus* strain AFNA providing physiological characteristics that includes:

a) catalase positive and oxidase negative;
b) fermentation of D-glucose (positive), L-arabinose (negative), D-xylose (negative),D-mannitol (negative), D-fructose (negative);
c) hydrolyse gelatine and casein; and
d) hydrolyse natural oils such as olive, sesame and corn oil.

10. The biologically pure cultures as claimed in claim 1(b), wherein the *Aneurinibacillus thermoaerophilus* strain AFNA encodes nucleotide sequence (16S rDNA) of SEQ ID NO 2 (EF032876).

11. The novel thermostable lipase isolated and characterized from *Geobacillus sp* strain ARM and encodes a nucleotide sequence of SEQ ID NO 3 (EF042975).

12. The novel thermostable lipase isolated and characterized from *Geobacillus sp* strain ARM encodes amino acid sequence of SEQ ID NO 4 (EF042975).

13. A method of obtaining, wherein the novel thermostable lipase isolated and characterized from *Geobacillus sp* strain ARM, wherein the method comprising the steps of:

a) conducting DNA extraction of the *Geobacillus sp* strain ARM,

b) obtaining a purified DNA from step (a),

c) identifying and amplifying 16S rDNA sequence by conducting polymerase chain reaction (PCR) technique conducting DNA extraction of the *Geobacillus sp* strain ARM;

d) obtaining mature lipase gene (ARM lipase) by performing cloning technique;

e) conducting secretory expression of the mature lipase gene (ARM lipase) and obtaining recombinant ARM lipase gene;

f) purifying the ARM lipase gene from step (e) and obtaining, a purified ARM lipase;

g) determining size of ARM lipase;

h) characterizing the ARM lipase using biochemical and biophysical methods.

14. The method as claimed in claim 12, wherein the ARM lipase provides the followings;

a) Working inducer (IPTG) for intracellular expression between 0-1.5 mM with optimum concentration of 1.0 mM at incubation time between 0 and 40 hours with optimum time of 18 hour.

b) Working inducer (IPTG) for secretory expression between 0-100 μM with optimum concentration of 50 μM at incubation time between 0 and 25 hours with optimum time of 16 hour.

15. The method as claimed in claim 12, wherein the mature ARM lipase compose of 388 amino acids and the mature ARM lipase having a size of 44 kDa.

16. The method as claimed in claim 12, wherein the ARM lipase is characterized as the followings:

a) lipase activity of 7092 $Umg^{-1}$
b) for intracellular expression induced with 1.0 mM of IPTG for 18 hours at optimum condition
c) for secretory expression induced with 50 μM of IPTG for 16 hours at optimum condition
d) increase of expression level of at least 1623 fold
e) lipase recovery of 63.2% and purification factor of 14.6;
f) ARM mature lipase
g) working temperature range from 40°C to 80°C with an optimum temperature of 65°C ;
h) working pH in the range of pH 6 to 9 with an optimum pH 8.0 at 65 °C;
i) inhibiting β-mercaptoethanol and phenylmethanesulfonyl fluoride (PMSF);
j) hydrolyse natural oils;
k) regioselective (1,3-specific);
l) organic solvent tolerant;
m) thermostability in ionic liquids such as [Emim][$BF_4$], [Emim][TfO], [Bmim][$BF_4$] and [Bmim][$PH_6$], wherein the lipase shows lipase thermostability in [Emim][TfO] and Bmim][$BF_4$].

17. Use of the *Geobacillus sp* strain ARM and *Aneurinibacillus thermoaerophilus* strain AFNA producing thermostable lipase according to any of claims 1 to 11 for industrial applications such as in food industry, oil processing, production of surfactants, oil processing, detergents, pesticides, environmental management and leather industry.

18. Use of the *Geobacillus sp* strain ARM producing thermostable lipase (ARM lipase) according to any of claims 1 to 15 for industrial applications such as in food industry, oil processing, production of surfactants, oil processing, detergents, pesticides, environmental management and leather industry.

**EXAMPLES**

**PREPARATION OF MEDIA AND SOLUTIONS**

**a) Preparation of tributyrin agar plates**

The medium was made by mixing 28 g/L nutrient agar and 1% of tributryin ($C_4$) as substrate in distilled water. The pH was adjusted to 7.0. The medium was homogenized for 4 min and autoclaved at 121°C (15 Ib/$in^2$) for 15 min. The medium was then cooled down to about 45°C and aseptically poured into sterile plates of 90 mm width (Mourney and Kilbertus, 1976).

**b) Preparation of Rhodamine B agar plates**

The medium was made by mixing 28 g/L nutrient agar and 1% of olive oil ($C_{18}$) as long chain substrate, specific for lipase detection in distilled water. The pH was adjusted to 7.0. The medium was homogenized for 4 min and autoclaved at 121°C (15 lb/in$^2$) for 15 min. The medium was then cooled down to about 45°C and 0.01 g/L Rhodamine B solution aseptically added by filtration (0.22 $\mu$m), and the medium poured into sterile plates of 90 mm (Kouker and Jaeger, 1987).

**c) Lipase production media**

In order to select the best lipase production medium, eight different media were tested. The composition of the media was (% w/v):

**M1:** peptone (3), yeast extract (1), NaCl (0.5), olive oil (1% v/v) (Chin *et al.,* 2003);

**A1:** (modified M1): M1+ $CaCl_2$.2H2O (0.05) + gum arabic (1);

**A2** (modified M1): A1+ $MgSO_4.7H_2O$ (0.01), $FeCl_3.6H_2O$ (0.004);

**GYP:** glucose (2), yeast extract (1), peptone (1), $CH_3COONa{\cdot}3H_{2O}$ (1), $MgSO_4{\cdot}7H_2O$ (0.03), $MnSO_4$ (0.01), KCl (0.05), olive oil (2% v/v) (Chin *et al.,* 2003);

**M3:** nutrient broth (0.325), gum arabic (1), $CaCl_2{\cdot}2H_2O$ (0.05), Tween 80 (1% v/v), olive oil (1% v/v) (Chin *et al.,* 2003);

**M5:** nutrient broth (0.8), triolein (1% v/v) (Chin *et al.,* 2003);

**TYEM:** tryptone (0.6), yeast extract (0.2), $CaCl_2.2H_2O$ (0.02), $MgSO_4.7H_2O$ (0.01), $FeCl_3.6H_2O$ (0.04), olive oil (1.5% v/v) (Lee *et al.,* 1999);

**MTYEM** (modified TYEM): tryptone (0.6), yeast extract (0.2), $CaCl_2.2H_2O$ (0.02), $MgSO_4.7H_2O$ (0.01), $FeCl_3.6H_2O$ (0.04), gum arabic (1), olive oil (1.5% v/v).

The media were homogenized for 5 min and sterilized for 15 min at 121°C after pH adjustment to 7.0.

**d) Preparation of the TFB1 Buffer**

$RbCl_2$ (100 mM), $MnCl_2$ (50 mM), potassium acetate (30 mM), $CaCl_2$ (10 mM) and 15% glycerol. The buffer was sterilized through a 0.22 $\mu$m membrane filter after pH adjustment to 5.8 with acetic acid (Sambrook *et al.,* 1989).

**e) Preparation of the TFB2 Buffer**

MOPS (10 mM), $RbCl_2$ (10 mM), $CaCl_2$ (75 mM) and 15% glycerol. The buffer was sterilized through a 0.22 $\mu$m membrane filter after pH adjustment to 6.8 with KOH (Sambrook *et al.,* 1989).

**f) Preparation of SOC medium**

SOC (Super Optimal Broth) is a rich medium used primarily in the recovery step of *E. coli* competent cell transformations. Use of SOC maximizes the transformation efficiency of competent cells. The medium was made of tryptone (pancreatic digest of casein) 2% (w/v), yeast extract 0.5% (w/v), NaCl 8.6 mM, KCl 2.5 mM, $MgSO_4$ 20 mM, glucose 20 mM. The medium was sterilized for 15 min at 121°C after pH adjustment to 7.0 (Sambrook *et al.,* 1989).

**ISOLATION AND SCREENING OF THERMOPHILIC LIPASE PRODUCING BACTERIA**

In order to isolate lipase producing bacteria, samples were collected from lipase substrate (oil). Samples (oil-contaminated soil) were collected from Shell Refining Co. in Port Dickson, Malaysia and Taman Seri Serdang, Seri Kembangan, Malaysia. One gram of each sample was suspended in 10 ml enrichment medium (Tryptic Soy Broth; TSB) and incubated at 60°C with shaking at 150 rpm for 12 h. Diluted enriched samples in normal saline (1/10) were spread on trybutyrin agar (TBA) plates (screening plates), and incubated at 60°C for 24, 48 and 72 h. Clear zone formations surrounding the colonies were considered positive. The lipase activity was confirmed by Rhodamine B agar (RBA) plate.

Colonies forming halos on TBA were taken out using a platinum loop and were streaked on the RBA plates (Rhodamine B forms a fluorescent complex with free fatty acids. Thus, the lipase-producing colonies give an orange fluorescent halo that is visible under UV light at 350 nm). The positive colonies were subcultured on nutrient agar plates several times to obtain pure cultures. In order to select the best lipase producing bacteria, bacterial inoculum (2% v/v; $Ab_{600}$ = 0.5 of overnight culture in TSB) of each isolate was individually inoculated into 50 ml of M1 and A1 production media and incubated by agitation under 150 rpm, for 48 h at 60°C. The cell free supernatant was obtained by centrifugation at 12,000 x g, 4°C for 15 min prior to lipase activity assay. After increasing the population of thermophilic bacteria in enrichment medium (TSB) at 60°C, a total of 50 different positive colonies on screening agar-plates **(Figure 1)** were isolated from oil-contaminated soil from Shell Refining Co., Port Dickson, Negeri Sembilan, Malaysia and a total of 8 different positive colonies from Taman Seri Serdang, Seri Kembangan, Malaysia. Lipase production ability and maximum cultivation temperature of selected isolates were tested in M1 and A1 production media and nutrient broth medium, respectively **(Table 1).**

**Table 1. Lipase production ability and maximum cultivation temperature of selected isolates (top five)**

| Isolates | Lipase activity ($Uml^{-1}$) after 48 h, 60°C, 150 rpm in M1 production medium | Lipase activity ($Uml^{-1}$) after 48 h, 60°C, 1.50 rpm in A1 production medium | Maximum cultivation temperature (°C) in nutrient broth medium, overnight, 150 rpm |
|---|---|---|---|
| P9 (AFNA) | 0.06 | 0.15 | 60 |
| P3 (ARM) | 0.04 | 0.10 | 70 |
| S21 | 0.03 | 0.10 | 60 |
| P1 | 0.03 | 0.08 | 60 |
| S12 | 0.03 | 0.04 | 60 |

According to the results (the highest lipase activity and cultivation temperature), the isolates P9 and P3 were selected as the best lipase producers and designated as strains AFNA and ARM, respectively. These strains were used for further investigation.

**a) Lipase activity assay**

Determination of liberated free fatty acid was measured by colorimetry method (Kwon and Rhee, 1986) using olive oil as substrate. The reaction mixture, consisting of 1 ml crude enzyme (culture filtrate), 2.5 ml olive oil emulsion (properly mixed of an equal volume olive oil with 50 mM sodium phosphate buffer, pH 7.0) and 0.02 ml of 20 mM $CaCl_2$, was incubated in a water bath shaker for 30 min at 50°C under 200 rpm agitation. The enzyme reaction in the emulsion system was stopped by adding 6 M HCl (1 ml) and isooctane (5 ml), followed by mixing for 1 min. The upper isooctane layer (4 ml) containing the free fatty acid was transferred to a test tube and properly mixed with 1 ml copper reagent.

The reagent was prepared by adjusting the solution of 5% (w/v) copper (II) acetate-1-hydrate to pH 6.1 with pyridine. The free fatty acid dissolved in isooctane was determined by measuring the absorbance of the upper layer at 715 nm after mixture settlement. Lipase activity was determined by measuring the amount of free fatty acid released based on the standard curve of oleic acid (0-50.0 μmole) in isooctane. One unit of lipase activity was defined as 1.0 μmol of free fatty acid liberated $min^{-1}$ and reported as $Uml^{-1}$.

**b) Glycerol stock preparation**

One single colony of each fresh pure culture was inoculated in 10 ml TSB and incubated at 60°C overnight under 150 rpm shaking. Broth culture aliquots of 1.5 ml were transferred into micro-centrifuge tubes, after centrifugation at 2500 x g for 2 min, supernatant was replaced with sterile pre-prepared 16% (v/v) glycerol in TS B. Bacterial pellets were resuspended and tubes were kept at -80°C.

**BACTERIAL POLYPHASIC STUDIES**

**a) Phenotypic studies**

Standard phenotypic tests were performed to characterize the selected isolates including morphological, physiological and chemotaxonomic studies.

**b) Gram characterization**

A very small sample of a fresh bacterial culture (12 h) was smeared onto a clean glass slide. The bacterial smear was fixed by heating for a few seconds. The slide was flooded with crystal violet for about 1 min. The crystal violet was gently washed off the slide with running water. The bacterial smear was then treated with Gram's iodine for about 1 min. The Gram's iodine was gently washed off the slide with running water. The bacterial smear was decolorized with 95% ethyl alcohol. The slide was gently washed with running water and flooded with safranin for about 45 s (counter stain). The slide was washed with water and placed between some absorbent papers to blot off the excess water gently. The slide was examined under the light microscope (Madigan *et al.,* 2004).

**c) Spore production and morphology**

To enhance sporulation, cells were grown on nutrient agar containing 10 mg $L^{-1}$ $MnSO_4.H_2O$ (as sporulation inducer) at 55°C for 12 h (Charney *et al.,* 1951). Cell and spore morphologies and cell motility were observed under phase-contrast microscope (Zeiss Axioplan) as well as light microscopy after Gram staining.

**d) Electron microscopy**

For scanning electron microscopy, the bacteria were grown on the agar plate and the agar including the bacteria was cut into 1 $cm^2$ blocks. Blocks were fixed with 4% glutaraldehyde in 0.1 M sodium cacodylate buffer for 24 h at 4°C, then washed with same buffer. The secondary fixative was 1% osmium tetroxide in sodium cacodylate buffer for 2 h at 4°C, followed by a buffer rinse. After the secondary fix, dehydration was accomplished through a series of ethanol solutions (35, 50, 75, 95, and 3 changes 100%); each change of dehydrant was held for 15 min.

Samples were then dried in a Critical Point Drier (Bal Tec CPD 030) with liquid $CO_2$ as the transitional fluid (Bozzola and Russell, 1998). Samples were then sputter-coated with gold and viewed by scanning electron microscope (Phillips XL30 ESEM). Flagella were observed by transmission electron microscopy (LEO 912AB EFTEM) after negative staining. For negative staining, concentrated late-exponential phase cells were dropped onto a copper grid (300 mesh), after 5 min cells were stained with 2% (w/v) tungstic acid neutralized to pH 7.2, for 5 min (Lai and Shih, 2001).

**e) Physiological characteristics**

The physiological characteristics including catalase and oxidase test, Voges-Proskauer test, lysozyme broth, fermentation of D-glucose, L-arabinose, D-xylose, D-mannitol, D-fructose, M-inosit, D-ribose, D-cellobiose, L-rhamnose, sorbitol, D-galactose, adonit, D-lactose, hydrolysis of starch, gelatin, casein and Tween 80, decomposition of tyrosine, use of citrate and propionate, nitrate reduction, indol production, phenylalanine deaminase and arginine dihydrolase test were determined with the conventional methods (Gordon *et al.,* 1973) and API systems (API Biotype 100 and API ZYM systems) (bioMerieux), according to the manufacturer's instructions (Appendix A).

All the tests were performed by the German Collection of Microorganisms and Cell Cultures (DSMZ; Deutsche Sammlung von Mikroorganismen und Zellkulturen GmbH), Braunschweig, Germany. The potential for growth at various initial pH values was determined using Nutrient Broth (NB) at 55°C. The growth temperature and optimal NaCl concentrations were determined using the same medium at pH 7.0. Anaerobic growth was examined by using an AnaeroPack jar (Mitsubishi Gas Chemical).

Colonies of strain ARM on nutrient agar were 1-2 mm in diameter, opaque, light-cream in colour, circular, convex on plate with entire margins where in the case of strain AFNA colonies were 2-10 mm in diameter, half-opaque, light-cream in colour, irregular, raised on plate with lobate margins. These strains were strictly aerobic micro-organisms, Gram-positive (strain ARM) or Gram-negative (strain AFNA), spore-forming, motile by means of flagella, occurred singly (strain AFNA) or singly, in pairs and chains (strain ARM), catalase-positive and oxidase-negative. Cells of strain ARM were 2.5->6.0 $\mu$m long and 0.8-1.0 $\mu$m in diameter with oval/cylindrical endospores at the terminal of the cells within a swollen sporangium, where cells of strain AFNA were 3.0-5.0 $\mu$m long and 0.9-1.0 $\mu$m in width with oval endospores at the middle of the cells within a swollen sporangium **(Figure 2).**

Physiological and biochemical characteristics are given in the species description and summarized in **Table 2.** The isolates were thermophilic, exhibiting growth between 45 and 60°C (strain AFNA) or 70°C (strain ARM), with optimum growth ($OD_{600}$) at 53°C (strain AFNA) or 55°C (strain ARM). The strains grew optimally in the absence of NaCl but they tolerated up to 2% (w/v) NaCl. The cells were able to grow in a pH range of 5.5-7.5 with an optimal pH for growth of 6.9 (strain AFNA) or 5.5-9.0 with an optimal pH for growth of 7.0 (strain ARM).

**Table 2. The differentiating characteristics of the thermophilic strains ARM and AFNA**

| Properties | AFNA | ARM |
|---|---|---|
| Catalase | + | + |
| Oxidase | - | - |
| Anaerobic growth | - | - |
| VP reaction | - | no growth |
| pH in VP | 8.8 | - |
| Growth in | | |
| medium pH 5.7 | - | + |
| NaCl 2% | + | + |
| 5% | - | - |
| Lysozyme broth | + | - |
| Acid from | | |
| D-glucose | + | + |
| L-arabinose | - | - |
| D-xylose | - | + |
| D-mannitol | - | + |
| D-fructose | - | + |
| Gas from glucose | - | - |
| Lecithinase | - | - |
| Hydrolysis of | | |
| starch | - | + |
| gelatin | + | w |
| Tween 80 | - | - |
| casein | + | - |
| Decomposition of tyrosine | + | - |
| Use of | | |
| citrate | - | + |
| propionate | + | + |
| $NO_2^-$ from $NO_3^-$ | - | - |
| Indol | - | - |
| Phenylalanine deaminase | - | - |
| Arginine dihydrolase | - | - |

Based on the phenotypic identification keys of aerobic spore-forming bacteria (Reva *et al.,* 2001), strain ARM stood in group IV region D1 that encompasses obligatory aerobic, starch-hydrolysing, nitrate reduction negative, with swollen sporangia, citrate positive, oxidase negative bacilli. There is no thermophile bacterium in this region. The only bacterium in this region is *Paenibacillus alginolyticus* that is completely different than ARM **(Figure 3A).** On the other hand, strain AFNA stood in group III region B1 section ii that encompasses obligatory aerobic, non-starch-hydrolysing, gelatin-hydrolysing, with swollen sporangia, glucose positive bacilli **(Figure 3B).** In spite of similar characteristics to *Aneurinibacillus thermoaerophilus* DSM 10154$^T$, strain AFNA could be clearly distinguished from this type strain, namely based on Gram staining, $NO_2^-$ from $NO_3^-$, catalase and oxidase tests, growth temperature and pH ranges, acid from L-arabinose, D-xylose and D-fructose, use of citrate, assimilation of fructose, glutamate and mannitol **(Table 3).**

**Table 3. Comparison of phenotypic characteristics of strain AFNA and *Aneurinibacillus thermoaerophilus* DSM 10154$^T$**

| Characteristic | AFNA | DSM 10154 |
|---|---|---|
| Gram staining | - | + |
| Catalase | + | w |
| Oxidase | - | + |
| Growth temperature range (°C) | 45-60 | 35-65 |
| pH range | 5.5-7.5 | 6.0-9.0 |
| Acid from | | |
| L-arabinose | - | w |
| D-xylose | - | + |
| D-fructose | - | + |
| Use of citrate | - | + |
| $NO_2^-$ from $NO_3^-$ | - | + |
| Assimilation of | | |
| fructose | - | v |
| glutamat | - | + |
| mannitol | - | v |

## CHEMOTAXONOMIC ANALYSES

Cellular fatty acids, peptidoglycan and cell wall sugars analyses were performed by DSMZ, Braunschweig, Germany . Cellular fatty acids were analysed with the MIDI Sherlock® Microbial Identification System and dendrogram was prepared using cluster analysis techniques to produce unweighted pair matchings based on fatty acid compositions (Sasser, 1990). The Sherlock Microbial Identification System is a fully automated gas chromatographic analytical system, which identifies bacteria based on their unique fatty acid profiles.

The peptidoglycan was isolated and its structure was determined by using methods according to Schleifer and Kandler (1972) and Rhuland *et al.* (1955) with a modification that TLC on cellulose was applied instead of paper chromatography. Cell wall sugars were also analysed according to Staneck and Roberts (1974). Analysis of polar lipids was carried out by the Identification Service and Dr. B.J. Tindall, DSMZ, Braunschweig, Germany.

The total hydrolysates of the peptidoglycan of both strains contained the amino acids meso-diaminopimelic acid (meso-Dpm), alanine and glutamic acid. In the partial hydrolysates the peptides meso-Dpm-D-Ala and L-Ala-D-Glu were detected. From these data, it was concluded that both isolates show the peptidoglycan type A1 $\gamma$ meso-Dpm-direct amino acids. In agreement to these results, the peptidoglycans of *A. thermoaerophilus* (Steindl *et al.,* 2002) and *B. stearothermophilus* (Egelseer *et al.,* 1998) were confirmed to be of the A1$\gamma$-chemotype (Schleifer and Kandler, 1972).

The cell wall sugars were xylose and lower amounts of galactose and mannose (strain AFNA) or glucose and lower amounts of xylose (strain ARM). The polar lipid pattern on TLC revealed the presence of diphosphatidylglycerol (DPG), phosphatidylglycerol (PG), phosphatidylethanolamine (PE), phosphatidylcholine (PC), phospholipids (PL) and aminolipid (AL) (strain AFNA) or diphosphatidylglycerol (DPG), phosphatidylglycerol (PG), phosphatidylethanolamine (PE), phospholipid (PL) and phosphoglycolipids (PLG1-PLG3) (strain ARM) **(Figure 4).** Although PG and DPG occur in most bacteria (Giordano *et al.,* 2007); PGL relatively has limited distribution among Gram-negatives (Singleton, 2004); PEA is more common and abundant in Gram-negative bacteria and PC can be found in some Gram-negative bacteria but not in Gram-positives (Giordano *et al.,* 2007). These results are in accordance to Gram staining results.

The fatty acid profile of both isolated strains was dominated by branched fatty acids, namely iso-15:0 and iso-17:0 (strain AFNA, as well as *Aneurinibacillus thermoaerophilus* DSM $10154^T$) or iso-17:0, iso-15:0 and anteiso-17:0 (strain ARM, as well as *Bacillus stearothermophilus* DSM $22^T$).

However, strain AFNA and *Aneurinibacillus thermoaerophilus* DSM $10154^T$ as well as strain ARM and different species of the genus *Geobacillus* could be distinguished based on the relative amounts of these fatty acids **(Table 4).** The dendrograms in **Figure 5** summarize the relatedness of the whole-cell fatty acid compositions of strain AFNA and *Aneurinibacillus thermoaerophilus* DSM $10154^T$ as well as strain ARM and *Bacillus stearothermophilus* spp. Strain AFNA clustered tightly with *Aneurinibacillus thermoaerophilus* DSM $10154^T$, at a Euclidean distance of about 2.7, while strain ARM formed a cluster with different *Bacillus stearothermophilus* subgroups at a Euclidean distance of about 24. Multiple analyses and experience with the MIS program have shown that species link 10 Euclidean distance, subspecies at about 6 Euclidean distance, and strain at about 2 Euclidean distance (www.midi-inc.com). Therefore, strain ARM clearly can be distinguished as a new species while the final decision for strain AFNA can be made based on its other properties.

**Table 4. Fatty acid contents (%) of strain ARM, *Geobacillus stearothermophilus* DSM $22^T$, strain AFNA and *Aneurinibacillus thermoaerophilus* DSM $10154^T$**

| Fatty acid | Strain ARM | *Geobacillus stearothermophilus* DSM $22^T$ | Strain AFNA | *Aneurinibacillus thermoaerophilus* DSM $10154^T$ |
|---|---|---|---|---|
| iSO-$C_{13:0}$ | - | - | 0.09 | 0.07 |
| anteiso-$C_{13:0}$ | - | 5.10 | - | - |
| iso-$C_{14:0}$ | 0.17 | 0.10 | 0.29 | 0.25 |
| iso-$C_{14:0}$3OH | - | - | - | 0.10 |
| $C_{14:0}$ | 0.46 | 1.50 | 0.16 | 0.18 |
| iso-$C_{15:0}$ | 26.52 | 39.80 | 60.95 | 58.58 |
| anteiso-$C_{15:0}$ | 1.98 | 6.40 | 0.30 | 0.49 |
| $C_{15:0}$ | 0.37 | 0.50 | 0.81 | 1.33 |
| iso-$C_{16:0}$ | 6.91 | 6.20 | 3.54 | 3.56 |
| Sum in feature 3 | 1.63 | - | - | - |
| $C_{16:0}$ | 8.03 | 9.20 | 1.74 | 2.17 |
| iso-$C_{17:1}$ ω5cis | 0.60 | - | - | - |
| iso-$C_{17:1}$ ω9cis | - | - | 0.16 | 0.14 |
| Sum in feature 4 | - | - | 0.79 | 0.59 |
| iso-$C_{17:0}$ | 35.58 | 17.10 | 30.67 | 31.63 |
| anteiso-$C_{17:0}$ | 14.19 | 13.30 | 0.25 | 0.42 |
| $C_{17:0}$ | 0.52 | - | 0.26 | 0.49 |
| iso-$C_{18:0}$ | 0.44 | - | - | - |
| $C_{18:0}$ | 1.17 | - | - | - |
| iSO-$C_{19:0}$ | 1.04 | - | - | - |
| anteiso-$C_{19:0}$ | 0.40 | - | - | - |
| Summed feature 3 | 1.63 | 0.80 | - | - |
| Summed feature 4 | - | - | 0.79 | 0.59 |

## GENOTYPIC STUDIES

### Genomic DNA extraction

Genomic DNA of the pure bacterial culture was obtained by standard procedures for DNA extraction (conventional method) (Sambrook *et al.,* 1989). The selected isolate was cultivated in TSB broth overnight, and the cells harvested by centrifugation at 12,000 x g and 4°C for 10 min. The bacterial cell pellet from 10 ml culture was washed twice with 1.0 ml GTE buffer (50 mM Glucose, 25 mM Tris-HCl, 10 mM EDTA, pH 8), centrifuged at 12,000 x g and 4°C for 10 min, resuspended in 200 μl GTE buffer (pH 8.0) and transferred to a microcentrifuge tube and kept on ice for 5 min. The mixture was incubated at 37°C for 2 h in 20 μl RNase A (25 mg/ml) and 50 μl lysozyme (10 mg/ml). 50 μl Proteinase K (10 mg/ml) and 50 μl SDS (25% v/v) were added and mixed by inverting the tube several times. After incubation at 55°C for 30 min, 500 μl phenol:chloroform:isoamyl alcohol (PCI) (25:24:1) was added to the lysate and mixed well by gentle inverting several times.

The mixture was centrifuged at 12,000 x g and 4°C for 10 min until it separated into two layers. The upper phase (~ 400 μl) was transferred into a new microcentrifuge tube. The DNA was precipitated with 400 μl sodium acetate (3 M, pH 5.5) and 800 μl isopropanol and mixed well gently. This reaction mixture was incubated at room temperature for 10 min and the DNA collected by centrifugation at 12,000 x g and 4°C for 10 min. The DNA pellet was then washed with 80% ice-cold ethanol (0.5 ml) and pelleted at 12,000 x g and 4°C for 10 min, air dried and resuspended in 30 μl sterile distilled water. To detect the extracted genomic DNA, 5 μl of the extracted was electrophoresed in agarose gel (1%) with the lambda DNA digested by *Hind*III as the standard marker at 90 volt for ~ 40 min. The gel then was stained with ethidium bromide (1 μg/ml) for 10 min and after washing, DNA was visualized under UV light.

### 16S rDNA sequence identification

The 16S rDNA of desired strain was amplified by PCR using the bacterial universal primers B27F (forward; 5'-AGAGTTTGATCMTGGCTCAG, positions 8-27 based on *Escherichia coli* numbering) and U1492RM (reverse; 5'-GGYTACCTTGTTACGACTT, positions 1510-1492 based on *E. coli* numbering). The following thermal cycle was used: One cycle of predenaturation: 94°C for 4 min; 30 cycles of: 94°C for 30 s (denaturation), 60°C for 30 s (annealing) and 72°C for 1.5 min (extension); one cycle final primer extension: 72°C for 7 min. The amplified PCR product was electrophoresed in 1% (w/v) agarose gel. The desired rDNA fragment was excised with a sterile scalpel, placed in a sterile microcentrifuge tube (1.5 ml) and purified using the QIAquik Gel Extraction Kit (QIAgen, Germany), according to the manufacturer's instructions. DNA sequencing was carried out by First BASE Laboratories Sdn. Bhd. (Shah Alam, Selangor, Malaysia). The 16S rDNA sequence was compared with available 16S rDNA sequences in the NCBI nucleotide sequence database using BLAST (http://www.ncbi.nlm.nih.gov/blast) (Altschul *et al.,* 1990).

### Phylogenetic tree analysis

A phylogenetic tree was constructed based on comparison of the 16S rDNA sequence of selected isolate with those of other related strains. All the sequences were aligned in Biology WorkBench database (http://workbench.sdsc.edu). Phylogenetic tree was constructed using Molecular Evolutionary Genetics Analysis (MEGA) software version 4.1 (http://www.megasoftware.net).

### DNA-DNA hybridization and DNA base composition

DNA-DNA hybridization between desired isolate and closely related type strains as well as the determination of its DNA G+C content was performed by the DSMZ, Braunschweig, Germany. DNA was isolated using a French pressure cell (Thermo Spectronic) and was purified by chromatography on hydroxylapatite as described by Cashion *et al.* (1977). DNA-DNA hybridization was carried out as described by De Ley *et al.* (1970) under consideration of the modification described by Huss *et al.* (1983) using a model Cary 100 Bio UV/VIS-spectrophotometer equipped with a Peltier-thermostatted 6 x 6 multicell charger and a temperature controller with *in-situ* temperature probe (Varian).

After the chromosomal DNA extraction, the G+C contents were determined by using chromatography conditions adopted from Tamaoka and Komagata (1984). The DNA was hydrolysed and the resultant nucleotides were analysed by reverse-phase HPLC (Mesbah *et al.,* 1989). The HPLC system (Shimadzu Crop., Japan) consisted of the following modules: LC-20AD solvent delivery module, DGU-3A online degasser, CTO-10AC column oven, SIL-9A automatic sample injector, and a SPD-6A UV spectrophotometric detector. The analytical column was a VYDAC 201SP54, $C_{18}$, 5 μm (250 x 4.6 mm) equipped with a guard column 201GD54H (Vydac, Hesperia, CA 92345, USA). Chromatography condition was as follow: Temperature 45°C, 10 μl sample, solvent 0.3 M $(NH_4)H_2PO_4$/ acetonitrile, 40:1 (v/v), pH 4.4, 1.3 ml/min. Chromatograms were analyzed by using the CLARITY (version 2.4.1.93) software package (DataApex Ltd.,

Czech Republic).

The almost full-length 16S rDNA sequences of strain AFNA (1504 bp) and strain ARM (1563 bp) were determined **(Figures 6 and 7).** Phylogenetic analysis based on 16S rDNA sequences **(Figure 8)** showed that strain AFNA was most closely related to *Aneurinibacillus thermoaerophilus* (DSM $10154^T$) and their 16S rDNA sequences showed 99.8% similarity where phylogenetic analysis of strain ARM showed similarities between 98.8% and 99.4% to different species of genus *Geobacillus.*

DNA-DNA hybridization is one of the most important approaches to carry out molecular systematic. In 1987, an *ad hoc* committee defined the bacterial species as a group of strains with approximately 70% or more DNA relatedness. It was also recommended that phenotypic and chemotaxonomic features should agree with this definition (Wayne *et al.,* 1987). The DNA-DNA reassociation value was 81.6% (75.4%), 11.7% (5.8%) and 8.9% (18.3%) between strain AFNA and *Aneurinibacillus thermoaerophilus* DSM $10154^T$, *Aneurinibacillus migulans* DSM $2895^T$, *Aneurinibacillus aneurinilyticus* DSM $5562^T$, respectively (values in parentheses are results of measurements in duplicate) while the DNA-DNA reassociation value between strain ARM and *Geobacillus thermocatenulatus* DSM $730^T$ and *Geobacillus stearothermophilus* DSM $22^T$ was 65.3% (64.9%) and 56.8% (57.4%), respectively. DNA-DNA similarity between the isolate AFNA and *Aneurinibacillus thermoaerophilus* DSM $10154^T$ was higher than the recommended value of $\geq$ 70%, which is accepted as the definition of distinct species (Wayne *et al.,* 1987). In contrast, DNA-DNA similarity of strain ARM with *Geobacillus thermocatenulatus* and *Geobacillus stearothermophilus* was less than the recommended value. The DNA G+C content of the strain AFNA was 45.1 mol%, which was different but near to that of the type strains of *Aneurinibacillus thermoaerophilus* DSM $10154^T$, *Aneurinibacillus migulans* DSM $2895^T$ and *Aneurinibacillus aneurinilyticus* DSM $5562^T$ at 44.4 mol%, 42.9 mol% and 43.3 mol% (Goto *et al.,* 2004), respectively. DNA G+C content of the strain ARM was 51.5 mol% that was in the range of DNA G+C content of *Geobacillus* spp. at 49-58% (Nazina *et al.,* 2001).

**Description of *Geobacillus* sp. strain ARM sp. nov.**

Cells were motile by means of flagella, spore-forming rods (2.5->6.0 μm long, 0.8-1.0 μm in diameter) occurred singly, in pairs and chains, with oval/cylindrical endospores at the terminal of the cells within a swollen sporangium. They were strictly aerobic and Gram-positive, catalase-positive and oxidase-negative. Colonies were 1-2 mm in diameter, opaque, light-cream in colour, circular, convex on plate with entire margins when grown on nutrient agar. It grew at 45-70°C and pH 5.5-9.0, with optimal growth at 55°C and pH 7.0. Optimal NaCl concentration was almost 0%, but strain ARM was able to grow at NaCl concentrations up to 2% (w/v). Negative growth was observed in lysozyme broth. Acid was produced from D-glucose, D-xylose, D-mannitol and D-fructose. Acid was not produced from L-arabinose or D-lactose. Starch was hydrolysed, but Tween 80 and casein were not hydrolysed. Gelatin hydrolysis was weak and decomposition of tyrosine was negative. Lecithinase activity was negative.

The strain showed lipolytic activity; it hydrolysed a wide range of natural oils. Indole, phenylalanine deaminase and arginine dihydrolase tests were negative. Nitrate and nitrite were not reduced. Production of gas from glucose was negative. Use of citrate and propionate were positive. The major fatty acids were iso-17:0 (35.58%), iso-15:0 (26.52%), anteiso 17:0 (14.19%). Strain ARM had a cell wall type A1 $\gamma$ peptidoglycan with meso-diaminopimelic-direct amino acids. The cell wall sugars of strain ARM contained glucose and lower amounts of xylose. The polar lipid pattern on TLC revealed the presence of diphosphatidylglycerol, phosphatidylglycerol, phosphatidylethanol-amine, phospholipid and phosphoglycolipids (PLG1-PLG3). DNA-DNA reassociation value between strain ARM and *Geobacillus thermocatenulatus* DSM $730^T$ and *Geobacillus stearothermophilus* DSM $22^T$ was 65.3% (64.9%) and 56.8% (57.4%), respectively. The DNA G+C content of this strain was 51.5 mol%.

**Description of *Aneurinibacillus thermoaerophilus* strain AFNA subsp. nov.**

Cells were motile by means of flagella, spore-forming rods (3.0-5.0 μm long, 0.9-1.0 μm in diameter) occurred singly, with oval endospores at the middle of the cells within a swollen sporangium. They were strictly aerobic and Gram-negative, catalase-positive and oxidase-negative. Colonies were 2-10 mm in diameter, half-opaque, light-cream in colour, irregular, raised morphology with lobate margins when grown on nutrient agar. It grew at 45-60°C and pH 5.5-7.5, with optimal growth at 53°C and pH 6.9. Optimal NaCl concentration was almost 0%, but strain AFNA was able to grow at NaCl concentrations up to 2% (w/v). Positive growth was observed in lysozyme broth. Acid was produced from D-glucose. Acid was not produced from L-arabinose, D-xylose, D-mannitol or D-fructose. Gelatin and casein were hydrolysed, but Tween 80 and starch were not hydrolysed. No decomposition of tyrosine was observed.

The strain showed lipolytic activity; it hydrolysed olive, sesame and corn oils. Lecithinase activity was negative. Indole, phenylalanine deaminase, arginine dihydrolase tests were found to be negative. Use of citrate was negative but propionate was positive. Nitrate and nitrite were not reduced. Production of gas from glucose was negative. Assimilation of alanin, fumarat, glycerol and phenylacetate were positive but fructose, glutamate, maltose and mannitol were negative. The major fatty acids were iso-15:0 (60.95%) and iso-17:0 (30.67%). Strain AFNA has a cell wall type A1 $\gamma$ peptidoglycan

with meso-diaminopimelic-direct amino acids. The cell wall sugars of strain AFNA contained xylose and lower amount of galactose and mannose. The polar lipid pattern on TLC revealed the presence of diphosphatidylglycerol, phosphatidylglycerol, phosphatidylethanolamine, phosphatidyl-choline, phospholipid and aminolipid. DNA-DNA relatedness between strain AFNA and *Aneurinibacillus thermoaerophilus* DSM $10154^T$ was 81.6% (75.4%). The DNA G+C content of this strain was 45.1 mol%.

## CULTURE PARAMETERS OPTIMIZATION FOR THERMOSTABLE LIPASE PRODUCTION

### Lipase production medium

In order to select the best lipase production medium, eight different media (M1, A1, A2, GYP, M3, M5, TYEM, MTYEM) were tested. Bacterial inoculum (2% v/v; $Ab_{600}$ = 0.5 of overnight culture in TSB) of each selected isolate was individually inoculated into 50 ml of different production media and incubated by agitation under 150 rpm, for 48 h at 60°C. The cell free supernatant was obtained by centrifugation at 12,000 x g, 4°C for 15 min prior to lipase activity assay.

### Experimental design

A five-level-six-factor central composite rotary design (CCRD) was employed in this study, requiring 33 experiments (Cohran and Cox, 2002) (Table 5). The variables and their levels selected for the lipase production optimization were: growth temperature (45-65°C); medium volume (50-200 ml); inoculum size (1-5%); agitation rate (0-200 rpm); incubation period (24-72 h) and initial pH (5-9). The experimental data [40 points include CCRD design **(Table 5)** and optimization data (Tables 12 and 13)] were divided into three sets: training set, testing set and validating set. All tests were performed in triplicate.

### Response surface methodology analysis

The CCRD design experimental data were used for model fitting in RSM to find the best polynomial equation. These data were analyzed using Design Expert version 6.06 (Stat Ease Inc. Minneapolis, USA) and then interpreted. Three main analytical steps: analysis of variance (ANOVA), a regression analysis and the plotting of response surface were performed to establish an optimum condition for the lipase production. Then, the predicted values obtained from RSM model, were compared with actual values for testing the model. Finally, the experimental values of predicted optimal conditions were used as validating set and were compared with predicted values.

**Table 5. Experimental design used in RSM and ANN studies by using six independent variables**

| Temperature (°C) | Medium volume (ml) | Inoculum size (%) | Agitation rate (rpm) | Incubation period (h) | Initial pH |
|---|---|---|---|---|---|
| 60 | 162.5 | 4 | 50 | 60 | 6 |
| 60 | 162.5 | 2 | 150 | 36 | 8 |
| 60 | 87.5 | 4 | 50 | 60 | 8 |
| 50 | 162.5 | 2 | 150 | 60 | 8 |
| 60 | 87.5 | 4 | 150 | 36 | 8 |
| 60 | 162.5 | 2 | 50 | 60 | 8 |
| 60 | 87.5 | 2 | 150 | 36 | 6 |
| 50 | 87.5 | 4 | 50 | 36 | 8 |
| 50 | 162.5 | 2 | 50 | 36 | 8 |
| 50 | 87.5 | 4 | 150 | 60 | 8 |
| 50 | 162.5 | 4 | 150 | 60 | 6 |
| 50 | 87.5 | 2 | 50 | 36 | 6 |
| 45 | 125.0 | 3 | 100 | 48 | 7 |
| 65 | 125.0 | 3 | 100 | 48 | 7 |
| 55 | 50.0 | 3 | 100 | 48 | 7 |
| 55 | 200.0 | 3 | 100 | 48 | 7 |
| 55 | 125.0 | 1 | 100 | 48 | 7 |
| 55 | 125.0 | 5 | 100 | 48 | 7 |

(continued)

| Temperature (°C) | Medium volume (ml) | Inoculum size (%) | Agitation rate (rpm) | Incubation period (h) | Initial pH |
|---|---|---|---|---|---|
| 55 | 125.0 | 3 | 0 | 48 | 7 |
| 55 | 125.0 | 3 | 200 | 48 | 7 |
| 55 | 125.0 | 3 | 100 | 24 | 7 |
| 55 | 125.0 | 3 | 100 | 72 | 7 |
| 55 | 125.0 | 3 | 100 | 48 | 5 |
| 55 | 125.0 | 3 | 100 | 48 | 9 |
| *55* | *125. 0* | *3* | *100* | *48* | *7* |
| *55* | *125. 0* | *3* | *100* | *48* | *7* |
| *55* | *125. 0* | *3* | *100* | *48* | *7* |
| *55* | *125. 0* | *3* | *100* | *48* | *7* |
| *55* | *125.0* | *3* | *100* | *48* | *7* |
| 60 | 162.5 | 4 | 150 | 36 | 6 |
| 50 | 162.5 | 4 | 50 | 36 | 6 |
| 60 | 87.5 | 2 | 50 | 60 | 6 |
| 50 | 87.5 | 2 | 150 | 60 | 6 |
| ANN training set: normal and italic (center points) numbers; ANN testing set: bold numbers | | | | | |

In order to select the best lipase production medium, the ability of bacteria to produce lipase was tested in eight different liquid media **(Figure 9).** Lipase activity in medium A1 was significantly higher than other production media, which was composed of peptone and yeast extract as organic nitrogen sources, olive oil as carbon source and lipase production inducer, sodium and calcium as metal ions, and gum arabic as emulsifier and lipase production inducer.

Modified cubic equations (equations 5 and 6) and their subsequent ANOVA **(Tables 6 and 7)** showed quite suitable models to optimize the lipase productions. In the case of strain ARM, indeed, the modified model was a quadratic model (response surface reduced cubic model) with one eliminated (V.Ag) and one additional (T.Ag.t) terms (equations 5).

$$\textbf{Lipase activity (U ml}^{-1}\textbf{)} = 4.41 - 0.06\ T - 0.01\ V - 0.32\ IS - 0.02\ Ag - 0.07\ t + 0.11\ pH - 1.5E\text{-}4\ T^2 + 6.9E\text{-}7\ V^2 - 1.1E\text{-}3\ IS^2 - 1.3E\text{-}6\ Ag^2 + 1.7E\text{-}5\ t^2 - 0.01\ pH^2 + 9.5E\text{-}5\ T.V + 3E\text{-}3\ T.IS + 3.8E\text{-}4\ T.Ag + 1.3E\text{-}3\ T.t + 7.2E\text{-}4\ T.pH + 8.8E\text{-}4\ V.IS + 3.7E\text{-}5\ V.t + 5.6E\text{-}4\ V.pH - 2.3E\text{-}5\ IS.Ag + 1.1E\text{-}3\ IS.t + 3.3E\text{-}3\ IS.pH + 5.5E\text{-}4\ Ag.t + 1.8E\text{-}4\ Ag.pH - 1.7E\text{-}3\ t.pH - 9.7E\text{-}6\ T.Ag.t \quad \textbf{(5)}$$

where T was temperature, V was medium volume, IS was inoculum size, Ag was agitation rate, t was incubation period and pH was initial medium pH.

**Table 6. ANOVA for joint test (strain ARM)**

| **Source** | **Sum of Squares** | **DF** | **Mean Square** | **F Value** | **Prob > F** | |
|---|---|---|---|---|---|---|
| Model | 0.083 | *27* | 3.075E-003 | 1176.88 | < 0.0001 | significant |
| *A* | *1.462E-004* | *1* | *1.462E-004* | *55.96* | *0. 0007* | |
| *B* | *4.418E-005* | *1* | *4.418E-005* | *16.91* | *0.0092* | |
| *C* | *3.536E-003* | *1* | *3.536E 003* | *1353.65* | *< 0.0001* | |
| *D* | *2.278E-003* | *1* | *2.278E-003* | *872. 01* | *< 0.0001* | |
| *E* | *1.625E-003* | *1* | *1.625E-003* | *621.82* | *< 0.0001* | |
| *F* | *0.000* | *1* | *0.000* | *0.000* | *1.0000* | |

(continued)

| Source | Sum of Squares | DF | Mean Square | F Value | Prob > F | |
|---|---|---|---|---|---|---|
| $A^2$ | 3.236E-004 | 1 | 3.236E-004 | 123.86 | 0. 0001 | |
| $B^2$ | 2.173E-005 | 1 | 2.173E-005 | 8.32 | 0. 0344 | |
| $C^2$ | 2.829E-005 | 1 | 2.829E-005 | 10.83 | 0.0217 | |
| $D^2$ | 2.322E-004 | 1 | 2.322E-004 | 88.89 | 0.0002 | |
| $E^2$ | 1.457E-004 | 1 | 1.457E-004 | 55.78 | 0.0007 | |
| $F^2$ | 3.089E-003 | 1 | 3.089E-003 | 1182.37 | < 0.0001 | |
| AB | 5.105E-003 | 1 | 5.105E-003 | 1954.11 | < 0.0001 | |
| AC | 3.499E-003 | 1 | 3.499E-003 | 1339.22 | < 0.0001 | |
| AD | 2.252E-003 | 1 | 2.252E-003 | 862.12 | < 0. 0001 | |
| AE | 1.873E-003 | 1 | 1.873E-003 | 716.75 | < 0. 0001 | |
| AF | 2.059E-004 | 1 | 2.059E-004 | 78.82 | 0. 0003 | |
| BC | 5.826E-003 | 1 | 5.826E-003 | 2229.90 | < 0. 0001 | |
| BE | 4.489E-003 | 1 | 4.489E-003 | 1718.28 | < 0.0001 | |
| BF | 2.346E-003 | 1 | 2.346E-003 | 898.14 | < 0.0001 | |
| CD | 2.162E-005 | 1 | 2.162E-005 | 8.28 | 0.0347 | |
| CE | 2.992E-003 | 1 | 2.992E-003 | 1145.30 | < 0.0001 | |
| CF | 5.720E-005 | 1 | 5.720E-005 | 21.90 | 0.0054 | |
| DE | 9.310E-004 | 1 | 9.310E-004 | 356.38 | < 0.0001 | |
| DF | 1.373E-003 | 1 | 1.373E-003 | 525.44 | < 0.0001 | |
| EF | 6.529E-003 | 1 | 6.529E-003 | 2499.00 | < 0.0001 | |
| ADE | 2.820E-003 | 1 | 2.820E-003 | 1079.53 | < 0.0001 | |
| Residual | 1.306E-005 | 5 | 2.613E-006 | | | |
| Lack of Fit | 5.625E-007 | 1 | 5.625E-007 | 0.18 | 0.6932 | not significant |
| Pure Error | 1.250E-005 | 4 | 3.125E-006 | | | |
| Cor Total | 0.083 | 32 | | | | |

A: Temperature
B: Medium volume
C: Inoculum size
D: Agitation rate
E: Incubation period
F: Initial medium pH

**Table 7. ANOVA for joint test (strain AFNA)**

| Source | Sum of Squares | DF | Mean Square | F Value | Prob > F | |
|---|---|---|---|---|---|---|
| Model | 0.16 | 23 | 6.963E-003 | 84.19 | < 0.0001 | significant |
| A | 9.275E-003 | 1 | 9.275E-003 | 112.13 | < 0.0001 | |
| B | 2.657E-003 | 1 | 2.657E-003 | 32.12 | 0.0003 | |
| C | 9.800E-007 | 1 | 9.800E-007 | 0.012 | 0.9157 | |
| D | 4.205E-006 | 1 | 4. 205E-006 | 0.051 | 0.8266 | |
| E | 4.351E-004 | 1 | 4.351E-004 | 5.26 | 0.0475 | |
| F | 1.186E-003 | 1 | 1.186E-003 | 14.34 | 0.0043 | |
| $A^2$ | 0.032 | 1 | 0.032 | 391.50 | < 0.0001 | |
| $B^2$ | 0.035 | 1 | 0.035 | 426.31 | < 0.0001 | |
| $C^2$ | 0.020 | 1 | 0.020 | 246.88 | < 0.0001 | |
| $D^2$ | 0.021 | 1 | 0.021 | 258. 79 | < 0.0001 | |
| $E^2$ | 0.052 | 1 | 0.052 | 633.43 | < 0.0001 | |
| $F^2$ | 0.076 | 1 | 0.076 | 920.88 | < 0.0001 | |

(continued)

| Source | Sum of Squares | DF | Mean Square | F Value | Prob > F | |
|---|---|---|---|---|---|---|
| *AD* | *3.035E-004* | *1* | *3.035E-004* | *3. 67* | *0.0877* | |
| *AE* | *6.475E-004* | *1* | *6.475E-004* | *7.83* | *0.0208* | |
| *AF* | *6.644E-004* | *1* | *6.644E-004* | *8. 03* | *0.0196* | |
| *BC* | *3.164E-003* | *1* | *3.164E-003* | *38. 25* | *0.0002* | |
| *BE* | *7.631E-004* | *1* | *7.631E-004* | *9.23* | *0.0141* | |
| *BF* | *1.775E-004* | *1* | *1.775E-004* | *2.15* | *0.1770* | |
| *CD* | *2.207E-003* | *1* | *2.207E-003* | *26. 68* | *0.0006* | |
| *CF* | *4.435E-004* | *1* | *4.435E-004* | *5.36* | *0.0458* | |
| *DE* | *4.988E-003* | *1* | *4.988E-003* | *60.30* | *< 0.0001* | |
| *DF* | *2.335E-003* | *1* | *2.335E-003* | *28. 23* | *0. 0005* | |
| *ADE* | *0.019* | *1* | *0.019* | *230. 77* | *< 0.0001* | |
| Residual 7.444E-004 | | 9 | 8.272E-005 | | | |
| *Lack of Fit5.050E-004* | | *5* | *1.010E-004* | *1. 69* | *0.3163* | *not significant* |
| *Pure Error2.395E-004* | | *4* | *5.987E-005* | | | |
| Cor Total | 0.16 | 32 | | | | |

A: Growth temperature
B: Medium volume
C: Inoculum size
D: Agitation rate
E: Incubation period
F: Initial medium pH

The computed model F-value of 1176.88 implied the model was significant and there was only a 0.01% chance that a model F-value this large could occur due to noise. The lack of fit F-value of 0.18 implied the lack of fit was not significant relative to the pure error. There was a 69.32% chance that a lack of fit F-value this large could occur due to noise. Non-significant lack of fit showed the model was significant.

On the other hand, the pure error was very low, indicating good reproducibility of the data obtained. With very small model P-value (< 0.0001) and large lack of fit P-value (0.6932) from the analysis of ANOVA and a suitable coefficient of determination ($R^2 = 0.9998$) and adjusted coefficient of determination ($R^2_{adjusted} = 0.999$), the modified cubic polynomial model was highly significant and sufficient to represent the actual relationship between the response and the significant variables **(Table 6).**

On the other hand, in the case of strain AFNA, indeed, the modified model (response surface reduced cubic model) was a quadratic model with five eliminated (T.V, T.IS, V.Ag, IS.t, t.pH) and one additional (T.Ag.t) terms (equations 6).

$$\textbf{Lipase activity (Uml}^{-1}\textbf{)} = -1.76 + 0.04\ T + 7.60\ E\text{-}3\ V + 0.22\ IS - 0.06\ Ag - 0.09\ t + 0.65\ pH - 1.51\ E\text{-}3\ T^2 - 2.79\ E\text{-}5\ V^2 - 0.03\ IS^2 - 1.22\ E\text{-}5\ Ag^2 - 3.32\ E\text{-}4\ t^2 - 0.06\ pH^2 + 1.18E\text{-}3\ T.Ag + 2.34\ E\text{-}3\ T.t + 1.29\ E\text{-}3\ T.pH - 6.50\ E\text{-}4\ V.IS + 1.53\ E\text{-}5\ V.t + 1.54\ E\text{-}4\ V.pH - 2.35\ E\text{-}4\ IS.Ag + 9.12\ E\text{-}3\ IS.pH + 1.34\ E\text{-}3\ Ag.t + 2.42\ E\text{-}4\ Ag.pH - 2.52\ E\text{-}5\ T.Ag.t \quad \textbf{(6)}$$

where T was temperature, V was medium volume, IS was inoculum size, Ag was agitation rate, t was incubation period and pH was initial medium pH.

The computed model F-value of 84.19 implied the model was significant and there was only a 0.01% chance that a model F-value this large could occur due to noise. The lack of fit F-value of 1.69 implied the lack of fit was not significant relative to the pure error. There was a 31.63% chance that a lack of fit F-value this large could occur due to noise. Non-significant lack of fit showed the model was significant. On the other hand, the pure error was very low, indicating good reproducibility of the data obtained. With very small model P-value (< 0.0001) and large lack of fit P-value (0.32) from the analysis of ANOVA and a suitable coefficient of determination ($R^2 = 0.99$) and adjusted coefficient of determination ($R^2_{adjusted} = 0.98$), the modified cubic polynomial model was highly significant and sufficient to represent the actual relationship between the response (lipase production) and the significant variables **(Table 7).**

**Artificial neural network analysis**

A commercial ANN software, NeuralPower version 2.5 (CPC-X Software) was used throughout the study. Multilayer normal feedforward and multilayer full feedforward neural networks were used to predict the lipase activity. Networks were trained by different learning algorithms (incremental back propagation, IBP; batch back propagation, BBP; quickprob, QP; genetic algorithm, GA; and Levenberg-Marquardt algorithm, LM). The ANN architecture consisted of an input layer with six neurons, an output layer with one neuron, and a hidden layer. To determine the optimal network topology, only one hidden layer was used and the number of neurons in this layer and the transfer functions of hidden and output layers (sigmoid, hyperbolic tangent function, Gaussian, linear, threshold linear and bipolar linear) were iteratively determined by developing several networks. Each ANN was trained until the network root of mean square error (RMSE) was lower than 0.0001, average correlation coefficient (R) and average determination coefficient (DC) were equal to 1. Other ANN parameters were chosen as the default values of the software. In the beginning, weights were initialized with random values and adjusted through a training process in order to minimize network error.

The CCRD design experimental data were divided into training and testing sets. For training, 25 points were used (Table 5). One strategy for finding the best model is to summarize the data. It is well established that in ANN modeling, the replicates at center point do not improve the prediction capability of the network because of the similar inputs (Bas and Boyaci, 2007b). Hence, we improved our model by using mean of center points instead of 5 center points (Table 5, italic numbers). To test the network, 4 remaining points were used (Table 5, bold numbers). On the other hand, experimental values of predicted optimal conditions were used as validating set.

**Tables 8 and 9** have summarized the top five ANN models for strains ARM and AFNA, respectively. The best models were achieved by multilayer full feedforward architecture for both strains.

Table 8. The effect of different neural network architectures and topologies on coefficient of determination, $R^2$, and absolute average deviation, AAD, in the estimation of lipase production by strain ARM obtained in the training and testing of neural networks

| Model | Learning algorithm | Connection type | Transfer function output | Transfer function hidden | Training set $R^2$ | Training set AAD (%) | Testing set $R^2$ | Testing set AAD (%) |
|---|---|---|---|---|---|---|---|---|
| 6-16-1 | IBP[a] | MFFF[b] | Linear | Gaussian | 1 | 0.100 | 1.000 | 0.231 |
| 6-16-1 | IBP | MNFF[c] | Linear | Gaussian | 1 | 0.145 | 0.990 | 0.358 |
| 6-15-1 | IBP | MFFF | Linear | Gaussian | 1 | 0.138 | 0.953 | 0.455 |
| 6-15-1 | IBP | MNFF | Linear | Tanh[d] | 1 | 0.167 | 0.938 | 0.552 |
| 6-15-1 | IBP | MFFF | Linear | Tanh | 1 | 0.196 | 0.908 | 0.639 |

[a] Incremental Back Propagation
[b] Multilayer Full FeedForward
[c] Multilayer Normal FeedForward
[d] Hyperbolic Tangent Function

Table 9. The effect of different neural network architectures and topologies on coefficient of determination, $R^2$, and absolute average deviation, AAD, in the estimation of lipase production by strain AFNA obtained in the training and testing of neural networks

| Model | Learning algorithm | Connection type | Transfer function output | Transfer function hidden | Training set $R^2$ | Training set AAD (%) | Testing set $R^2$ | Testing set AAD (%) |
|---|---|---|---|---|---|---|---|---|
| 6-16-1 | IBP[a] | MFFF[b] | Linear | Gaussian | 1.00 | 0.20 | 0.99 | 0.08 |
| 6-16-1 | IBP | MNFF[c] | Linear | Gaussian | 0.98 | 0.22 | 0.98 | 0.12 |
| 6-15-1 | IBP | MFFF | Linear | Gaussian | 0.99 | 0.26 | 0.94 | 0.20 |

(continued)

| Model | Learning algorithm | Connection type | Transfer function output | Transfer function hidden | Training set $R^2$ | Training set AAD (%) | Testing set $R^2$ | Testing set AAD (%) |
|---|---|---|---|---|---|---|---|---|
| 6-15-1 | IBP | MNFF | Linear | Gaussian | 0.93 | 0.29 | 0.91 | 0.23 |
| 6-15-1 | IBP | MFFF | Linear | Tanh[d] | 0.97 | 0.30 | 0.89 | 0.25 |

[a] Incremental Back Propagation
[b] Multilayer Full FeedForward
[c] Multilayer Norman FeedForward
[d] Hyperbolic Tangent Function

**Verification of estimated data**

To test the estimation capabilities of the techniques, the estimated responses obtained from RSM and ANNs were compared with the observed responses. The coefficient of determination ($R^2$) and absolute average deviation (AAD) were determined and these values were used together to compare ANNs to each other for finding the best ANN model, and the best ANN model with RSM. The AAD and $R^2$ were calculated by equations 2 and 3, respectively.

$$\mathrm{AAD} = \left\{ \left[ \sum_{i=1}^{p} \left( \left| y_{i,\exp} - y_{i,cal} \right| / y_{i,\exp} \right) \right] / p \right\} \times 100 \qquad (2)$$

where $y_{i,exp}$ and $y_{i,cal}$ are the experimental and calculated responses, respectively, and p is the number of the experimental run.

$$R^2 = 1 - \frac{\sum_{i=1-n} (\text{model prediction}_i - \text{experimental value}_i)^2}{\sum_{i=1-n} (\text{average experimental value} - \text{experimental value}_i)^2} \qquad (3)$$

where n is the number of experimental data.

$R^2$ is a measure of the amount of the reduction in the variability of response obtained by using the repressor variables in the model. Since $R^2$ alone is not a measure of the model's accuracy, it is necessary to use absolute average deviation (AAD) analysis, which is a direct method for describing the deviations. Evaluation of $R^2$ and AAD values together would be better to check the accuracy of the model. $R^2$ must be close to 1.0 and the AAD between the predicted and observed data must be as small as possible. The acceptable values of $R^2$ and AAD mean that the model equation defines the true behavior of the system and it can be used for interpolation in the experimental domain (Bas and Boyaci, 2007a).

The best ANN chosen in the case of strain ARM was a 6-16-1 multilayer full feedforward incremental back propagation network with Gaussian transfer function (Table 13). The optimized values of network for learning rate and momentum were 0.15 and 0.8, respectively. The learning was completed in RMSE = 9.99E-5, R = 1 and DC = 1. In the case of training data set, the coefficient of determination ($R^2$) and absolute average deviation (AAD) were 1 and 0.1%, respectively, whereas for the testing data set, $R^2$ was 1 and AAD was 0.231% **(Table 10)** and for validating data sets $R^2$ and AAD were 0.989 and 0.059%, respectively **(Table 12).**

The best ANN chosen in the case of strain AFNA was a multilayer full feedforward incremental back propagation network with Gaussian transfer function (Table 9) that consisted of a 6-16-1 topology. The optimized values of network for learning rate and momentum were 0.15 and 0.8, respectively. The learning was completed in RMSE = 9.87E-5, R = 1 and DC = 1. In the case of training data set, the coefficient of determination ($R^2$) and absolute average deviation (AAD) were 1 and 0.20%, respectively, whereas for the testing data set, $R^2$ was 0.99 and AAD was 0.08% **(Table 11)** and for validating data sets $R^2$ and AAD were 0.98 and 1.21%, respectively **(Table 13).**

**Table 10 Actual and predicted lipase activity of strain ARM by ANN and RSM models along with absolute deviation, coefficient of determination, $R^2$ and absolute average deviation, AAD**

| **Actual activity** | **ANN predicted activity** | **ANN absolute deviation** | **RSM predicted activity** | **RSM absolute deviation** |
|---|---|---|---|---|
| 0.2063 | 0.2063 | 0.0000 | 0.2061 | 0.0010 |
| 0.0000 | 0.0000 | 0.0000 | 0.0002 | 0.0000 |
| 0.0207 | 0.0208 | 0.0048 | 0.0209 | 0.0097 |
| 0.0000 | 0.0001 | 0.0000 | 0.0002 | 0.0000 |
| 0.0000 | 0.0002 | 0.0000 | 0.0002 | 0.0000 |
| 0.0291 | 0.0291 | 0.0000 | 0.0289 | 0.0069 |
| 0.0000 | 0.0000 | 0.0000 | 0.0002 | 0.0000 |
| 0.0886 | 0.0886 | 0.0000 | 0.0888 | 0.0023 |
| 0.0724 | 0.0724 | 0.0000 | 0.0722 | 0.0028 |
| 0.0000 | 0.0000 | 0.0000 | 0.0002 | 0.0000 |
| 0.0907 | 0.0908 | 0.0011 | 0.0909 | 0.0022 |
| 0.1945 | 0.1945 | 0.0000 | 0.1947 | 0.0010 |
| 0.0400 | 0.0400 | 0.0000 | 0.0400 | 0.0000 |
| 0.0229 | 0.0227 | 0.0087 | 0.0229 | 0.0000 |
| 0.0457 | 0.0457 | 0.0000 | 0.0457 | 0.0000 |
| 0.0551 | 0.0552 | 0.0018 | 0.0551 | 0.0000 |
| 0.0000 | 0.0000 | 0.0000 | 0.0000 | 0.0000 |
| 0.0841 | 0.0840 | 0.0012 | 0.0841 | 0.0000 |
| 0.0675 | 0.0677 | 0.0030 | 0.0675 | 0.0000 |
| 0.0000 | 0.0000 | 0.0000 | 0.0000 | 0.0000 |
| 0.0851 | 0.0851 | 0.0000 | 0.0851 | 0.0000 |
| 0.0281 | 0.0280 | 0.0044 | 0.0281 | 0.0000 |
| 0.0000 | 0.0000 | 0.0000 | 0.0000 | 0.0000 |
| 0.0000 | 0.0000 | 0.0000 | 0.0000 | 0.0000 |
| *0.0467* | *0.0467* | *0.0000* | *0.0465* | *0.0043* |
| **0.0000** | **0.0010** | 0.0000 | 0.0002 | 0.0000 |
| **0.0693** | **0.0692** | 0.0014 | 0.0691 | 0.0029 |
| **0.0792** | **0.0795** | 0.0038 | 0.0794 | 0.0025 |
| **0.0996** | **0.0992** | 0.0040 | 0.0994 | 0.0020 |

ANN training set $R^2$ = 1
ANN training set AAD = 0.1%
RSM $R^2$ = 1
RSM AAD = 0.13%
**ANN testing set $R^2$ = 1**
**ANN testing set AAD = 0.23%**
ANN training set: normal and italic (center points) numbers
ANN testing set: bold numbers

**Table 11: Actual and predicted lipase activity of strain AFNA by ANN and RSM models along with absolute deviation, coefficient of determination, R and absolute average deviation, AAD**

| **Actual activity. ($Uml^{-1}$)** | **ANN predicted activity ($Uml^{1}$)** | **ANN absolute deviation** | **RSM predicted activity ($Uml^{-1}$)** | **RSM absolute deviation** |
|---|---|---|---|---|
| 0.09 | 0.09 | 0.00 | 0.09 | 0.00 |
| 0.16 | 0.16 | 6.42E-05 | 0.15 | 0.04 |
| 0.07 | 0.07 | 0.00 | 0.07 | 0.11 |
| 0.16 | 0.16 | 0.00 | 0.16 | 0.01 |
| 0.11 | 0.11 | 0.00 | 0.11 | 0.01 |
| 0.09 | 0.09 | 0.00 | 0.09 | 0.00 |
| 0.08 | 0.08 | 0.00 | 0.08 | 0.12 |
| 0.09 | 0.09 | 0.00 | 0.08 | 0.13 |
| 0.07 | 0.07 | 4.47E-07 | 0.07 | 0.06 : |
| 0.09 | 0.09 | 0.00 | 0.09 | 0.07 |
| 0.09 | 0.09 | 0.00 | 0.09 | 0.03 |
| 0.08 | 0.08 | 4.62E-05 | 0.08 | 0.05 |
| 0.18 | 0.18 | 0.00 | 0.18 | 0.00 |
| 0.04 | 0.04 | 0.00 | 0.04 | 0.00 |
| 0.07 | 0.07 | 0.00 | 0.07 | 0.00 |
| 0.14 | 0.14 | 6.00E-05 | 0.14 | 0.00 |
| 0.14 | 0.14 | 8.78E-05 | 0.14 | 0.00 |
| 0.14 | 0.14 | 0.00 | 0.14 | 0.00 |
| 0.14 | 0.14 | 0.00 | 0.14 | 0.00 |
| 0.14 | 0.14 | 0.00 | 0.14 | 0.00 |
| 0.08 | 0.08 | 0.00 | 0.08 | 0.00 |
| 0.05 | 0.05 | 0.00 | 0.05 | 0.00 |
| 0.05 | 0.05 | 0.00 | 0.05 | 0.00 |
| 0.00 | 0.00 | 0.03 | 0.00 | 4.66E-14 |
| *0.26* | *0.26* | *7.30E-05* | *0.26* | *0.00* |
| **0.06** | **0.06** | 6.63E-05 | 0.05 | 0.02 |
| **0.09** | **0.09** | 0.00 | 0.10 | 0.04 |
| **0.06** | **0.06** | 0.00 | 0.05 | 0.14 |
| **0.10** | **0.10** | 0.00 | 0.09 | 0.05 |

ANN training set $R^2 = 1$
ANN training set AAD (%) = 0.20
RSM$R^2 = 0.99$
RSM AAD (%) = 3.11
**ANN testing set $R^2 = 0.99$**
**ANN testing set AAD (%) = 0.08**
ANN training set: normal and italic (center points) numbers
ANN testing set: bold numbers

**Table 12 : Solution of optimum condition for strain ARM**

| Growth temperature (°C) | Medium volume (ml) | Inoculum size (%) | Agitation rate (rpm) | Incubation period (h) | Initial pH, | ANN predicted activity ($Uml^{-1}$) | Actual activity ($Uml^{-1}$) | RSM predicted activity ($Uml^{-1}$) |
|---|---|---|---|---|---|---|---|---|
| 52.3 | 50 | 1.0 | 0 | 24.0 | 5.8 | 0.47 | 0.47 | 0.48 |
| 50.0 | 50 | 2.0 | 10 | 24.0 | 6.0 | 0.45 | 0.45 | 0.46 |
| 48.0 | 50 | 1.4 | 0 | 33.6 | 5.8 | 0.44 | 0.44 | 0.44 |
| 48.0 | 50 | 1.4 | 10 | 33.6 | 5.7 | 0.42 | 0.42 | 0.43 |
| 48.0 | 50 | 1.4 | 20 | 33.6 | 5.8 | 0.42 | 0.42 | 0.42 |
| 49.0 | 50 | 1.0 | 40 | 33.6 | 6.0 | 0.42 | 0.42 | 0.41 |
| 48.0 | 50 | 1.5 | 20 | 33.6 | 5.8 | 0.41 | 0.41 | 0.41 |

ANN $R^2$ = 0.99 RSM    RSM $R^2$ = 0.95
ANN AAD = 0.06%    RSM AAD = 0.08%

**Table 13: Solution of optimum condition for strain AFNA**

| Growthtemperature (°C) | Medium volume (ml) | Inoculum size (%) | Agitation rate (rpm) | Incubation period (h) | Initial pH | ANN predicted activity ($Uml^{-1}$) | Actual activity ($Uml^{-1}$) | RSM predicted activity ($Uml^{-1}$) |
|---|---|---|---|---|---|---|---|---|
| 52.70 | 133.11 | 2.95 | 102.70 | 48.00 | 6.89 | 0.28 | 0.28 | 0.27 |
| 50.90 | 146.20 | 2.50 | 57.31 | 45.00 | 7.02 | 0.24 | 0.25 | 0.25 |
| 52.93 | 130.50 | 3.43 | 97.31 | 54.04 | 6.90 | 0.25 | 0.24 | 0.25 |
| 51.70 | 132.75 | 2.22 | 94.79 | 49.40 | 6.52 | 0.25 | 0.29 | 0.26 |
| 50.11 | 119.90 | 3.01 | 130.58 | 43.33 | 6.43 | 0.21 | 0.22 | 0.22 |
| 56.76 | 143.74 | 3.26 | 65.15 | 41.03 | 6.67 | 0.19 | 0.19 | 0.19 |
| 57.64 | 132.82 | 3.83 | 62.01 | 53.33 | 7.75 | 0.17 | 0.17 | 0.17 |

ANN $R^2$ = 0.98 RSM $R^2$ = 0.97
ANN AAD (%) = 1.21 RSM AAD (%) = 2.17

Comparison of predicted and experimental values in training, testing and validating data sets, not only revealed capability of ANN in prediction of known data responses (the data that have been used for training) but also showed the ability of generalization for unknown data (the data that have not been used for training) and implying that empirical models derived from ANN can be used to adequately describe the relationship between the input factors and lipase production.

ANOVA analysis (Table 6) and the three dimensional plots **(Figure 10)** revealed that growth temperature, medium volume, inoculum size and incubation period had significant effects on lipase production. ANOVA analysis shows that although pH was not a significant parameter (P value > 0.05), it had important and significant interactions with other parameters, hence it has been used to develop the model. On the other hand, among the different interactions, interaction between agitation rate and growth medium volume, did not show significant effect on lipase production (P value > 0.05).

**Figure 10B** depicts that lipase activity effectively increased with a decrease in growth volume but agitation rate did not show significant effect on lipase production. On the other hand, ANOVA analysis and **Figure 10C** reveal that agitation was one of the most important parameters for lipase production. As a conclusion, though both agitation rate and growth medium volume parameters were significant, yet their interaction was not a significant parameter for lipase production. Hence modification of model via the removal of this interaction using backward elimination strategy improved the model (Equation 5).

**Figure 10A** represents the three dimensional plot as function of temperature and inoculum size on lipase activity. Maximum lipase activity of 0.47 $Uml^{-1}$ was obtained at the 52.3°C and 1% inoculum size. Further increase or decrease in the temperature, and increase in the inoculum size led to the decrease in the enzyme production.

**Figures 10B and 10C** depict the medium volume-agitation rate and initial pH-agitation rate interactions, respectively. These plots reveal that the lipase activity increased with a decrease in culture volume and agitation rate. The maximum lipase activity was obtained at 50 ml culture volume and moderately acidic pH (5.8), under static condition. Similarly, static condition had resulted in comparatively high lipase production for *Pseudomonas* sp. strain S5 (Rahman *et al.,* 2006), *Syncephalastrum racemosum* (Chopra and Chander, 1983) and *Pseudomonas aeruginosa* (Nadkarni, 1971). Basically, suitable agitation lead to sufficient supply of dissolved oxygen in the media (Kumar and Takagi, 1999). Nutrient uptake by bacteria also will be increased (Beg *et al.,* 2003), but the degree of aeration appears to be critical in some cases since shallow layer (static) cultures (moderate aeration) produced much more lipase than shake cultures (high aeration) (Rahman *et al.,* 2006).

The medium volume may have a great effect on the enzyme production. Although a larger medium volume initially contains more oxygen, nutrients and space for growth of bacteria, the void in the container and subsequently oxygenation of the medium will be decreased. On the other hand, it seems that ratio of surface area to volume (A/V) is important for lipase production where higher ratio cause higher oxygenation and lipase production (Woolley and Petersen, 1994). The combined effect of initial pH and incubation time on lipase production is shown in **Figure 10D.** According to the plot, a moderately acidic initial pH (5.8) caused maximum lipase production after 24 h of cultivation. The activity was decreased remarkably as the incubation period changed. pH plays an important role in all the biological processes.

***Aneurinibacillus thermoaerophilus*** **strain AFNA**

**Figure 11A** represents the three dimensional plot as function of growth temperature and inoculum size on lipase activity when other parameters were kept at optimum point.

Maximum lipase activity of 0.28 $Uml^{-1}$ was obtained at the 52.7°C and 2.9% inoculum size. Further increase or decrease in these parameters led to the decrease in the enzyme production. Figure 11B depicts the interaction between medium volume and agitation rate. The maximum lipase activity was obtained at 133.1 ml culture volume and 103 rpm agitation rate. The combined effect of growth medium initial pH and incubation time on lipase production is shown in **Figure 11C.** According to the plot, a neutral initial pH (6.9) caused maximum lipase production after 48 h of cultivation. The activity decreased remarkably as the parameters changed.

The importance of effective parameters on the lipase production is shown in **Figure 12.** In the case of strain ARM (A) inoculum size of 18.15% was the most important factor on the lipase production, incubation period of 17.01%, agitation rate of 16.78%, growth temperature and medium volume of 16.46% and 16.44%, respectively and pH of 15.19% were subsequent degrees of importance. On the other hand, in the case of strain AFNA (B) pH with 18.23% was the most important factor on the lipase production, growth temperature with 17.66%, medium volume with 17.43%, incubation period with 16.94%, agitation rate with 16%, and inoculum size with 13.73% were subsequent degrees of importance.

The optimal conditions for lipase production were predicted as presented in Tables 12 and 13 along with their predicted and actual values. Among the various optimum conditions, in the case of strain ARM, the highest lipase activity (0.47 $Uml^{-1}$; 4.7-fold increase) was obtained at following condition: growth temperature (52.3°C), medium volume (50 ml), inoculum size (1%), agitation rate (static condition), incubation period (24 h) and initial pH (5.8). In the case of strain AFNA, the highest lipase activity (0.28 $Uml^{-1}$; 1.85-fold increase) was obtained at following condition: growth temperature (52.7°C), medium volume (133.1 ml), inoculum size (2.9%), agitation rate (103 rpm), incubation period (48 h) and initial pH (6.9). Attention to $R^2$ and AAD values between actual and estimated responses demonstrated a higher prediction accuracy of ANN compared to RSM.

Polyphasic taxonomy studies revealed that strain ARM was a novel species of the genus *Geobacillus,* while strain AFNA was a novel subspecies of the *Aneurinibacillus thermoaerophilus.* Since the species is the basic unit and certainly the most important and the central element of bacterial taxonomy, the study of strain ARM and its products including industrially important enzymes can be interesting for the microbiologists, biochemists and biotechnologists: On the other hand, analyzing and modeling of lipase production using RSM and ANN methods showed that strain ARM was a better thermostable lipase producer than strain AFNA. Hence, strain ARM was selected as a novel species and subsequently new lipase producer for further studies. The full length of ARM thermostable lipase gene was successfully amplified using primer walking strategy and sequenced **(Figures 13 and 14).**

Phylogenetic analysis confirmed that this gene was a new lipase gene and was related to the thermostable lipases from the thermophilic *Geobacillus* spp. **(Figure 15).** Thus, the sequence was submitted in National Center for Biotechnology Information (NCBI) Genbank database (http://www.ncbi.nih.gov) as ARM lipase gene with accession number EF042975. The entire 1588 bp containing the putative lipase gene was analysed including a single open reading frame (ORF) comprising 1257 bp extending from 93 to 1349 (Figure 14). Putative -35 (ttgggt) from 16 to 21 and -10 (tggtaat) from 39 to 45, promoter sequences, were observed as well as a ribosomal binding site (RBS) (aggagagg) from 79 to 86 and stem loop structure (aaaaaagc gctttttt) from 1365 to 1372 and from 1390 to 1397, downstream from the stop codon (taa). In addition, a signal peptide sequence (mmkccrrval vllglwfvfc isvlggraea) was detected from 93 to 183. Gierasch (1989) has stated that signal peptide is usually composed of 13 to 36 amino acids having at least one positively charged residue followed by a hydrophobic core of 10 to 15 residues and end with a small, neutral residue, often Ala.

The deduced mature lipase was composed of 388 amino acids, which correspond to a molecular weight of 43.3 kDa. **Figure 16** shows The pairwise alignment of amino acid sequence of mature ARM lipase with thermostable lipases from *Geobacillus kaustophilus* HTA426, *Geobacillus stearothermophilus* (TW1) and *Geobacillus zalihae* (T1)

**Sequencing, cloning and expression of the thermostable lipase gene**

**Amplification, sequencing and analysis of the thermostable lipase gene**

In order to amplify the specific sequence encoding the thermostable lipase gene, sets of oligonucleotide primers were designed **(Table 15)** based on significant gene sequences sharing and structural similarities of *Bacillus* spp. thermostable lipases. The following thermal cycle was used: One cycle of pre-denaturation: 94°C for 4 min; 30 cycles of: 94°C for 1 min (denaturation), X°C (based on primer set $T_m$) for 1 min (annealing) and 72°C for 1.5 min (extension); one cycle final primer extension: 72°C for 7 min. The amplified PCR product was electrophoresed in 1% (w/v) agarose gel. The desired lipase gene fragment was excised with a sterile scalpel, placed in a sterile microcentrifuge tube (1.5 ml) and purified using the QIAquik Gel Extraction Kit (QIAgen, Germany), according to the manufacturer's instructions.

DNA sequencing was carried out by First BASE Laboratories Sdn. Bhd. (Shah Alam, Selangor, Malaysia). According to the sequencing result, new set of primers were designed and the previous steps were repeated (primer walking). This process was repeated several times to achieve the complete lipase gene. The homology search was performed with the Basic Local Alignment Search Tool (BLAST) of National Center for Biotechnology Information (NCBI) Genbank database, (http://www.ncbi.nlm.nih.gov/blast) (Altschul *et al*., 1990). Phylogenetic tree was constructed using Molecular Evolutionary Genetics Analysis (MEGA) software version 4.1 (http://www.megasoftware.net). The open reading frame was predicted by NCBI ORF Finder (http://www.ncbi.nlm.nih.gov/gorf/gorf.html). The putative signal peptide and its cleavage site was predicted by the SignalP (a neural network for predicting signal peptides) V3.0 (http://www.cbs.dtu.dk/services/SignalP) (Emanuelsson *et al*., 2007).

**Table 15. Oligonucleotides used as primers for amplification of the *Bacillus* sp. thermostable lipase gene**

| Oligonucleotide | Nucleotide sequence |
|---|---|
| ORF F | 5'-ATGATGAAAGGCTGTCGGATTATG-3' |
| ORF R | 5'-TTAAGGCCGCAAACTCGCCAAC-3' |
| MD F | 5'-ATGATGAAAGGCTGCCGGGTGATGGTT-3' |
| MD R (PIR1) | 5'-TTAAGGCTGCAAGCTCGCCAACTGCTC-3' |

(continued)

| Oligonucleotide | Nucleotide sequence |
|---|---|
| lip F1 | 5'-GGCTGAACGACAACGGTTATC-3' |
| lip R1 | 5'-TCAATGCCGAGCGTCGGATT-3' |
| lip F | 5'-TCAYMRRAAAACCCGACAATTG-3' |
| lip R | 5'-CCCATCCGCTTGCTTTGCCCTTATTAT-3' |
| lip F 30 | 5'-GCATCCCTACGCGCCCATGAT-3' |
| lip R 30 | 5'-TTAGGNTGCNAGCTTGCCAACTGC-3' |

**Preparation of competent cells**

A single colony of *E. coli* TOP10 cells (Invitrogen, USA) from a fresh LB agar was inoculated in 5 ml LB broth. The culture was incubated at 37°C with shaking at 150 rpm. One ml of the overnight culture was added to 50 ml LB broth medium and shaken at 37°C until $A_{6oo}$ = 0.5 was reached. The culture was cooled on ice for 5 min, and then transferred to round-bottom centrifuge tubes. The cells were collected after low speed centrifugation (5 min at 4,000 x g and 4°C). The supernatant was carefully discarded, and the tubes kept on ice. The cells were resuspended in cold (4°C) TFB1 buffer (15 ml for a 50 ml culture) and kept on ice for 60 min. Then they were harvested by centrifugation (5 min at 4,000 x g and 4°C). The supernatant was carefully discarded, and the cells were kept on ice. The cells were resuspended in 4 ml ice-cold TFB2 buffer. Aliquots of 100 µl were prepared in sterile microcentrifuge tubes and stored at -80°C (Sambrook *et al.,* 1989).

**Cloning and transformation of the thermostable lipase gene**

The purified PCR product of mature thermostable lipase gene (without signal peptide) using designed ARM mature lipase primers: forward 5'-GCGGCTTCGCGAGCCAAC GAT-3' and reverse 5'-TTAAGGTTGCAAGCTCGCCAACTGC-3', was cloned into the pTrcHis TOPO vector using the pTrcHis TOPO TA expression kit (Invitrogen, USA), according to the manufacturer's instructions (catalog no. K4410-01, version I, 2001). The fresh PCR product (4 µl) was mixed gently with 1 µl pTrcHis TOPO vector (10 ng/µl) and incubated for 5 min at room temperature (~25°C). The TOPO cloning reaction (2 µl) was added into a vial of TOP10 competent cells gently and incubated on ice for 30 min.

The cells were heat shocked for 30 s at 42°C without shaking and the vial immediately was transfered to ice. Then 250 µl of room temperature SOC medium was added and the vial was shaked horizontally at 37°C for 30 min. The transformed cells (50 µl) were spread on a prewarmed LB tributyrin agar plate containing 50 µg/ml ampicillin and 0.5% glucose (as selection plate) and incubated overnight at 37°C.

**Analysis of the positive colonies**

The positive transformed colonies (with clear surrounding zone on ampicillin tributyrin agar plate) were subcultured (as master plate). The presence of the lipase gene (insert) was confirmed by lipase gene amplification (PCR) and sequencing after the plasmid extraction.

**Expression of the recombinant thermostable lipase gene**

A transformed colony, harboring thermostable lipase gene was cultured in 5 ml LB broth medium containing 50 µg/ml ampicillin and incubated overnight at 37°C with 150 rpm shaking. Blue cap media-lab bottle (1000 ml) containing 200 ml LB broth medium was autoclaved and supplemented with 50 µg/ml ampicillin. LB medium was inoculated with two ml of precultured cells. The culture was grown at 37°C with 150 rpm shaking to $A_{600}$ = 0.5 (mid-log phase). Aliquot of 10 ml from the culture was taken and kept. This aliquot was considered to be as zero time. Then the culture was induced with a final concentration of 1.0 mM Isopropyl-β-D-thiogalactopyranoside (IPTG) and grown at 37°C with 150 rpm shaking. Samples of 10 ml were taken every 3 h for 30 h to assess gene expression.

The aliquots were centrifuged at 10,000 x g for 10 min at 4°C and the pellet was frozen at - 20°C. The 10 ml pellets were resuspended in 10 ml 50 mM phosphate buffer prior to sonication (Branson 250 sonifier: 20 cycles of intermittent sonication at 25 W for 30 s in an ice bath). The cell extracts were obtained by centrifugation at 12,000 x g for 10 min at 4°C. The lipase activities of supernatants (as the intracelullar lipase activity) were assayed. The same process was performed for negative control cells, transformed with vector (pTrcHis) without insert (lipase gene).

**Optimization of the expression inducer concentration**

In order to find the optimum concentration of IPTG, the expressions were performed for 18 h (optimal expression time), 37°C under 150 rpm shaking with different concentrations of IPTG (0.1-1.5 mM).

**Secretory expression of ARM thermostable lipase**

The bacteriocin release protein (BRP) expression vector, pJL3 (Mobitech, Germany), was used for secretory expression. Competent cells of *E. coli* TOP10, strain harboring the ARM lipase expression vector-recombinant pTrcHis (pTrcHis/ARM) were prepared by using conventional calcium transformation procedure (Miller, 1972). The pJL3 plasmid was then co-transformed into competent cells for extracellular expression. The transformants were selected on LB plates containing chloramphenicol (34 μg/ml) and ampicillin (50 μg/ml) for pJL3 and pTrcHis/ARM plasmids, respectively.

**Optimization of secretory expression of ARM thermostable lipase**

The optimization study was carried out by inoculating ($Ab_{600}$ = 0.5) recombinant cultures harboring pTrcHis/ARM and pJL3 vectors in 200 ml LB broth containing chloramphenicol (34 μg/ml) and ampicillin (50 μg/ml) into 1 L blue cap bottle. The intra- and extracellular fractions were obtained as described below. Culture (10 ml) was centrifuged at 10,000 x g for 10 min at 4°C. The supernatant was analyzed for extracellular ARM lipase activity. The pellet was resuspended with equal volume of 50 mM of sodium phosphate buffer (pH 7.0) prior to sonication (Branson 250 sonifier: 20 cycles of intermittent sonication at 25 W for 30 s in an ice bath) and cleared by centrifugation (12,000 x g, 10 min, 4°C). The clear crude lysate was used to measure the intracellular lipase activity. For finding the optimum induction time and concentration of inducer (IPTG), the cells were induced with different concentration of IPTG (0-100 μM) at $Ab_{600}$ = 0.5 for 0, 4, 8, 12, 20, 28, and 32 h of induction times, 37°C under 150 rpm shaking.

**Purification of the recombinant thermostable lipase**

The recombinant thermostable lipase was purified using the immobilized metal ion affinity chromatography (ProBond™ Purification System (Invitrogen)) following the manufacturer's purification protocol for native conditions (Catalog nos. K850-01, K851-01, K852-01, K853-01, K854-01, R801-01, R801-15, Version K, 2004).

**Crude enzyme preparation**

A transformed colony harboring thermostable lipase gene was cultured in 5 ml LB broth medium containing 50 μg/ml ampicillin and incubated overnight at 37°C with 150 rpm shaking. Supplemented LB broth medium (200 ml) with 50 μg/ml ampicillin, was inoculated with 2 ml of precultured cells and grown at 37°C with 150 rpm shaking to $A_{6oo}$ = 0.6 (mid-log phase). Then the culture was induced with a final concentration of 1.0 mM Isopropyl-β-D-thiogalactopyranoside (IPTG) and grown at 37°C with 150 rpm shaking for 18 h. The cells were harvested by centrifugation at 10,000 x g for 10 min at 4°C and resuspended in 50 ml native binding buffer (50 mM $NaH_2PO_4$, pH 8 containing 0.5 M NaCl and 10 mM imidazole). The cells were lysed by 20 cycles of intermittent sonication at 25 W for 30 s in an ice bath. The disrupted cells were removed by centrifugation at 10,000 x g and 4°C for 15 min and the supernatant was filtered through a 0.45 μm membrane filter.

**The purification procedure**

Purification was performed at 4°C using 10 ml purification column filled with 2 ml of ProBond™ Nickel-Chelating Resin. The resin was washed with 10 volumes of distilled water followed by equilibration with 10 volumes of native binding buffer (50 mM $NaH_2PO_4$, pH 8 containing 0.5 M NaCl and 10 mM imidazole). Five ml of the crude enzyme preparation were loaded in the column. The column was washed with 24 volumes of native wash buffer (50 mM $NaH_2PO_4$, pH 8 containing 0.5 M NaCl and 20 mM imidazole). The binding protein was eluted off the column in 1 ml fractions with native elution buffer (50 mM $NaH_2PO_4$, pH 8 containing 0.5 M NaCl and 250 mM imidazole). Total protein was assessed by the Bradford method (Stoscheck, 1990) using the Bio-Rad assay reagent and bovine serum albumin as standard according to the manufacturer's instructions. The purity of the protein fractions were analyzed using SDS-PAGE (Laemmli, 1970).

The mature ARM lipase gene was amplified and cloned in pTrcHis expression system (Invitrogen, USA) and transformed into *E. coli* TOP10 host cells. The formation of three positive transformed colonies (with clear surrounding zone on tributyrin agar plate; **Figure 17)** indicated a putative positive transformation. The lipase gene amplification and sequencing after the plasmids extraction confirmed the presence of the insert (ARM lipase gene) in the positive colonies in a correct direction and frame. The removal of signal peptide is necessary to improve the expression level since high hydrophobic region of signal peptide might contribute to the aggregation of fusion protein as inclusion body. Furthermore, this region does not confer structural function of ARM lipase. Besides, it cannot be recognized by the host *E. coli* to secrete out fusion lipase (van den Burg, 2003; Rahman *et al.,* 2005a).

The ability of the positive transformed colonies for expression of ARM recombinant thermostable lipase gene was tested. The optimum expression was detected at 18 h incubation after induction by 1.0 mM IPTG **(Figure 18)** where lipase activity was 162.3 $Uml^{-1}$ and approximately 1623-fold higher than the wild-type in standard condition (2% inoculum size in 50 ml A1 production medium at 60°C and 150 rpm for 48 h). In the negative control, cells transformed with pTrcHis vector without lipase gene, no lipase production could be detected. Since each recombinant protein has different characteristics that may affects its optimum expression, it is helpful to determine the best condition for maximizing the expression of the protein. The effect of different concentration of IPTG on recombinant thermostable ARM lipase gene expression was investigated. The optimal concentration of IPTG for the highest lipase production, after 18h of induction (162.45 $Uml^{-1}$) was 1 mM **(Figure 19).** ARM thermostable lipase gene expression in the pTrcHis system was 162.3 $Uml^{-1}$, 219.31 $Umg^{-1}$, in standard assay condition (50°C, pH 7.0 Phosphate buffer, 200 rpm with 20 $\mu$l of 20 mM $CaCl_2$ in reaction mixture, using olive oil as substrate) and 512 $Uml^{-1}$, 690 $Umg^{-1}$ in optimized assay condition (65°C, pH 8.0 Tris-HCl, 200 rpm, without $CaCl_2$ in reaction mixture, using olive oil as substrate.

Therefore, ARM thermostable lipase gene expression in the pTrcHis system was one of the most effective expression systems as compared to other reported thermostable lipases so far in prokaryotic system such as: solvent-stable thermostable lipase from *Bacillus* sp. strain 42 in pET51b (80.0 $Uml^{-1}$, 160.0 $Umg^{-1}$), pQE-30UA (17 $Uml^{-1}$, 34 $Umg^{-1}$ protein) (Rahman *et al.,* 2008); thermostable lipase from *Geobacillus zalihae* (T1 lipase) in the pBAD (8.76 $Umg^{-1}$), pRSET C (6.41 $Umg^{-1}$), pET22b(+) (2.54 $Umg^{-1}$) and pGEX (20.02 $Umg^{-1}$) (Leow *et al.,* 2004), thermostable lipase from *Bacillus thermoleovorans* ID-1 in pET22b(+) and pGEX (320 U/g cells) (Cho *et al.,* 2000); thermostable lipase from *Bacillus thermocatenufatus* in pLIP2 (4 $Umg^{-1}$) (Schmidt-Dannert *et al.,* 1996); thermostable lipase from *Bacillus licheniformis* in pET20b(+) (1.6 $Umg^{-1}$) (Nthangeni *et al.,* 2001); thermostable lipase from *Bacillus stearothermophilus* P1 in pREP4 (46 $Umg^{-1}$) (Sinchaikul *et al.,* 2001b). In addition, the expression of ARM lipase was even better than some expressed thermostable lipases in yeast system such as: thermostable lipase from *Bacillus* sp. strain L2 into a *Pichia pastoris* expression vector (125 $Uml^{-1}$) (Sabri *et al.,* 2009); *Galactomyces geotrichum* Y05 thermostable lipase in methylotrophic *Pichia pastoris* GS115 (100.0 $Uml^{-1}$) (Yan *et al.,* 2007); *Bacillus thermocatenulatus* BTL2 in *Pichia pastoris* (35 $Umg^{-1}$) (Quyen *et al.,* 2003) and thermostable lipase from *Streptomyces fradiae* var. k11 in *Pichia pastoris* (154 $Umg^{-1}$) (Zhang *et al.,* 2008).

The effects of various concentrations of IPTG and time on extracellular expression of ARM lipase are shown in **Figure 20 and Figure 21,** respectively. IPTG at 50 $\mu$M was the most efficient concentration for lipase secretion with in *E. coli* TOP10 host strain, in which 10610.46 U (72.8%) of lipase was secreted in culture medium after 16 h of post induction incubation time. Further increase in the IPTG concentration led to the decrease in the enzyme production.

On the other hand, almost all of the produced lipases could easily leak to the medium upon induction with 75 and 100 $\mu$M of IPTG. These results implied that the host (*E. coli* TOP10) encountered quasi-lysis upon induction with high concentration of IPTG. These findings are in accordance to previous report by Rahman *et al.* (2005a). The effect of post-induction time in BRP expression on ARM lipase release was investigated (Figure 35). Initially, the ARM lipase was in abundance intracellularly but higher amount of ARM lipase activity was detected in culture medium after 12 h of induction time, reaching a maximum level at 16 h (10610.46 U).

In order to facilitate the recombinant lipase purification, ARM thermostable lipase was expressed with a six-His-tag at its C-terminus end in *E. coli.* The His-tagged enzyme was purified using immobilized metal affinity chromatography (IMAC). Non-specific binding between the $Ni^{2+}$ and non-tagged proteins was reduced by inducing a low concentration of imidazole and NaCl to prevent undesirable ionic interactions. After removing unwanted proteins, the recombinant thermostable lipase was eluted using high concentration of imidazole. Chromatogram, purification table and SDS-PAGE results were shown in **Figure 22, Table 16 and Figure 23,** respectively.

**Table 20. Purification table. Summary of the His-tagged recombinant ARM thermostable lipase purification**

| Purification Step | Protein concentration (mg) | Total activity (U) | Specific activity ($Umg^{-1}$) | Purification factor | Recovery (%) |
|---|---|---|---|---|---|
| **Crude** | 3.735 | 579.4 | 155.13 | 1 | 100 |
| **IMAC** | 0.162 | 366.1 | 2258.48 | 14.6 | 63.2 |

The purified lipase was confirmed to be homogenous by a prominent single band on SDS-PAGE **(Figure 23)** with an overall yield of 63.2% and a purification fold of 14.6 **(Table 16).** A comparison of the cell lysate (Figure 23, Lane 2) with the flow through fraction (Figure 23, Lane 3) demonstrated that the matrix worked effectively to adsorb the His-tagged protein. As a conclusion, IMAC was an efficient and rapid strategy to purify His-tagged recombinant ARM thermostable lipase in one step purification procedure. In comparison, recombinant His-tagged lipases such as, SGNH lipase of *Streptomyces coelicolor* (Bielen *et al.,* 2009); an organic solvent tolerant lipase from *Pseudomonas fluorescens* JCM5963 (Zhang *et al.,* 2009) and thermostable lipase from *Geobacillus thermoleovorans* YN (Soliman *et al.,* 2007) were purified in two steps using IMAC followed by gel filtration with fold/overall yield of 34.1/21.4%, 3.5/53.5% and 130/2.1%, respectively.

**Characterization of the purified recombinant thermostatble lipase**

**Determination of ARM lipase molecular weight**

**SDS-polyacrylamide gel electrophoresis**

After protein purification, the purity of the protein fractions and their molecular weights were analyzed using SDS-PAGE (Laemmli, 1970). Each protein sample was mixed with 5X sample buffer (4:1; 10% w/v SDS, 10 mM β-mercaptoethanol, 20% v/v glycerol, 0.2 M Tris-HCl, pH 6.8, 0.05% w/v bromophenol-blue) and boiled for 10 min. Polyacrylamide gels were prepared with a 4% stacking gel and a 12% resolving gel. The gel was loaded with 10 μl aliquots of samples. The unfolded protein size was estimated using a protein marker (Fermentas; including β-galactosidase, 116 kDa, bovine serum albumin, 62.2 kDa, ovalbumin, 45 kDa, lactate dehydrogenase, 35 kDa, RE Bsp981, 25 kDa, β-lactoglobulin, 18.4 kDa, and lysozyme, 14.4 kDa). The electrophoresis cell was loaded into the gel tank which was filled with electrophoresis running buffer (25 mM Tris, 200 mM glycin and 0.1% w/v SDS). The electrophoresis was run under a constant 30 mA current until the front dye reached the end of the gel. The gel was stained by shaking gently, for 30 min, in sufficient Coomassie brilliant blue R 250 staining solution to cover the gel. Excess stain was removed by soaking 1-2 h in a large volume of Coomassie blue destaining solution.

The molecular weight of the purified unfolded His-tagged recombinant ARM thermostable lipase was estimated to be approximately 44 kDa. This estimation corresponded with the size of the eluted protein fractions visualized by SDS-PAGE (**Figure 23,** Lanes 8-13).

**Size exclusion chromatography**

The native molecular weight of the purified ARM lipase was determined by size exclusion chromatography (SEC) using a Sephadex G-75 column and standards of known molecular weight (Kim *et al.,* 1992). The column was equilibrated in Tris-HCl buffer (50 mM, pH 8.0, at 4°C) containing 150 mM NaCl and developed at a flow rate of 0.5 ml/min. The peak elution volume ($v_e$) was taken to be the average of the volumes at which each protein eluted in three independent experiments. The void volume ($v_o$) and total volume ($v_t$) were determined by independently applying blue dextran and xylene cyanol, respectively. The fractional retention ($K_{av}$) was calculated by Equation 4:

$$K_{av} = \frac{V_e - V_\sigma}{V_t - V_O} \quad (4)$$

where $v_e$ is the peak elution volume. A standard curve of $K_{av}$ *versus* log $M_r$ was generated by applying protein standards (Amersham Biosciences) to the column under the conditions described above. Protein standards (Amersham Biosciences) included ribonuclease A (13.7 kDa), chymotrypsinogen A (25 kDa), ovalbumin (43 kDa) and bovine serum albumin (BSA) (67 kDa) (final concentration 4 μM). The $K_{av}$ calculated for ARM lipase was used to extrapolate its molecular weight from the standard curve.

The molecular weight of the purified native enzyme was determined from the protein standard curve by size exclusion chromatography **(Figure 24).** The calculated molecular weight of native ARM lipase was around 44 kDa. This result was confirmed the estimated molecular weight from SDS-PAGE and suggesting that the ARM lipase was monomer.

**Effects of pH and temperature on lipase activity**

The effects of pH and temperature on the lipase activity were investigated by emulsifying the substrate (olive oil) in buffers of different pH ranging from 4.4 to 10.6 at different temperatures (40-80°C). The used buffers (50 mM) were sodium acetate (4.4-5.6), sodium citrate-sodium phosphate (5.6-6.5), sodium phosphate (6.0-8.0), Tris-HCl (7.5-8.5), glycine-NaOH (8.0-9.5) and sodium carbonate (9.6-10.6). All buffers were prepared at exact testing temperatures.

The results showed that this enzyme can be operated in a broad range of pH and temperature **(Figures 25, 26 and 27)** with maximum lipolytic activity at pH 8.0 (Tris-HCl buffer) and 65°C. However, lipase was found to be most active at a pH between 6.0 and 9.0 while the lipase activity decreased sharply when the pH was below 7.0 or over 8.0.

The tertiary structure of an enzyme is formed by electrostatic bonds. Enzymes are amphoteric molecules containing a large number of acidic and basic groups, mainly situated on their surface. The pH affects the ionization state of acidic or basic amino acids, thus the charges on these groups will vary, according to their acid dissociation constants, with the pH of their environment. This will affect the total net charge of the enzymes and the distribution of charge on their exterior surfaces, in addition to the reactivity of the catalytically active groups. If the state of ionization of amino acids in a protein is altered then the ionic bonds that help to determine the tertiary structure of the protein can be altered. This can lead to enzyme inactivation. Taken together, the changes in charges with pH can affect the activity, structural stability and solubility of the enzyme. In addition, changes in pH may also change the shape or charge properties of the substrate(s). So that either the substrate cannot bind to the active site or it cannot undergo catalysis. As a conclusion, the pH can affect both the lipase adsorption at the oil-water interface and the catalytic step (Chahinian *et al.,* 2006).

**Effects of pH and temperature on lipase stability**

The effects of pH and temperature on the lipase stability were investigated. The purified enzyme was pre-incubated in buffers (1:1 v/v) of different pH from 4.4 to 10.6 at different temperatures (50-70°C) for 180 min. The used buffers (50 mM) were sodium acetate (4.4-5.6), sodium citrate-sodium phosphate (5.6-6.5), sodium phosphate (6.0-8.0), Tris-HCl (7.5-8.5), glycine-NaOH (8.0-9.5) and sodium carbonate (9.6-10.6). All buffers were prepared at exact testing temperatures. The lipase activity was assayed at 65°C using olive oil as substrate.

The stability of purified lipase was examined at pH 5.0-10.0 and 50-70°C **(Figure 28).** The half-life of the ARM lipase was only few min at 60 and 70°C. The enzyme retained 50% residual activity at 50°C after incubation at pH 5.0-7.0 for more than 150 min, pH 8.0 (PBS buffer) for 130 min, pH 8.0 (Tris-HCl buffer) for 90 min, pH 9.0 around 110 min and pH 10.0 more than 50 min. Therefore, ARM lipase was stable in a broad range of pH (5.0-8.0) for > 2 h, 9.0 for > 1.5 h and 10.0 for around 50 min at 50°C. The highest stability was seen at acidic pH (5.0 and 6.0). The results revealed a big difference between lipase stabilities **(Figure 28)** as well as lipase activities (Figure 27) at pH 8.0 in different buffers. ARM lipase showed the highest activity and the lowest thermostability in Tirs-HCl buffer. Since protein thermostability decreased as protein flexibility increased (Vieille and Zeikus, 2001) these results implied that the enzyme was more flexible in Tris-HCl buffer than other buffers. On the other hand, the highest activity was seen in alkaline pH (8.0), while the highest stability was seen in acidic pH (5.0 and 6.0). Lipase activity showed a sharp decrease in different pH during the first 25 min, and then the activity was almost stable in some pH even until 120 min when the second sharp decrease happened **(Figure 28).** It has been implied that pH could affect on ARM lipase at two phases. At the first phase (the first 25 min), the first critical ionizeable residue(s) could be changed and the first decrease in activity was seen. The second critical ionizeable residue(s) was more stable and needed more time or higher pH to be ionized.

**Effect of metal ions on lipase activity**

To determine the effects of metal ions on the lipase activity, the purified enzyme was pre-incubated for 30 min at 30°C with various metal ions. Two concentrations (1 and 5 mM) of each of the following ions were used: $CO^{2+}$, $CU^{2+}$, $Mg^{2+}$, $Ni^{2+}$, $Mn^{2+}$, $Zn^{2+}$ $Fe^{2+}$, $Ca^{2+}$, $Sr^{2+}$, $K^{+}$, $Na^{+}$, $Rb^{+}$ and $Ag^{+}$. All salts were dissolved in MOPS buffer (50 mM, pH 8.0) (Aslamkhan *et al.,* 2002). The control contained no ion. The relative activity was measured at 65°C using olive oil (emulsified, 1:1 v/v, in 50 mM Tris-HCl buffer, pH 8.0) as substrate. Metal cations play different and important roles in influencing the structure and function of lipases (Gupta *et al..,* 2004a; Kim *et al.,* 2005). The effect of different metal ions on the purified ARM lipase activity was studied **(Figure 29).** Most of the bivalents decreased the lipase activity except

$Ca^{2+}$ and 1 mM $Sr^{2+}$ which stimulated the lipase activity. The $Ca^{2+}$-binding motif was not found in ARM lipase sequence. It indicates that ARM lipase was not a $Ca^{2+}$-dependent lipase.

**Effect of surfactants on lipase activity**

The effect of surfactants on the lipase activity was investigated by pre-incubating the purified enzyme for 30 min at 30°C in Tris-HCl buffer (50 mM, pH 8.0) containing 0.1 and 1% (v/v) of following surfactants (nonionic surfactants): Tween 20, Tween 40, Tween 80, Tween 85, Triton X-100, Triton B1958, Span 20. In the case of SDS (ionic surfactant) concentrations were 1 and 5 mM. The control contained no surfactant. The relative activity was measured at 65°C using olive oil (emulsified, 1:1 v/v, in 50 mM Tris-HCl buffer, pH 8.0) as substrate. The effects of various surfactants on the purified ARM lipase are depicted in **Figure 30.**

The activity was not affected by Span 20 while 1% Triton X-100 caused an increase of 20% in lipase activity. Other surfactants had inhibitory effect and especially lipase activity strongly inhibited by SDS. The response to the presence of detergents by reported thermophilic lipases is variable.

**Effect of inhibitors on lipase activity**

The effect of inhibitors on the lipase activity was investigated by pre-incubating the purified enzyme for 30 min at 30°C in Tris-HCl buffer (50 mM, pH 8.0) containing 1 and 5 mM of following inhibitors: ethylene diamine tetra-acetic acid (EDTA) as di- and tri-valent metal ions chelating agent; β-mercaptoethanol as redox reagent and phenyl methane sulphonyl fluoride or phenyl methyl sulphonyl fluoride (PMSF) as serine hydrolase inhibitor. The control contained no inhibitor. The relative activity was measured at 65°C using olive oil (emulsified, 1:1 v/v, in 50 mM Tris-HCl buffer, pH 8.0) as substrate. The effect of various classes of inhibitors on purified lipase activity was investigated **(Figure 31).** The metal chelating agent EDTA (ethylene diamine tetra-acetic acid) was the most effective agent in activation of the lipase around 20% at concentration of 1 mM and more than 60% at 5 mM, suggesting that the enzyme was not a metalloenzyme. Although the serine hydrolase inhibitor PMSF (phenylmethylsulphonyl fluoride) showed no significant effect on lipase activity at 1 mM, it completely inhibited the lipolytic activity at 5 mM, which suggests the presence of a serine residue at the catalytic triad of the active site, thus ARM lipase belonged to serine hydrolase family.

**Specificity of lipase towards natural oils**

To determine the lipase selectivity towards natural oils, the following oils were tested: olive, corn, sunflower, soy bean, rice bran, sesame, canola, grape seed, walnut, palm, palm olein, palm stearin, palm kernel, palm kernel olein and virgin coconut. The oils were emulsified (1:1 v/v) in 50 mM Tris-HCl (pH 8.0). The lipase activity was assayed at 65°C. The relative activity was expressed as a fraction of the activity obtained by olive oil (as the standard substrate). Substrate selectivity of lipases is one of the crucial properties which determines their ultimate usage. Therefore, characterization of lipases in relation to their substrate selectivity is an important task. The substrate preferences of the purified recombinant ARM lipase were characterized with various natural oils. As shown in **Figure 32,** The relative activity was expressed as a fraction of the activity obtained by olive. ARM lipase hydrolyzed all the natural oils tested, with the highest activity toward palm kernel and palm kernel olein with 105 and 101%, respectively. Except palm kernel oil that its major fatty acyl chain was lauric acid (C12:0), other oils that cause relative activity more than 50% as substrate, contained long, unsaturated fatty acyl chains especially C18:1 and C18:2.

**Regioselectivity study of ARM lipase**

The regioselectivity of the purified recombinant lipase was studied using high performance thin layer chromatography (HPTLC). The enzymatic reaction was performed using pure triolein as substrate. The reaction mixture, consisting of 1 ml purified enzyme, 2.5 ml triolein emulsion (properly mixed of an equal volume triolein with 50 mM Tris-HCl buffer, pH 8.0) and 0.02 ml of 20 mM $CaCl_2$, was incubated in a water bath shaker for 0 and 4 h at 65°C under 200 rpm agitation. After the incubation, 5 ml n-hexane was added to stop the reaction and extract the products. The products were separated and analysed by mono-dimensional HPTLC using the following solvent system and condition. Mobile phase: n-hexane/diethyl ether/acetic acid, 54:45:1 (v/v), the separation was carried out on 20 x 10 cm HPTLC plates silica gel 60 F 254 (MERCK). Products were visualized with 10% concentrated sulfuric acid in methanol. The quantities of products were determined by densitometry and comparison with 0.1-2 μg of standard compounds using the CAMAG TLC scanner 3 under wavelength of 385 nm and winCATS Planar Chromatography Manager software (CAMAG). Pure monoolein, 1,2 (2,3) diolein, 1,3 diolein, oleic acid and triolein were used as the reference glycerides.

The positional specificity of purified lipase was investigated using triolein as substrate. **Figure 33** shows the chromatograms of HPTLC analysis of triolein hydrolysis products. The comparison between the chromatograms before (0 h; Figure 33A) and after (4 h; Figure 33B) hydrolysis, indicated that this lipase hydrolysed triacylglycerol to produce free fatty acids (FFA), 1,2-diacylglycerols (1,2-DAG), and 2-monoacylglycerol (MAG). This means that cleavage was only occurred at the Sn-1 and Sn-3 positions and therefore, ARM lipase was a regioselective enzyme (1,3-specific lipase).

**Lipase organic solvent-stability**

Aliquots of the enzyme were incubated with the solvents (30%, v/v) for 30 min at 30°C with shaking at 150 rpm. The solvents were selected based on their different log P values (values in the parenthesis): DMSO (-1.378), 2,3-butanediol (-0.92), methanol (-0.76), acetonitrile (-0.394), Ethanol (-0.235), acetone (-0.208), 2-propanol (0.074), 1-propanol (0.28), iso-butanol (0.79), 1-butanol (0.8), bnzyl alcohol (1.1), iso-amyl alcohol (1.3), benzene (2), toluene (2.5), 1-octanol (2.9), o-xylene (3.12), ethylbenzene (3.3), Hexanes (3.6), 1-decanol (4), heptan (4.4), iso-octane (4.5), 1-undecanol (4.6), 1-dodecanol (5), n-tetradecane (7), n-pentadecane (7.71), heptadecane (8.69), 1-hexadecane (8.8). The stability was expressed as the remaining lipolytic activity relative to that of the control (without solvent). The relative activity was measured at 65°C as described before. The effect of various polar and non-polar organic solvents (log P values: -1.378 to 8.8) on the stability of purified ARM lipase was studied **(Figure 34).** Among 26 different tested organic solvents, in the presence of more than half of them the enzyme activity was more than 100% (100.4-165%) including 2,3-butanediol, methanol, ethanol, benzene, toluene, 1-octanol, o-xylene, hexanes, heptan, iso-octane, 1-dodecanol, n-tetradecane, n-pentadecane and heptadecane. In addition, ARM lipase retained its activity of 86%, 78%, 77%, 74% and 62.5% in the presence of 1-decanol, 1-hexadecane, ethylbenzene, 1-undecanol and DMSO, respectively. Acetonitrile, acetone, 2-propanol, 1-propanol, 1-butanol, iso-amyl alcohol, iso-butanol and bnzyl alcohol dramatically inhibited the lipase activity. On the other hand ARM lipase not only showed stability in the presence of some water miscible solvents such as DMSO and 2,3-butanediol with relative activity of 62.4 and 100.4%, respectively but also some of water miscible solvents even could activate the enzyme such as methanol and ethanol with relative activity of 107.3 and 116.4%, respectively. As a conclusion, although, there was no clear correlation between the P value of an water miscible organic solvent and the stability of ARM lipase in its presence, results indicated that the solvents with P values in the range of ~ -0.2 and ~ 1.3 had the highest destabilizing effect on ARM lipase **(Figure 34),** suggesting that maybe solvents can be categorized in three groups; water miscible solvents, water immiscible solvents and partially water miscible solvents.

**Effect of ionic liquids on lipase thermostability**

Aliquots of the enzyme were suspended in 90% (v/v) of four different ionic liquids (ILs) properly. Then, the mixture of lipase and ILs was incubated for 0-90 min at 60°C with shaking at 150 rpm. The selected ILs were [Emim][$BF_4$], [Emim][TfO], [Bmim][$BF_4$] and [Bmim][$PH_6$]. The thermostability was expressed as the remaining lipolytic activity relative to that of the control (without ILs) after incubation at high temperature. The residual activity was measured at 65°C as described before. **Figure 35 and Figure 36** show the effect of ILs on lipase hydrolytic activity (at 0 min, without incubation) and lipase thermostability at 60°C (during the incubation time), respectively. Lipase activity was increased by [Emim][TfO] and [Bmim][$PH_6$] up to 120%. [Emim][$BF_4$] had no significant effect on the activity. In contrast, [Bmim][$BF_4$] decreased lipase activity. [Emim][TfO] and [Emim][$BF_4$] showed significant effects on lipase thermostability enhancement. One possible explanation of higher thermal stability of enzyme in some ILs is that the structure of enzyme may be compact in ILs which prevents the thermal denaturation of enzyme under the temperature condition tested.

**Claims**

1. A biologically pure culture of *Geobacillus* sp. strain ARM; wherein the *Geobacillus sp* strain ARM is deposited under the accession NCIMB 41583 at The National Collection of Industrial, Marine and Food Bacteria (NCIMB), UK.

2. The biologically pure cultures according to claim 1, wherein the *Geobacillus sp* strain ARM provides characteristics including:

   a) a gram positive microorganism ;
   b) having the capability to grow between 45°C and 70°C with optimum growth 55°C;
   c) showing a positive growth in tributyrin agar and Rhodemine B agar and nutrient agar;
   d) having the capability to grow at pH between 5.5-9 with optimal pH growth of 7;
   e) negative anaerobic growth; and
   f) organic solvent tolerant.

3. The biologically pure culture according to claim 1, wherein the *Geobacillus sp* strain ARM shows physiological characteristics including:

   a) catalase positive and oxidase negative;
   b) fermentation of D-glucose (positive), L-arabinose (negative), D-xylose (positive),D-mannitol (positive, D-fructose (positive);
   c) hydrolyse Tween 8;
   d) hydrolyse natural oils.

4. The biologically pure culture according to claim 1, wherein the *Geobacillus sp* strain ARM comprises a nucleotide sequence 16S rDNA of SEQ ID NO 1 (EF025325) and/or nucleotide sequence (EF042975) of SEQ ID NO 3 and/or amino acid sequence (EF042975)of SEQ ID NO 4.

5. A method of obtaining a novel thermostable lipase isolated and characterized from *Geobacillus sp* strain ARM, wherein the method comprising the steps of:

   a) conducting DNA extraction of the *Geobacillus sp* strain ARM,
   b) obtaining a purified DNA from step (a),
   c) identifying and amplifying 16S rDNA sequence by conducting polymerase chain reaction (PCR) technique conducting DNA extraction of the *Geobacillus sp* strain ARM;
   d) obtaining mature lipase gene (ARM lipase) by performing cloning technique;
   e) conducting secretory expression of the mature lipase gene (ARM lipase) and obtaining recombinant ARM lipase gene;
   f) purifying the ARM lipase gene from step (e) and obtaining a purified ARM lipase;
   g) determining size of ARM lipase;
   h) characterizing the ARM lipase using biochemical and biophysical methods.

6. Use of the *Geobacillus sp* strain ARM, producing a thermostable lipase, according to any one of claims 1 to 4, for industrial applications such as in food industry, oil processing, production of surfactants, oil processing, detergents, pesticides, environmental management and leather industry.

## Patentansprüche

1. Eine biologisch reine Kultur eines *Geobacillus sp.* Stamms ARM; worin der *Geobacillus sp* Stamm ARM unter der Hinterlegungsnummer NCIMB 41583 bei The National Collection of Industrial, Marine and Food Bacteria (NCIMB), UK, hinterlegt ist.

2. Die biologisch reine Kultur gemäß Anspruch 1, worin der *Geobacillus sp* Stamm ARM Charakteristika bereitstellt einschließlich:

   a) ein grampositiver Mikroorganismus;
   b) aufweisend die Fähigkeit, zwischen 45°C und 70°C zu wachsen mit einem optimalen Wachstum bei 55°C;
   c) zeigt ein positives Wachstum in Tributyrin Agar und Rhodemin B Agar und Nährstoff Agar;
   d) aufweisend die Fähigkeit, bei einem pH zwischen 5,5-9 zu wachsen mit einem optimalen Wachstum bei pH 7;
   e) negatives anaerobes Wachstum; und
   f) organisches Lösungsmittel tolerant.

3. Die biologisch reine Kultur gemäß Anspruch 1, worin der *Geobacillus sp* Stamm ARM physiologische Charakteristika zeigt einschließlich:

   a) Katalase positiv und Oxidase negativ;
   b) Fermentation von D-Glukose (positiv), L-Arabinose (negatic), D-Xylose (positiv), D-Mannitol (positiv, D-Fruktose (positiv);
   c) hydrolisiert Tween 8;
   d) hydrolysiert natürliche Öle.

4. Die biologisch reine Kultur gemäß Anspruch 1, worin der *Geobacillus sp.* Stamm ARM eine Nukleotidsequenz einer 16S rDNA von SEQ ID Nr. 1 (EF025325) umfasst, und/oder eine Nukleotidsequenz (EF042975) der SEQ ID Nr. 3 umfasst und/oder eine Aminosäuresequenz (EF042975) von SEQ ID Nr. 4 umfasst.

5. Ein Verfahren zur Gewinnung einer neuen thermostabilen Lipase isoliert und charakterisiert von *Geobacillus sp* Stamm ARM, worin das Verfahren die Schritte umfasst:

   a) durchführen einer DNA Extraktion des *Geobacillus sp* Stamm ARM,
   b) Gewinnung einer aufgereinigten DNA aus Schritt (a),
   c) identifizieren und amplifizieren einer 16s rDNA Sequenz durch Durchführung einer Polymerase Kettenreaktion (PCR) Technik durchführen DNA Extraktion des *Geobacillus sp* Stamms ARM;
   d) Gewinnung eines maturierten Lipase Gens (ARM Lipase) durch Durchführung einer Klonierungstechnik;
   e) Durchführung einer sekretierenden Expression des maturierten Lipasegens (ARM Lipase) und Gewinnung des rekombinanten ARM Lipase Gens;
   f) Aufreinigung des ARM Lipase Gens aus Schritt (e) und Gewinnung einer aufgereinigten ARM Lipase;
   g) Bestimmung der Größe der ARM Lipase;
   h) charakterisieren der ARM Lipase mittels biochemischer und biophysikalischer Verfahren.

6. Verwendung des *Geobacillus sp* Stamms ARM, herstellen einer thermostabilen Lipase, gemäß eines jeden der Ansprüche 1-4, für industrielle Anwendungen wie in der Nahrungsmittelindustrie, Öl-Prozessierung, Herstellung von Tensiden, Öl-Prozessierung, Detergenzien, Pestizide, Umweltmanagement und Lederindustrie.

## Revendications

1. Culture biologiquement pure de la souche de *Geobacillus sp.* ARM ; où la souche de *Geobacillus sp.* ARM est déposée sous le numéro d'accès NCIMB 41583 auprès de la Collection Nationale de Bactéries Industrielles, Marines et Alimentaires (NCIMB), Royaume-Uni.

2. Cultures biologiquement pures selon la revendication 1, dans lesquelles la souche de *Geobacillus sp.* ARM fournit des caractéristiques comportant :

   a) un micro-organisme gram-positif ;

b) le fait d'avoir la capacité de croître entre 45°C et 70°C avec une croissance optimale à 55°C ;
c) le fait de présenter une croissance positive dans la gélose à base de tributyrine et la gélose à base de Rhodamine B et la gélose nutritive ;
d) le fait d'avoir la capacité de croître à un pH compris entre 5,5 et 9 avec une croissance à un pH optimal de 7 ;
e) une croissance anaérobie négative ; et
f) une tolérance à un solvant organique.

**3.** Culture biologiquement pure selon la revendication 1, dans laquelle la souche de *Geobacillus sp.* ARM présente des caractéristiques physiologiques comportant :

a) catalase positive et oxydase négative ;
b) la fermentation de D-glucose (positive), de L-arabinose (négative), de D-xylose (positive), de D-mannitol (positive), de D-fructose (positive) ;
c) l'hydrolyse de Tween 8 ;
d) l'hydrolyse des huiles naturelles.

**4.** Culture biologiquement pure selon la revendication 1, dans laquelle la souche de *Geobacillus sp.* ARM comprend une séquence nucléotidique d'ADNr 16S de la séquence SEQ ID N° 1 (EF025325) et/ou une séquence nucléotidique (EF042975) de la séquence SEQ ID N° 3 et/ou une séquence d'acides aminés (EF042975) de la séquence SEQ ID N° 4.

**5.** Procédé pour obtenir une nouvelle lipase thermostable isolée et caractérisée à partir de la souche de *Geobacillus sp.* ARM, où le procédé comprenant les étapes qui consistent .

a) à réaliser l'extraction d'ADN de la souche de *Geobacillus sp.* ARM,
b) à obtenir un ADN purifié de l'étape (a),
c) à identifier et à amplifier la séquence d'ADNr 16S en réalisant la technique d'amplification en chaîne par polymérase (PCR) réalisant l'extraction d'ADN de la souche de *Geobacillus sp.* ARM ;
d) à obtenir un gène de lipase mature (lipase d'ARM) en réalisant une technique de clonage ;
e) à réaliser une expression sécrétoire du gène de lipase mature (lipase d'ARM) et à obtenir un gène de lipase d'ARM recombinante ;
f) à purifier le gène de lipase d'ARM de l'étape (e) et à obtenir une lipase d'ARM purifiée ;
g) à déterminer la taille de la lipase d'ARM ;
h) à caractériser la lipase d'ARM en utilisant des procédés biochimiques et biophysiques.

**6.** Utilisation de la souche de *Geobacillus sp.* ARM, produisant une lipase thermostable, selon l'une quelconque des revendications 1 à 4, pour des applications industrielles telles que dans l'industrie alimentaire, le traitement d'huiles, la production de tensioactifs, le traitement d'huiles, des détergents, des pesticides, la gestion de l'environnement et l'industrie du cuir.

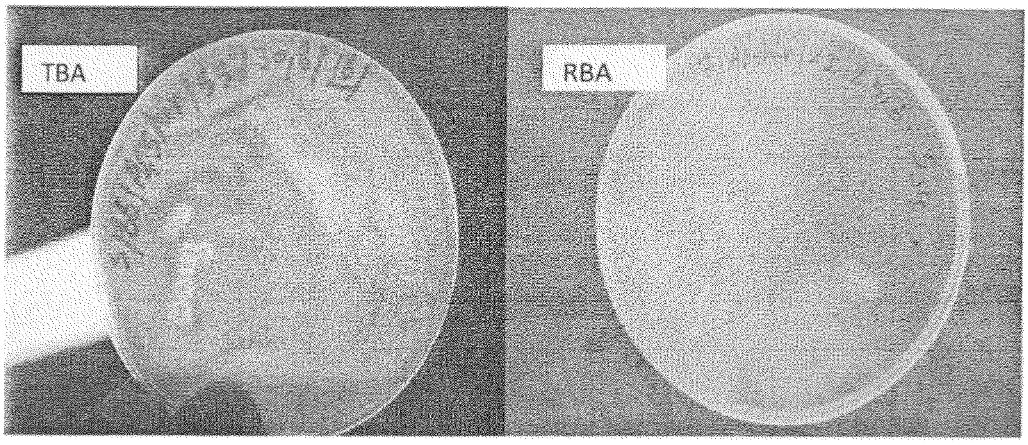


FIGURE 1

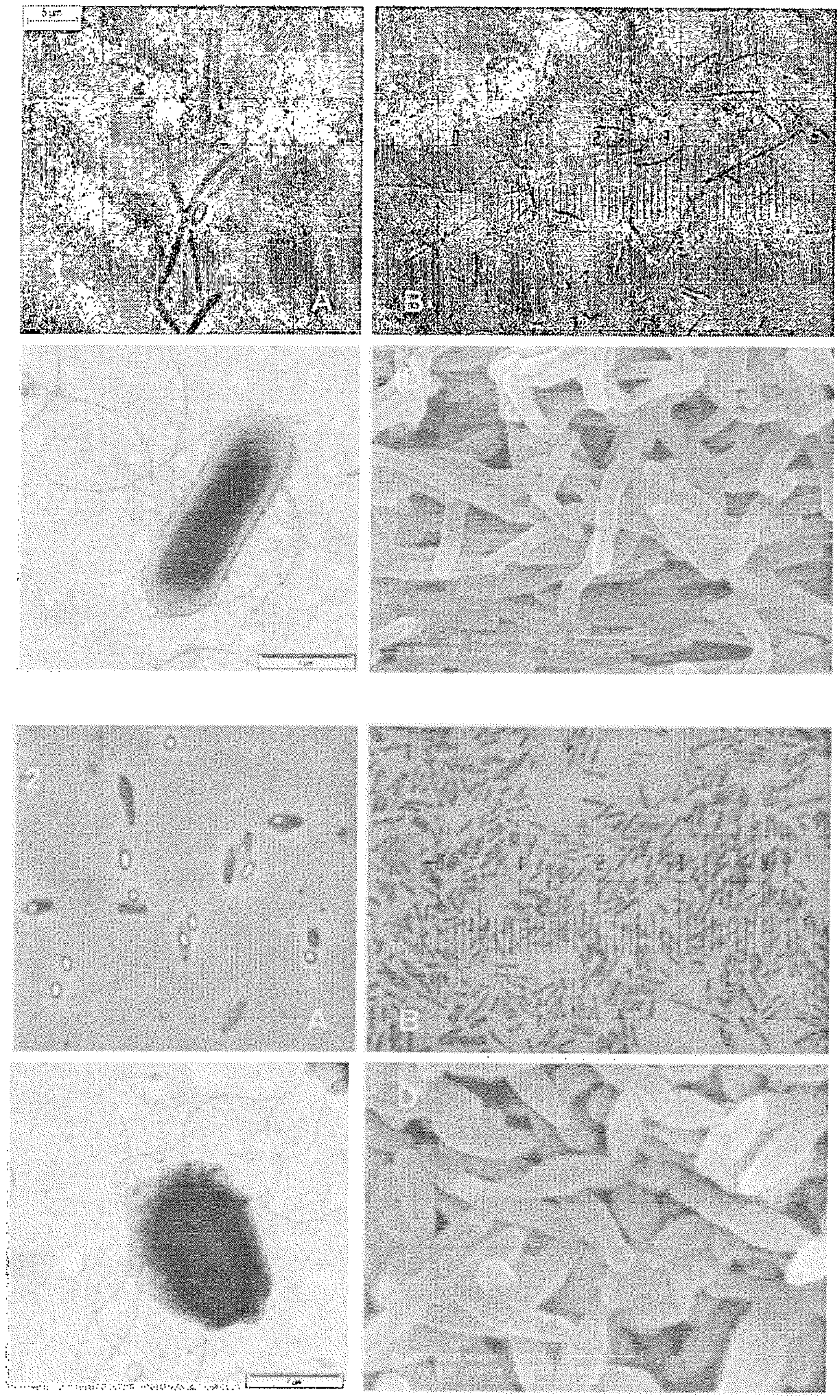


FIGURE 2

A

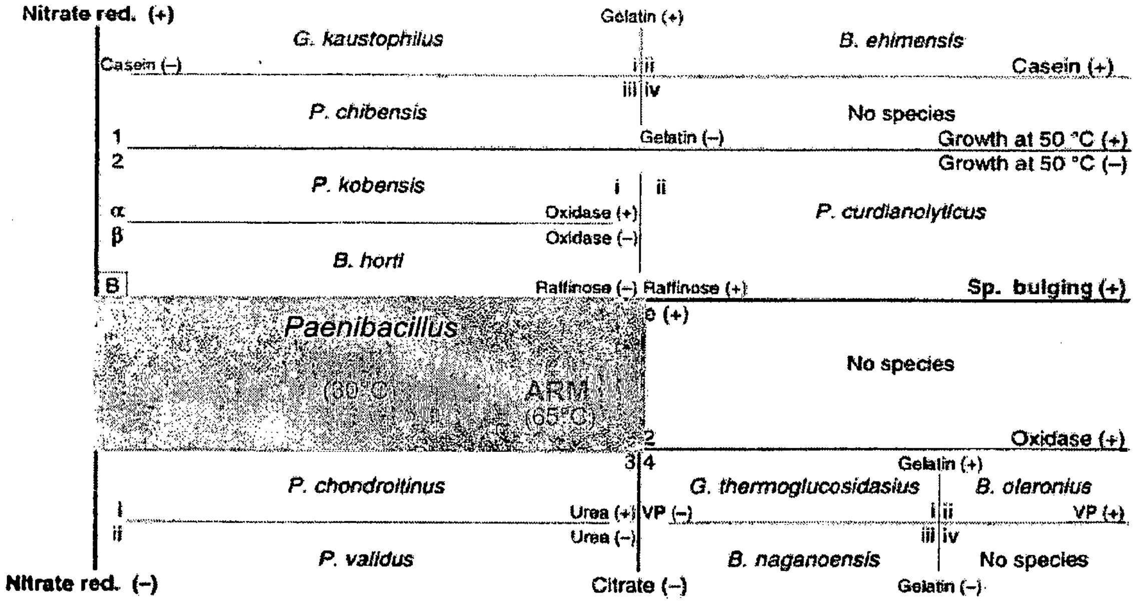

Nitrate red. (+)
Gelatin (+)
G. kaustophilus
B. ehimensis
Casein (-)
Casein (+)
i ii
iii iv
P. chibensis
No species
1
Gelatin (-)
Growth at 50 °C (+)
2
Growth at 50 °C (-)
P. kobensis
i ii
α
Oxidase (+)
P. curdianolyticus
β
Oxidase (-)
B. horti
B
Raffinose (-) Raffinose (+)
Sp. bulging (+)
Paenibacillus
(30°C)
ARM
(65°C)
No species
2
Oxidase (+)
3 4
Gelatin (+)
P. chondroitinus
G. thermoglucosidasius
B. oleronius
i
Urea (+)
VP (-)
i ii
VP (+)
ii
Urea (-)
iii iv
P. validus
B. naganoensis
No species
Nitrate red. (-)
Citrate (-)
Gelatin (-)


B

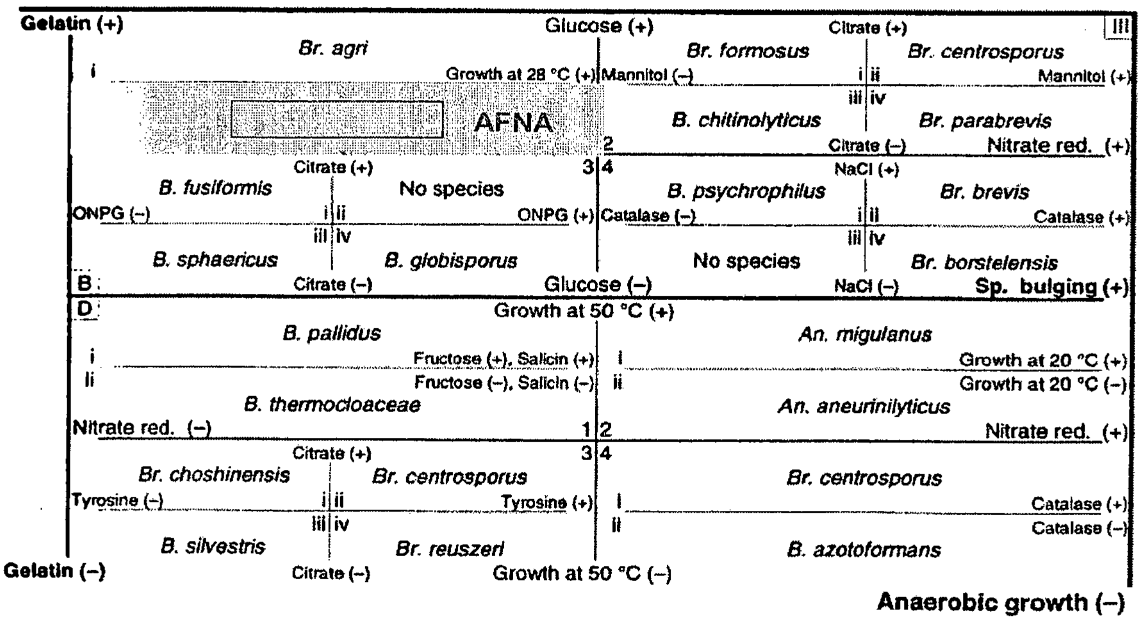

Gelatin (+)
Glucose (+)
Citrate (+)
III
Br. agri
Br. formosus
Br. centrosporus
i
Growth at 28 °C (+)
Mannitol (-)
i ii
Mannitol (+)
iii iv
AFNA
B. chitinolyticus
Br. parabrevis
2
Citrate (-)
Nitrate red. (+)
Citrate (+)
3 4
NaCl (+)
B. fusiformis
No species
B. psychrophilus
Br. brevis
ONPG (-)
i ii
ONPG (+)
Catalase (-)
i ii
Catalase (+)
iii iv
iii iv
B. sphaericus
B. globisporus
No species
Br. borstelensis
B
Citrate (-)
Glucose (-)
NaCl (-)
Sp. bulging (+)
D
Growth at 50 °C (+)
B. pallidus
An. migulanus
i
Fructose (+), Salicin (+)
i
Growth at 20 °C (+)
ii
Fructose (-), Salicin (-)
ii
Growth at 20 °C (-)
B. thermocloaceae
An. aneurinilyticus
Nitrate red. (-)
1 2
Nitrate red. (+)
Citrate (+)
3 4
Br. choshinensis
Br. centrosporus
Br. centrosporus
Tyrosine (-)
i ii
Tyrosine (+)
i
Catalase (+)
iii iv
ii
Catalase (-)
B. silvestris
Br. reuszeri
B. azotoformans
Gelatin (-)
Citrate (-)
Growth at 50 °C (-)
Anaerobic growth (-)


FIGURE 3

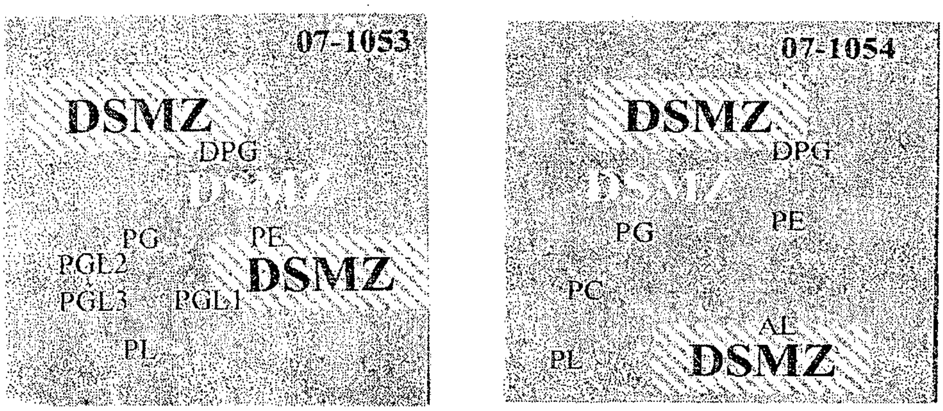


A B

FIGURE 4

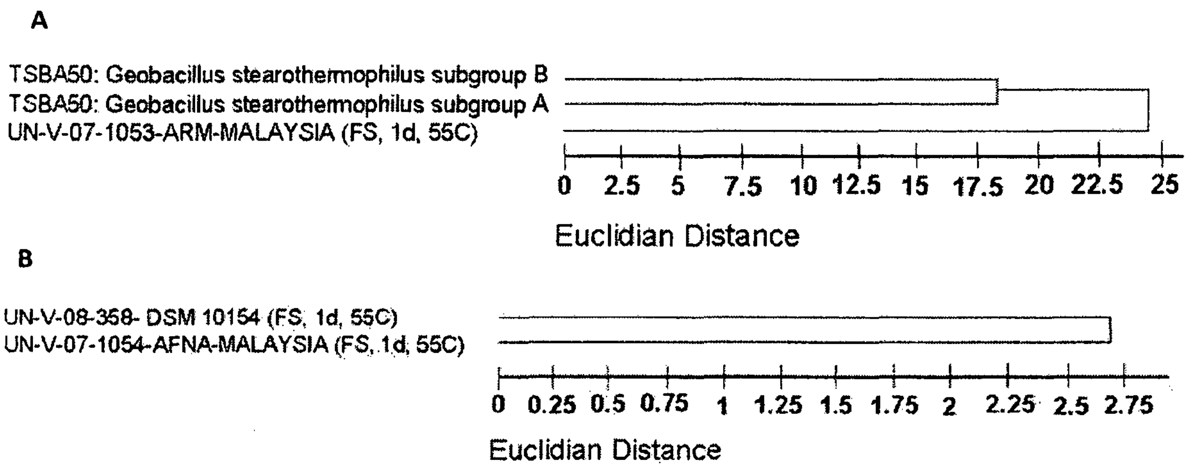


FIGURE 5

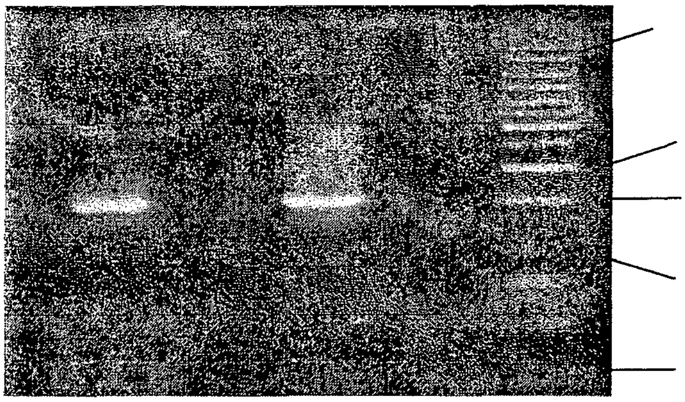


**FIGURE 6**

```
  1 ttcttttgga gagtttgatc ctggctcagg acgaacgctg gcggcgtgcc taatacatgc
 61 aagtcgagcg gactggattg gagcttgctc tgattcggtc agcggcggac gggtgagtaa
121 cacgtgggca acctgcccgc aagaccggga taactccggg aaaccggagc taataccgga
181 taacaccgaa gaccgcatgg tctttggttg aaaggcggcc tttggctgtc acttgcggat
241 gggcccgcgg cgcattagct agttggtgag gtaacggctc accaaggcga cgatgcgtag
301 ccggcctgag agggtgaccg gccacactgg gactgagaca cggcccagac tcctacggga
361 ggcagcagta gggaatcttc cgcaatgggc gaaagcctga cggagcgacg ccgcgtgagc
421 gaagaaggcc ttcgggtcgt aaagctctgt tgtgagggac gaaggagcgc cgttcgaaga
481 gggcggcgcg gtgacggtac ctcacgagga agccccggct aactacgtgc cagcagccgc
541 ggtaatacgt aggggggcgag cgttgtccgg aattattggg cgtaaagcgc gcgcaggcgg
601 ttccttaagt ctgatgtgaa agcccacggc tcaaccgtgg agggtcattg gaaactgggg
661 gacttgagtg caggagagga gagcggaatt cccacgtgta gcggtgaaat gcgtagagat
721 gtggaggaac accagtggcg aaggcggctc tctggcctgc aactgacgct gaggcgcgaa
781 agcgtgggga gcaaacagga ttagataccc tggtagtcca cgccgtaaac gatgagtgct
```

841 aagtgttaga ggggtcacac cctttagtgc tgcagctaac gcgataagca ctccgcctgg

901 ggagtacggc cgcaaggctg aaactcaaag gaattgacgg gggcccgcac aagcggtgga

961 gcatgtggtt taattcgaag caacgcgaag aaccttacca ggtcttgaca tccctgaca

1021 acccaagaga ttgggcgttc ccccttcggg gggacagggt gacaggtggt gcatggttgt

1081 cgtcagctcg tgtcgtgaga tgttgggtta agtcccgcaa cgagcgcaac cctcgcctct

1141 agttgccagc attcggttgg gcactctaga gggactgccg gcgacaagtc ggaggaaggt

1201 ggggatgacg tcaaatcatc atgcccctta tgacctgggc tacacacgtg ctacaatggg

1261 cggtacaaag ggctgcgaac ccgcgagggg gagcgaatcc caaaaagccg ctctcagttc

1321 ggattgcagg ctgcaactcg cctgcatgaa gccggaatcg ctagtaatcg cggatcagca

1381 tgccgcggtg aatacgttcc cgggccttgt acacaccgcc cgtcacacca cgagagcttg

1441 caacacccga agtcggtgag gcaacccgca agggagccag ccgccgaagg tggggcaagt

1501 gattggggtg aagtcgtaac aaggtagccg taccggaagg tgcggctgga tcacctcctt

1561 tct

**B**

1 gagagtttga tcctggctca ggacgaacgc tggcggcgtg cctaatacat gcaagtcgag

61 cgaaccgatg gagtgcttgc attcctgagg ttagcggcgg acgggtgagt aacacgtagg

121 caacctgcct gtacgaccgg gataactccg ggaaaccgga gctaataccg gataggatgc

181 cgaaccgcat ggttcggcat ggaaaggcct ttgagccgcg tacagatggg cctgcggcgc

241 attagctagt tggtggggta acggcctacc aaggcgacga tgcgtagccg acctgagagg

301 gtgaacggcc acactgggac tgagacacgg cccagactcc tacgggaggc agcagtaggg

361 aatcttccgc aatggacgaa agtctgacgg agcaacgccg cgtgagtgag gaaggtcttc

421 ggatcgtaaa actctgttgt cagggaagaa ccgccgggat gacctcccgg tctgacggta

481 cctgacgaga aagccccggc taactacgtg ccagcagccg cggtaatacg taggggcaa

541 gcgttgtccg gaattattgg gcgtaaagcg cgcgcaggcg gcttcttaag tcaggtgtga

601 aagcccacgg ctcaaccgtg gagggccatc tgaaactggg gagcttgagt gcaggagagg

661 agagcggaat tccacgtgta gcggtgaaat gcgtagagat gtggaggaac accagtggcg

721 aaggcggctc tctggcctgt aactgacgct gaggcgcgaa agcgtgggga gcaaacagga

781 ttagataccc tggtagtcca cgccgtaaac gatgagtgct aggtgttggg gagtccacct

841 cctcagtgcc gcagctaacg caataagcac tccgcctggg gagtacggcc gcaaggctga

901 aactcaaagg aattgacggg gacccgcaca agcggtggag catgtggttt aattcgaagc

961 aacgcgaaga accttaccag ggcttgacat cccgctgacc cctccagaga tggaggcttc

1021 cttcgggaca gcggtgacag gtggtgcatg gttgtcgtca gctcgtgtcg tgagatgttg

1081 ggttaagtcc cgcaacgagc gcaacccttg tcctttgttg ccagcattca gttgggcact

1141 ctaaggagac tgccgtcgac aagacggagg aaggtgggga tgacgtcaaa tcatcatgcc

1201 ccttatgtcc tgggctacac acgtgctaca atggacggta caacgggcgt gccaacccgc

1261 gagggtgagc caatccctaa aaaccgttct cagttcggat tgcaggctgc aactcgcctg

1321 catgaagccg gaatcgctag taatcgcgga tcagcatgcc gcggtgaata cgttcccggg

1381 tcttgtacac accgcccgtc acaccacgag agtttgcaac acccgaagtc ggtgaggtaa

1441 ccttctggag ccagccgccg aaggtggggc agatgattgg ggtgaagtcg taacaaggta

1501 tccg

**FIGURE 7**

**A**

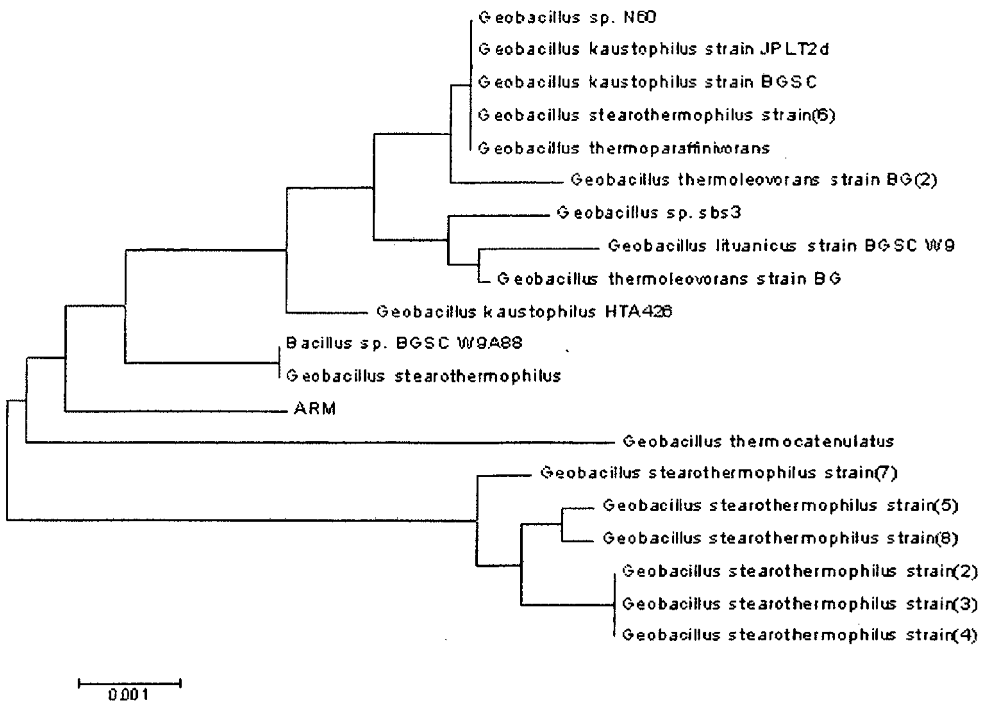
Geobacillus sp. N60
Geobacillus kaustophilus strain JPLT2d
Geobacillus kaustophilus strain BGSC
Geobacillus stearothermophilus strain(6)
Geobacillus thermoparaffinivorans
Geobacillus thermoleovorans strain BG(2)
Geobacillus sp. sbs3
Geobacillus lituanicus strain BGSC W9
Geobacillus thermoleovorans strain BG
Geobacillus kaustophilus HTA426
Bacillus sp. BGSC W9A88
Geobacillus stearothermophilus
ARM
Geobacillus thermocatenulatus
Geobacillus stearothermophilus strain(7)
Geobacillus stearothermophilus strain(5)
Geobacillus stearothermophilus strain(8)
Geobacillus stearothermophilus strain(2)
Geobacillus stearothermophilus strain(3)
Geobacillus stearothermophilus strain(4)
0.001

**B**

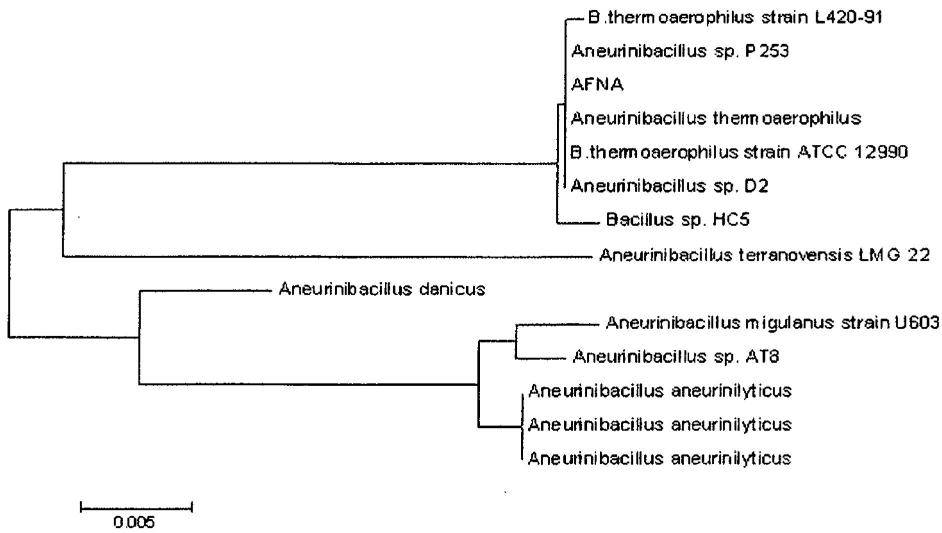
B.thermoaerophilus strain L420-91
Aneurinibacillus sp. P253
AFNA
Aneurinibacillus thermoaerophilus
B.thermoaerophilus strain ATCC 12990
Aneurinibacillus sp. D2
Bacillus sp. HC5
Aneurinibacillus terranovensis LMG 22
Aneurinibacillus danicus
Aneurinibacillus migulanus strain U603
Aneurinibacillus sp. AT8
Aneurinibacillus aneurinilyticus
Aneurinibacillus aneurinilyticus
Aneurinibacillus aneurinilyticus
0.005

**FIGURE 8**

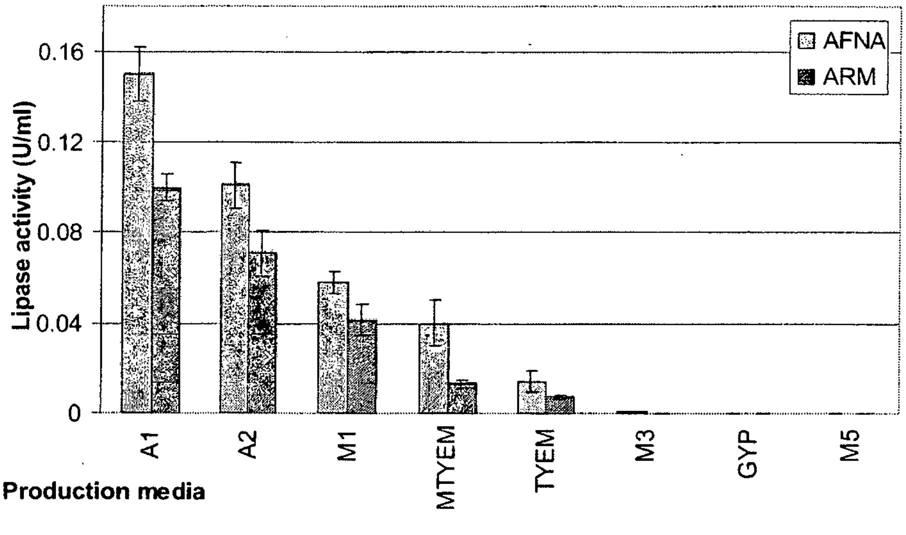


**FIGURE 9**

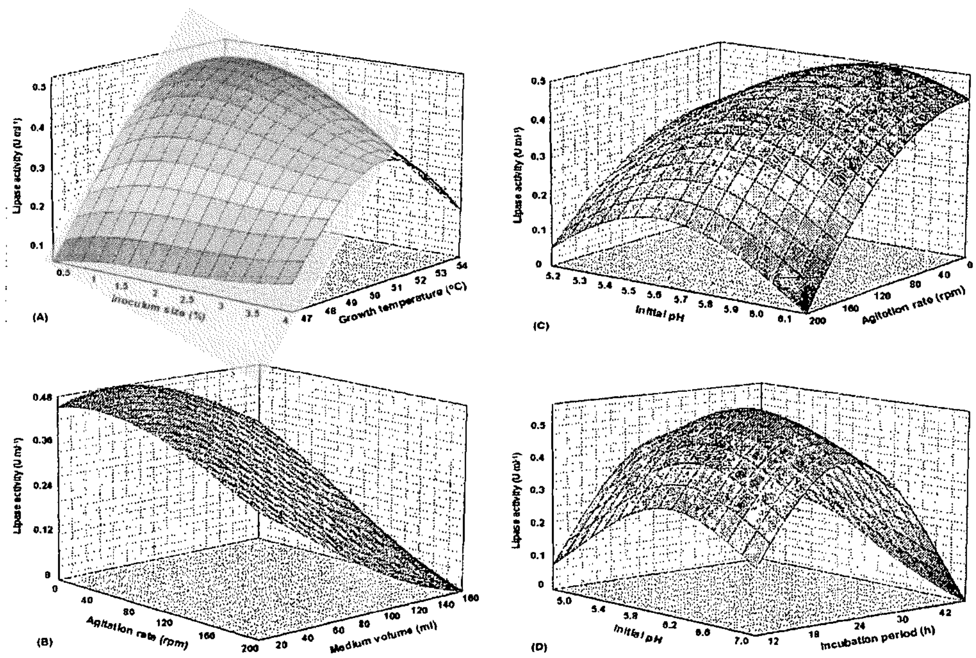


**FIGURE 10**

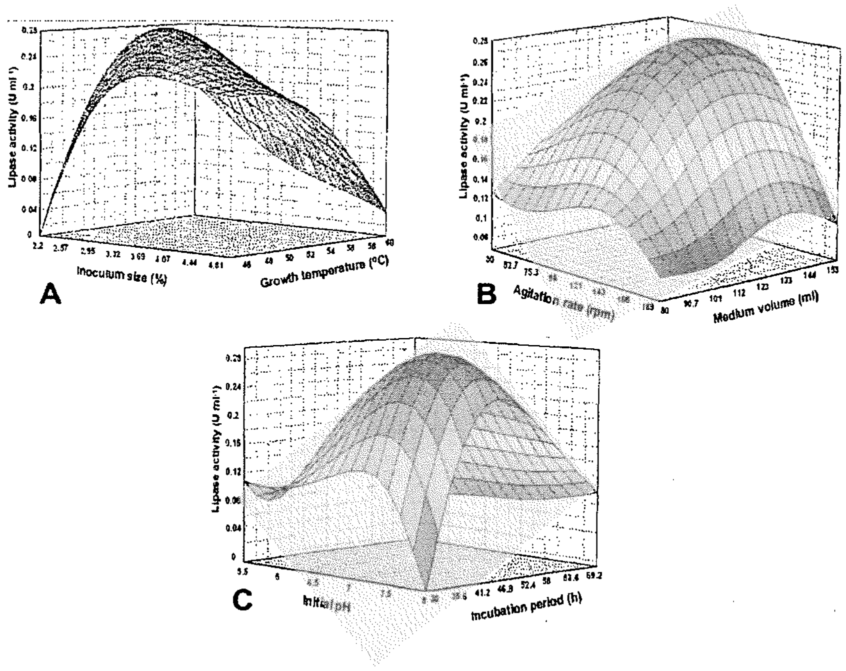


FIGURE 11

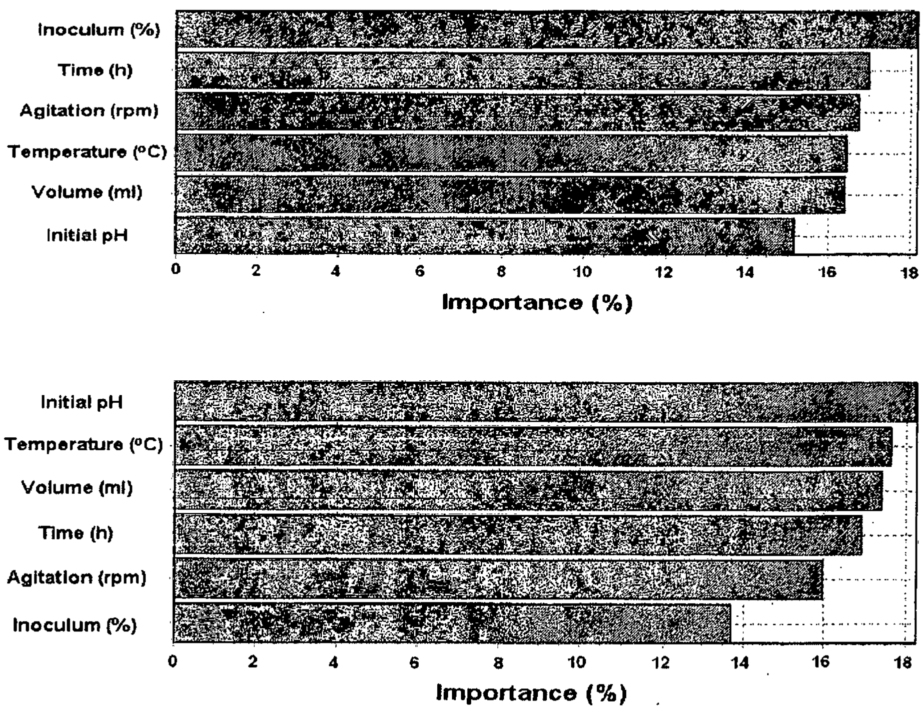


FIGURE 12

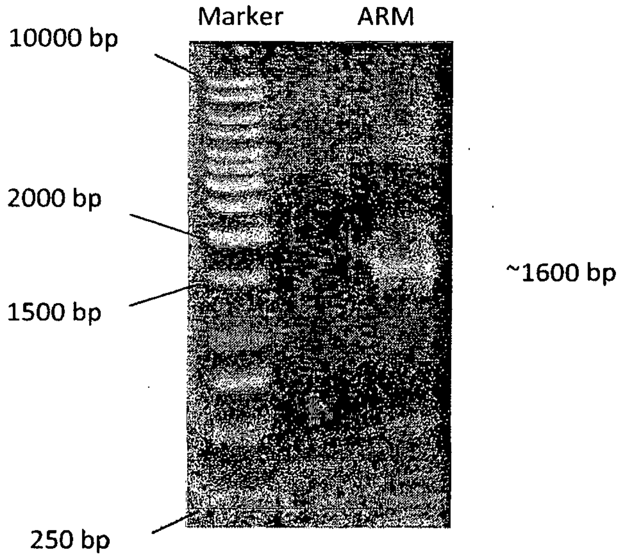


**FIGURE 13**

```
                   -35                              -10
gggttggaaggggccttgggtcttatgtataaatattctggtaattcagaatgaaaagattt
                     RBS       Start          Signal peptide
cggcctattgaaggaaaggagagggagaacatgatgaaatgctgtcggcgtgttgccctt
                              M  M  K  C  C  R  R  V  A  L
gtgttgctcggattatggtttgtgttctgcatatctgttctgggagggcgagctgaagcg
 V  L  L  G  L  W  F  V  F  C  I  S  V  L  G  G  R  A  E  A
gcggcttcgcgagccaacgatgcgccgattgtacttctccacgggtttaccggctggga
 A  A  S  R  A  N  D  A  P  I  V  L  L  H  G  F  T  G  W  G
agagaagaaatgtttgggttcaagtactggggcggcgtgcgcggcgatatcgaacaatgg
 R  E  E  M  F  G  F  K  Y  W  G  G  V  R  G  D  I  E  Q  W
ctgaacgacaacggttatcgaacttatacgctggcggtcggcccgctctcgagcaactgg
 L  N  D  N  G  Y  R  T  Y  T  L  A  V  G  P  L  S  S  N  W
gatcgggcgtgcgaagcgtatgcccagcttgtcggcgggacggtcgattatggggcagcc
 D  R  A  C  E  A  Y  A  Q  L  V  G  G  T  V  D  Y  G  A  A
catgcggcaaagcacggccatgcgcggtttggccgcacctatcccggcctgttgccggaa
 H  A  A  K  H  G  H  A  R  F  G  R  T  Y  P  G  L  L  P  E
ttgaaaaggggcggtcgcatccatatcatcgcccacagccaaggagggcagacggcccgc
 L  K  R  G  G  R  I  H  I  I  A  H  S  Q  G  G  Q  T  A  R
atgcttgtatcgctcctagagaatggaagccaagaagagcgggagtacgctaaggcgcat
 M  L  V  S  L  L  E  N  G  S  Q  E  E  R  E  Y  A  K  A  H
aacgtgtctttgtcgccgttgtttgaaggcggacatcattttgtgttgagagtgacaacc
 N  V  S  L  S  P  L  F  E  G  G  H  H  F  V  L  R  V  T  T
attgccacccctcatgacgggacgacgcttgtcaacatggttgatttcaccgatcgcttt
 I  A  T  P  H  D  G  T  T  L  V  N  M  V  D  F  T  D  R  F
tttgacttgcaaaaagcggtgttgaaagcagcggctgtcgccagcaatgtgccgtacaca
 F  D  L  Q  K  A  V  L  K  A  A  A  V  A  S  N  V  P  Y  T
agtcaagtatacgattttaagctcgaccaatggggactgcgccgccagccaggtgaatcg
 S  Q  V  Y  D  F  K  L  D  Q  W  G  L  R  R  Q  P  G  E  S
tttgaccaatattttgaacggctcaaacggtcccctgtttggacgtcgacagataccgct
```

```
 F   D   Q   Y   F   E   R   L   K   R   S   P   V   W   T   S   T   D   T   A
cgctatgatttatccgttcccggggctgagaagttgaatcaatgggtgaaagcaagcccg
 R   Y   D   L   S   V   P   G   A   E   K   L   N   Q   W   V   K   A   S   P
aatacgtattatttgagctttgccacagaacggacgtatcgcggagcgctcacaggcaac
 N   T   Y   Y   L   S   F   A   T   E   R   T   Y   R   G   A   L   T   G   N
tattatcccgaactcggaatgaatgcattcagcgccgtcgtatgcgccccgtttctcggt
 Y   Y   P   E   L   G   M   N   A   F   S   A   V   V   C   A   P   F   L   G
tcgtaccgcaatgcgacgctcggcattgacgaccgctggcttgagaacgatggcatcgtc
 S   Y   R   N   A   T   L   G   I   D   D   R   W   L   E   N   D   G   I   V
aatacgttttcgatgaacggaccaaagcgcggatcgaccgatcggatcgtgccgtatgac
 N   T   F   S   M   N   G   P   K   R   G   S   T   D   R   I   V   P   Y   D
gggacaataaaaaaaggggtttggaatgatatgggaacgtacaatgtcgatcatttggaa
 G   T   I   K   K   G   V   W   N   D   M   G   T   Y   N   V   D   H   L   E
gtcatcggcgttgacccgaatccgttgtttgatatccgtgcctttatttgcgccttgcc
 V   I   G   V   D   P   N   P   L   F   D   I   R   A   F   Y   L   R   L   A
gagcagttggcgagcttgcaaccttaaaactagtattttgcgaaaaaagccatctcgatc
 E   Q   L   A   S   L   Q   P Stop                         Stem loop
ggatggcgcttttttttcgtgaacattcatgcgccttggttggtcattttcatgcccgggt

ggagaacaaacgctgttcatgaacttttctgtgaatatgcacgttcctttaactttcagt

ttcatgtcggggtgaaaaacgaaaaatggtttatggatgttttggagaacaagcagctga

ttggtatgacggccgcggaatcccga
```

FIGURE 14

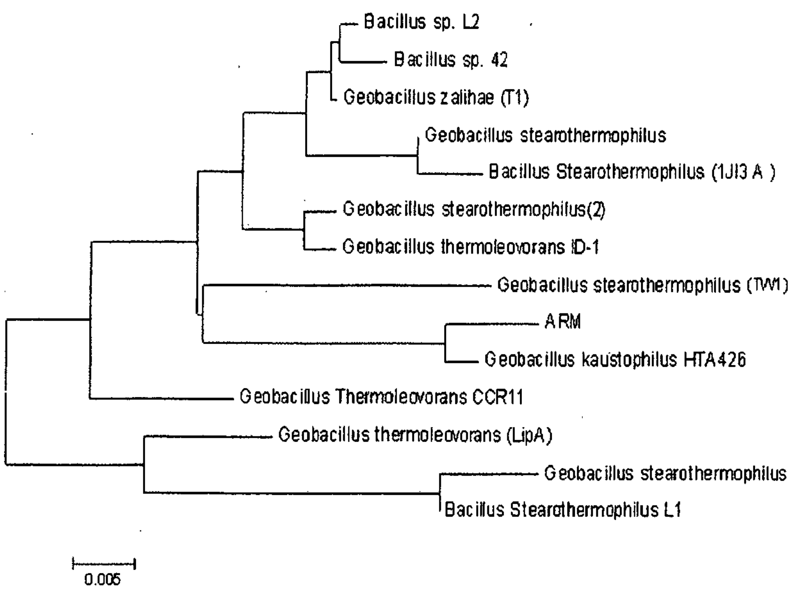


FIGURE 15

| | |
|---|---|
| ARM | AASRANDAPIVLLHGFTGWGREEMFGFKYWGGVRGDIEQWLNDNGYRTYTLAVGPLSSNW |
| HTA426 | AASRANDAPIVLLHGFTGWGREEMFGFKYWGGVRGDIEQWLNDNGYRTYTLAVGPLSSNW |
| TW1 | A**T**SRANDAPIVLLHGFTGWGREEMFGFKYWGGVRGDIEQWLNDNGYRTYTLAVGPLSSNW |
| T1 | A**SL**RANDAPIVLLHGFTGWGREEMFGFKYWGGVRGDIEQWLNDNGYRTYTLAVGPLSSNW |

| | |
|---|---|
| ARM | DRACEAYAQLVGGTVDYGAAHAAKHGHARFGRTYPGLLPELKRGGRIHIIAHSQGGQTAR |
| HTA426 | DRACEAYAQLVGGTVDYGAAHAAKHGHARFGRTYPGLLPELKRGGRIHIIAHSQGGQTAR |
| TW1 | DRACEAYAQLVGGTVDYGAAHAAKHGHARFGRTYPGLLPELKRGGRIHIIAHSQGGQTAR |
| T1 | DRACEAYAQLVGGTVDYGAAHAAKHGHARFGRTYPGLLPELKRGGRIHIIAHSQGGQTAR |

| | |
|---|---|
| ARM | MLVSLLENGSQEEREYAKAHNVSLSPLFEGGHHFVLRVTTIATPHDGTTLVNMVDFTDRF |
| HTA426 | MLVSLLENGSQEEREYAKAHNVSLSPLFEGGHHFVL**S**VTTIATPHDGTTLVNMVDFTDRF |
| TW1 | MLVSLLENGSQEEREYAKAHNVSLSPLFEGGHHFVL**S**VTTIATPHDGTTLVNMVDFTDRF |
| T1 | MLVSLLENGSQEEREYAKAHNVSLSPLFEGGHHFVL**S**VTTIATPHDGTTLVNMVDFTDRF |

| | |
|---|---|
| ARM | FDLQKAVLKAAAVASNVPYTSQVYDFKLDQWGLRRQPGESFDQYFERLKRSPVWTSTDTA |
| HTA426 | FDLQKAVL**E**AAAVASNVPYTSQVYDFKLDQWGLRRQPGESFD**H**YFERLKRSPVWTSTDTA |
| TW1 | FDLQKAVL**E**AAAVASNVPYTSQVYDFKLDQWGLRRQPGESFD**H**YFERLKRSPVWTSTDTA |
| T1 | FDLQKAVL**E**AAAVASNVPYTSQVYDFKLDQWGLRRQPGESFD**H**YFERLKRSPVWTSTDTA |

| | |
|---|---|
| ARM | RYDLSVPGAEKLNQWVKASPNTYYLSFATERTYRGALTGNYYPELGMNAFSAVVCAPFLG |
| HTA426 | RYDLSVPGAEKLNQWVKASPNTYYLSFATERTYRGALTGNYYPELGMNAFSAVVCAPFLG |
| TW1 | RYDLSV**S**GAEKLNQWV**Q**ASPNTYYLSFATERTYRGALTGNYYPELGMNAFSAVVCAPFLG |
| T1 | RYDLSV**S**GAEKLNQWV**Q**ASPNTYYLSF**S**TERTYRGALTGN**H**YPELGMNAFSAVVCAPFLG |

| | |
|---|---|
| ARM | SYRNATLGIDDRWLENDGIVNTFSMNGPKRGSTDRIVPYDGTIKKGVWNDMGTYNVDHLE |
| HTA426 | SYRNATLGIDDRWLENDGIVNTFSMNGPKRGSTDRIVPYDGTIKKGVWN**Y**MGTYNVDHLE |
| TW1 | SYRN**P**TLGIDDRWLENDGIVNT**V**SMNGPKRGS**S**DRIVPYDG**AL**KKGVWNDMGTYNVDHLE |
| T1 | SYRN**P**TLGIDDRWLENDGIVNT**V**SMNGPKRGS**S**DRIVPYDGT**L**KKGVWNDMGTYNVDHLE |

| | |
|---|---|
| ARM | VIGVDPNPLFDIRAFYLRLAEQLASLQP |
| HTA426 | VIGVDPNPLFDIRAFYLRLAEQLASLQP |
| TW1 | **I**IGVDPNP**S**FDIRAFYLRLAEQLASLQP |
| T1 | **I**IGVDPNP**S**FDIRAFYLRLAEQLASLQP |

**FIGURE 16**

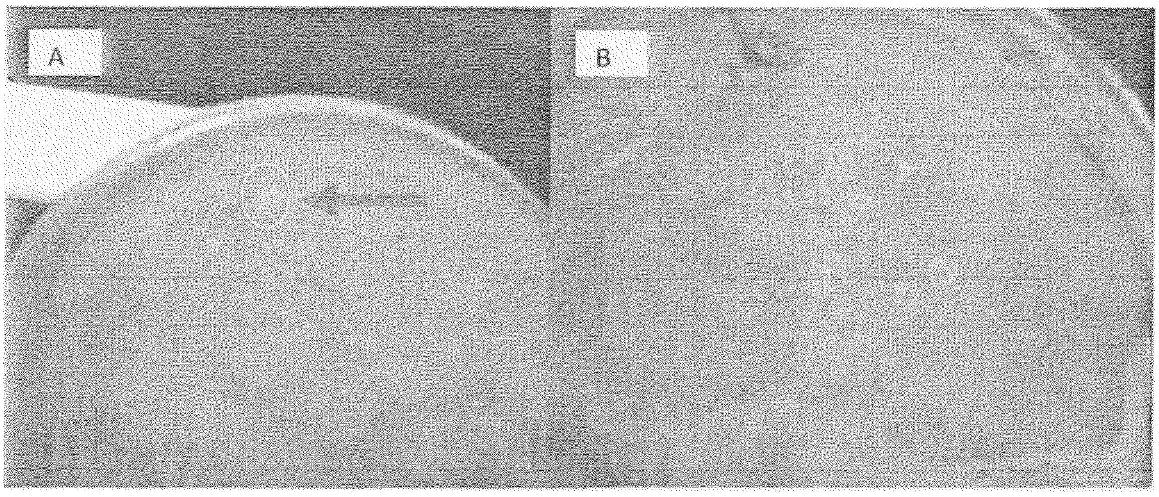


FIGURE 17

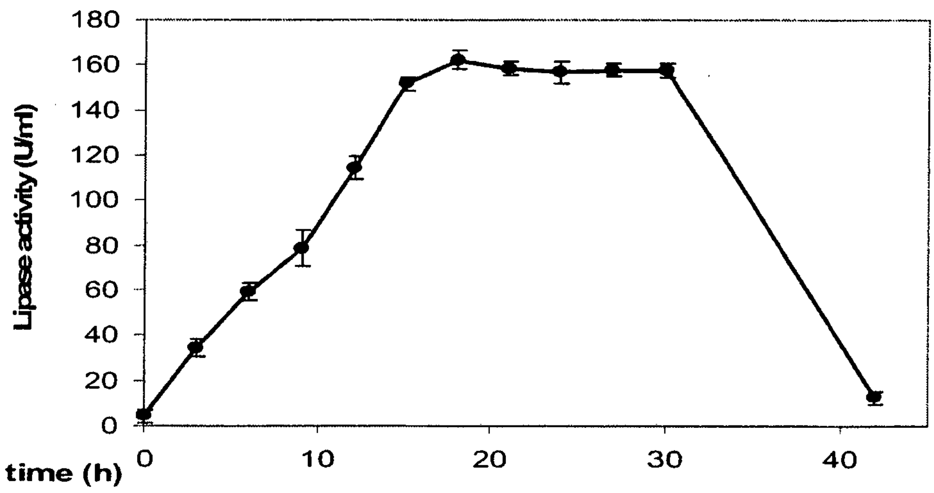


FIGURE 18

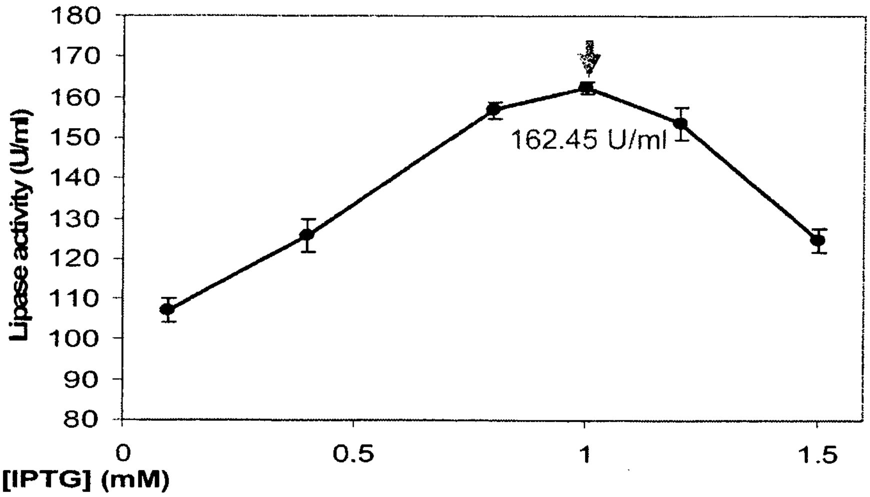


FIGURE 19

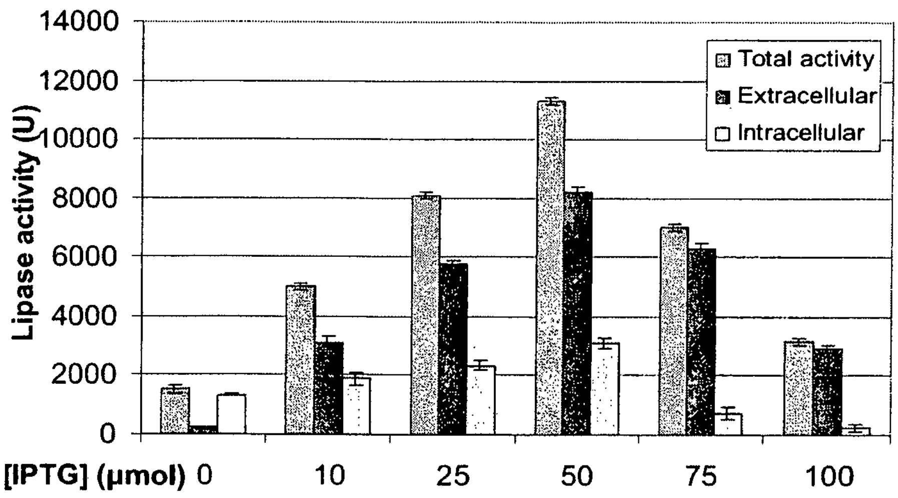


FIGURE 20

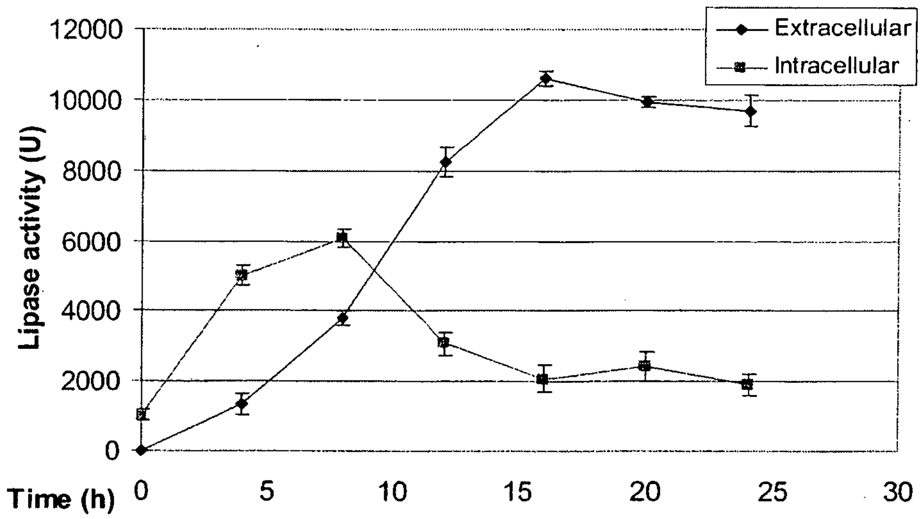


FIGURE 21

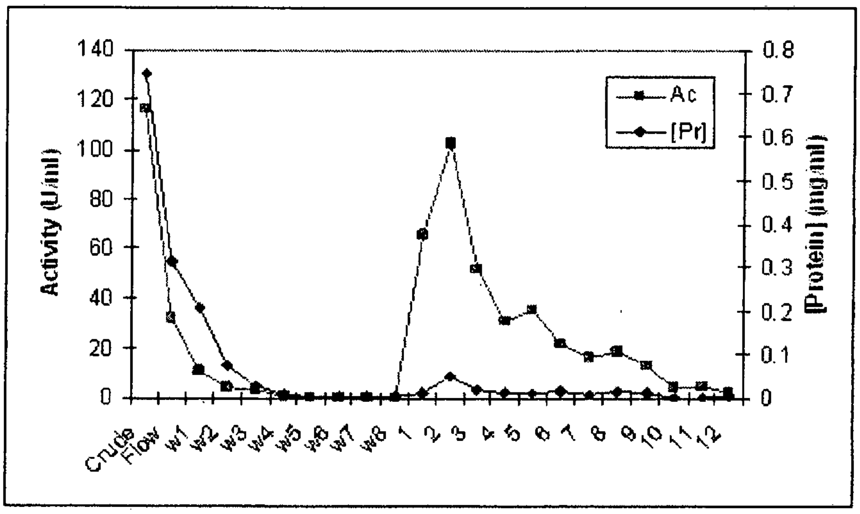


FIGURE 22

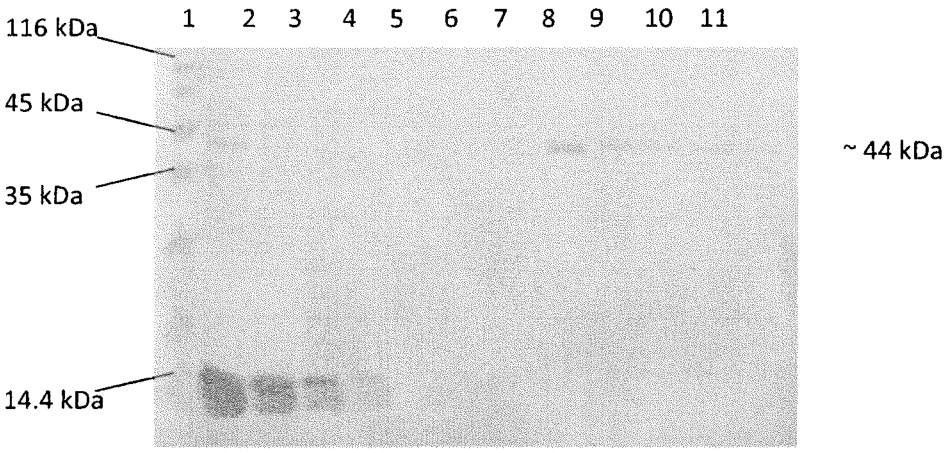


FIGURE 23

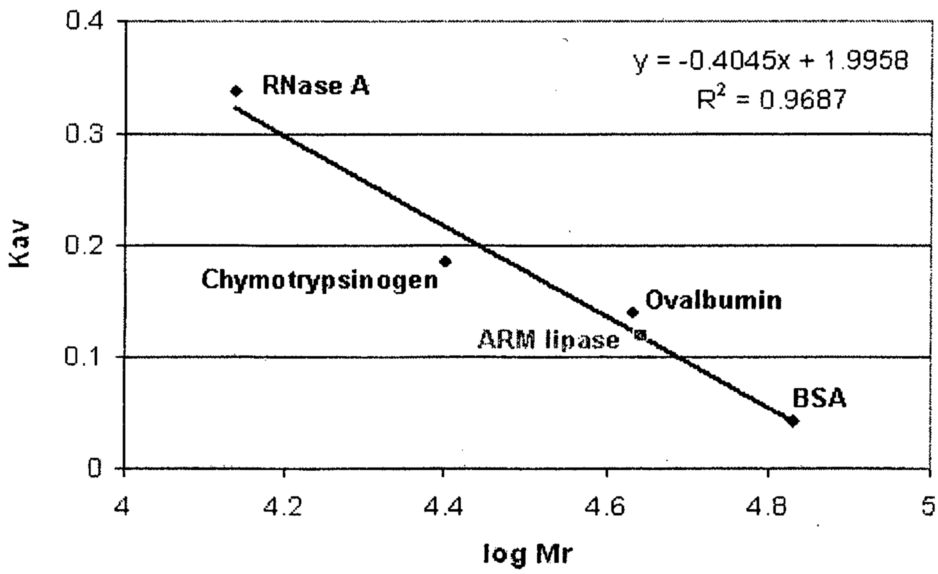


FIGURE 24

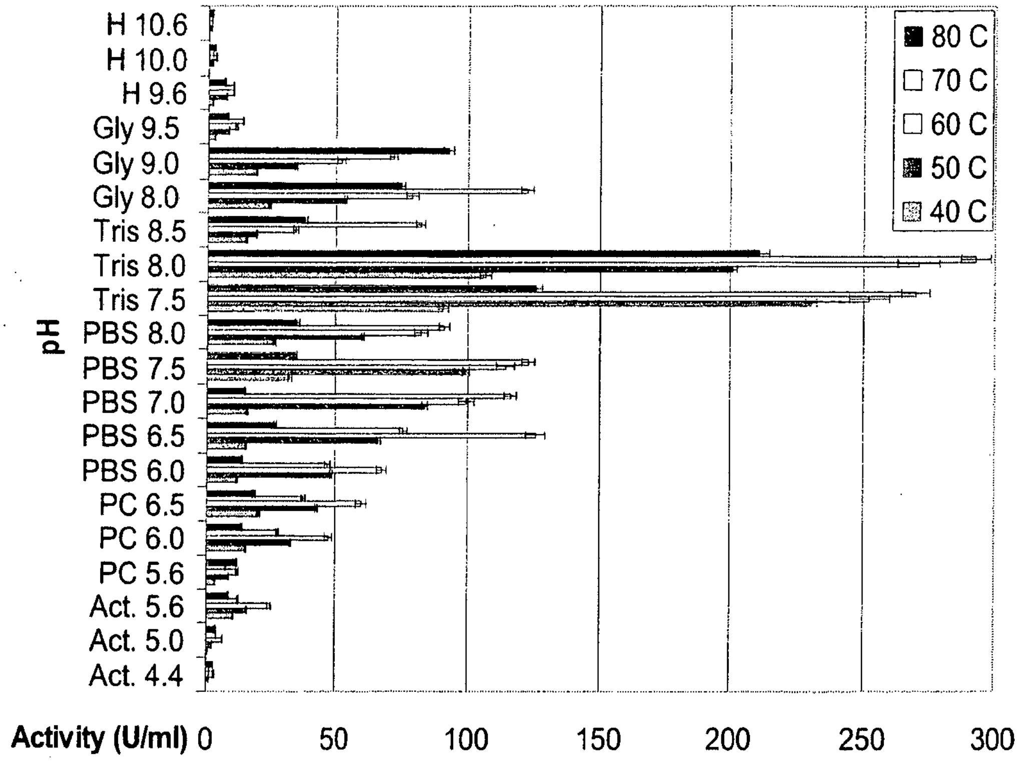
H 10.6
H 10.0
H 9.6
Gly 9.5
Gly 9.0
Gly 8.0
Tris 8.5
Tris 8.0
Tris 7.5
PBS 8.0
PBS 7.5
PBS 7.0
PBS 6.5
PBS 6.0
PC 6.5
PC 6.0
PC 5.6
Act. 5.6
Act. 5.0
Act. 4.4
pH
80 C
70 C
60 C
50 C
40 C
Activity (U/ml)
0
50
100
150
200
250
300

FIGURE 25

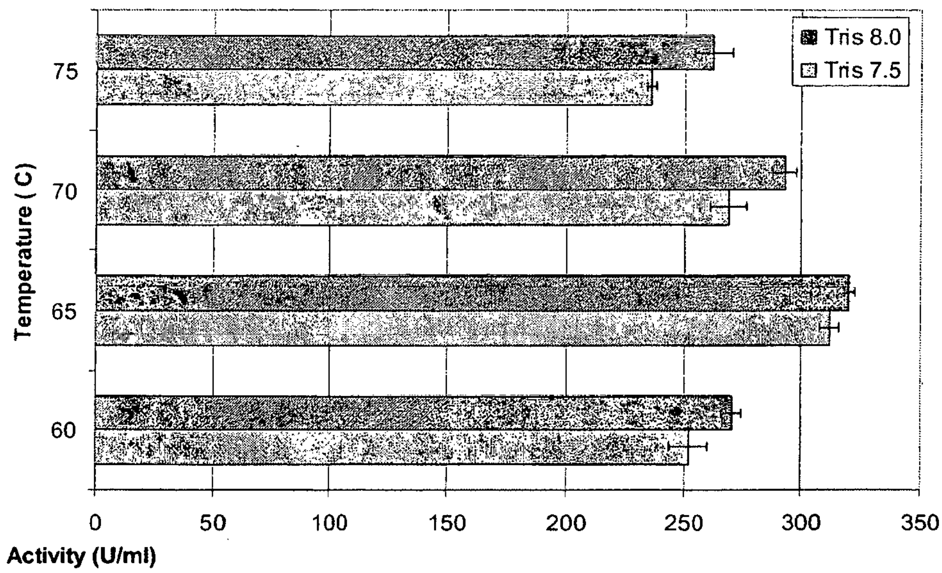


FIGURE 26

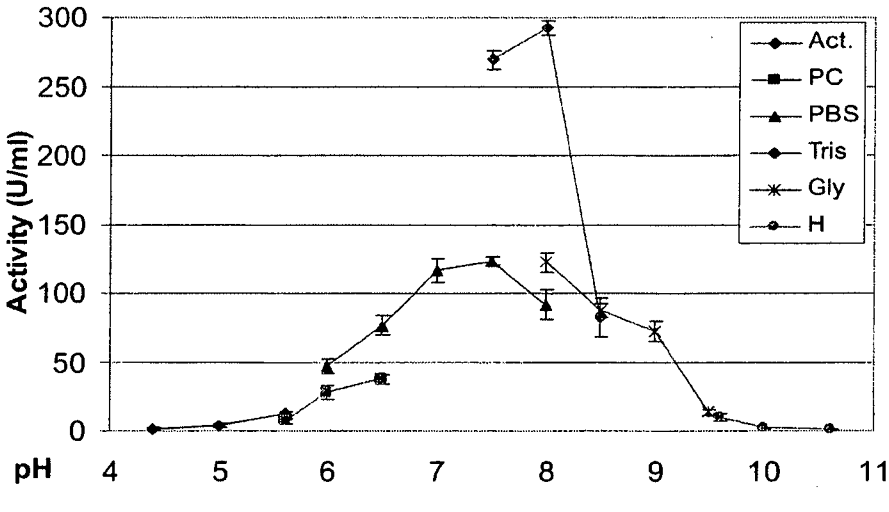


FIGURE 27

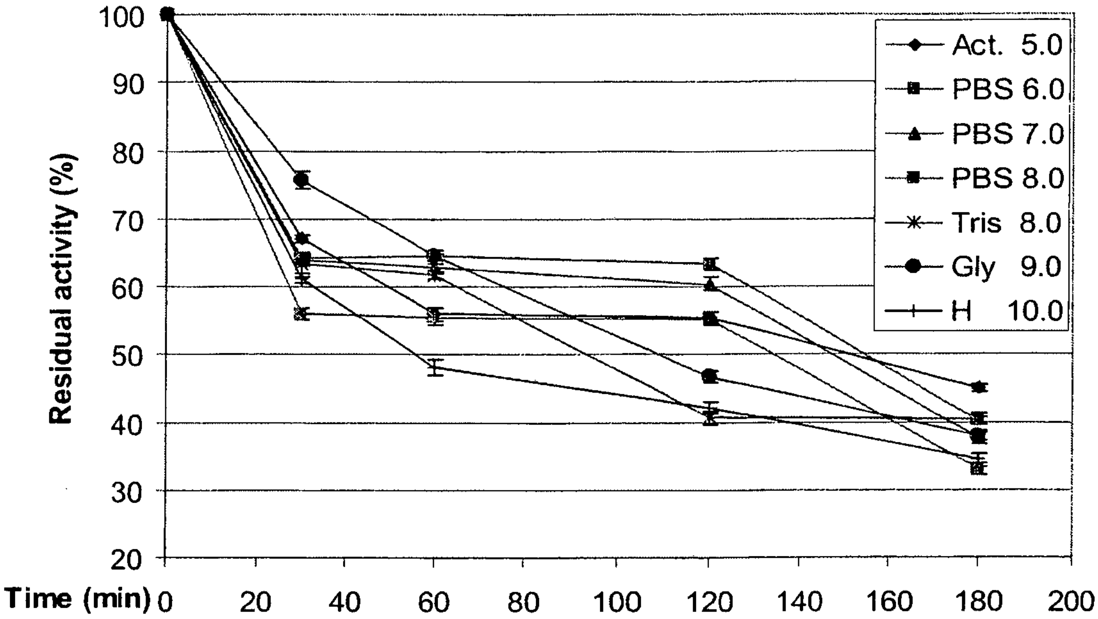


FIGURE 28

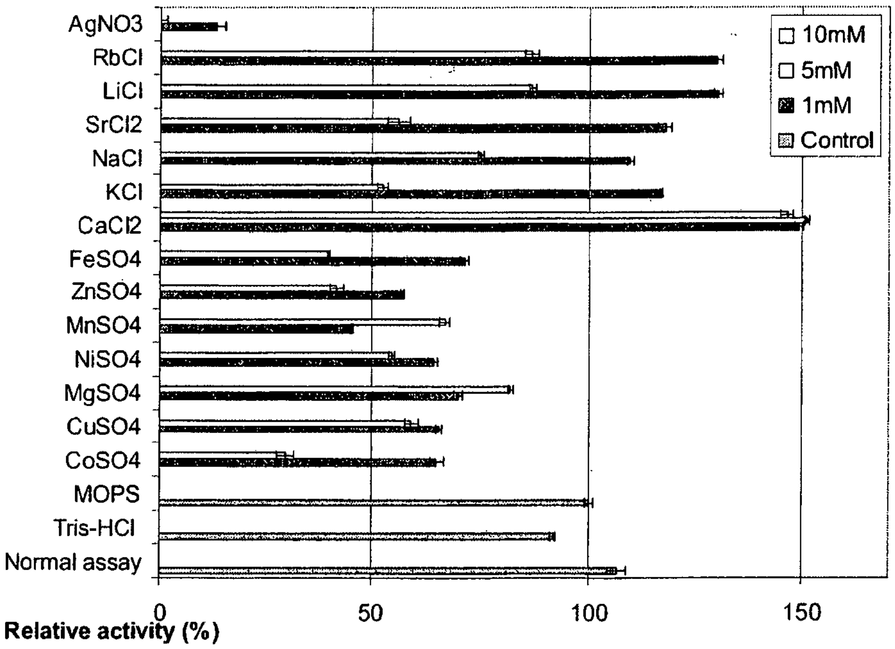


FIGURE 29

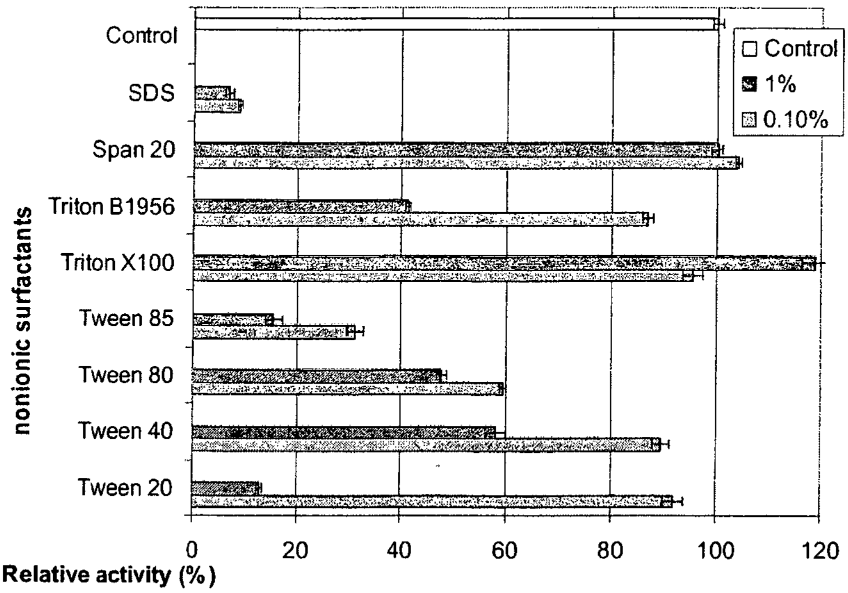


FIGURE 30

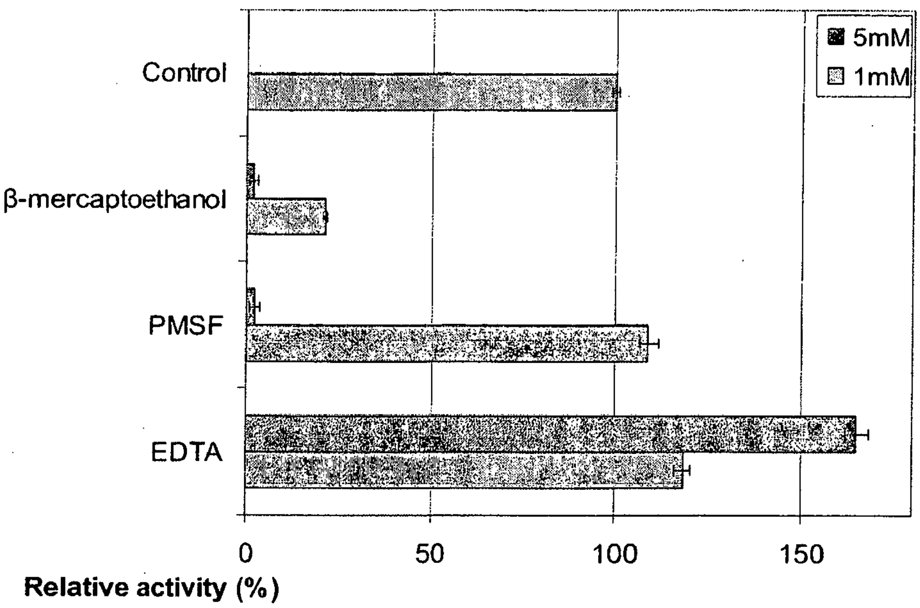


FIGURE 31

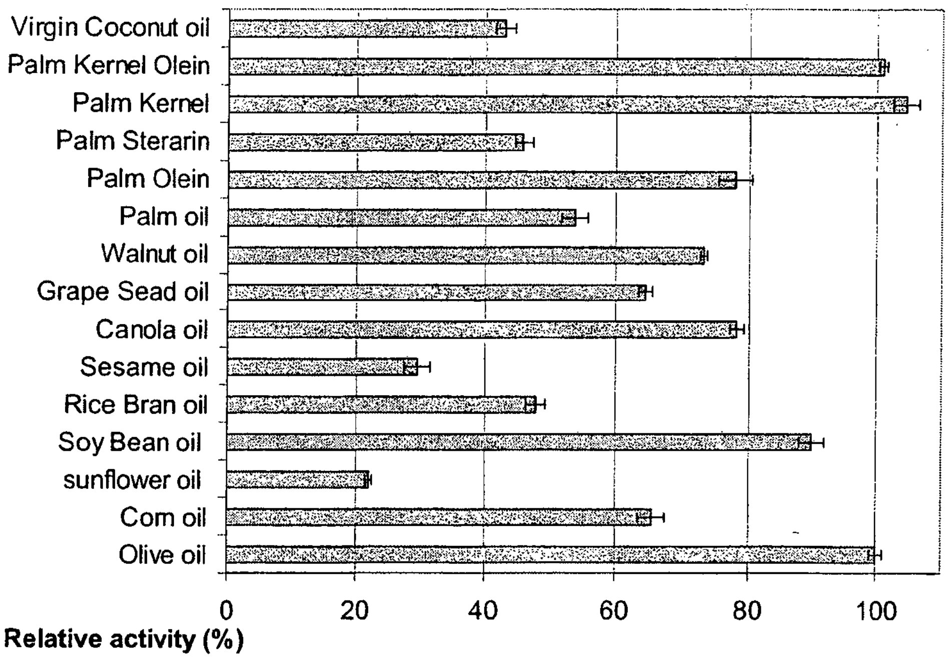
Virgin Coconut oil
Palm Kernel Olein
Palm Kernel
Palm Sterarin
Palm Olein
Palm oil
Walnut oil
Grape Sead oil
Canola oil
Sesame oil
Rice Bran oil
Soy Bean oil
sunflower oil
Corn oil
Olive oil
0
20
40
60
80
100
Relative activity (%)

FIGURE 32

A

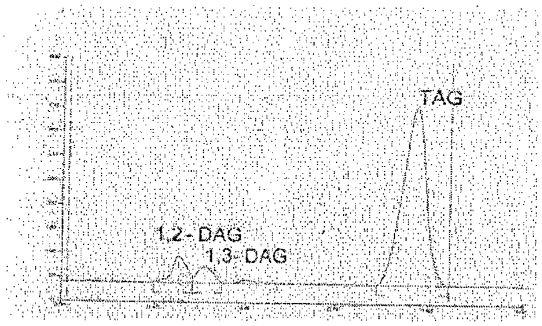


B

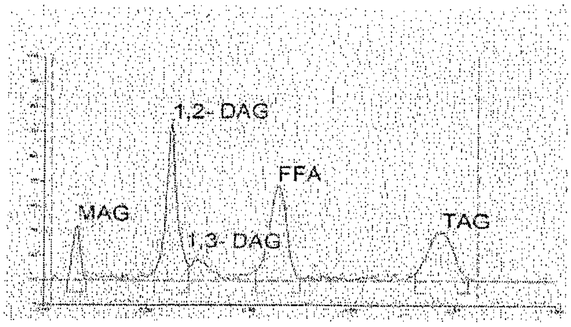


A

| Peak | Start Rf | Start Height | Max Rf | Max Height | Max % | End Rf | End Height | Area | Area % | Assigned substance |
|---|---|---|---|---|---|---|---|---|---|---|
| 1 | 0.199 | 1.9 | 0.251 | 55.2 | 12.14 | 0.283 | 15.3 | 1484.0 | 8.48 | 1,2- DAG |
| 2 | 0.283 | 15.3 | 0.307 | 34.8 | 7.66 | 0.347 | 2.0 | 1007.3 | 5.75 | 1,3- DAG |
| 3 | 0.690 | 0.1 | 0.773 | 364.4 | 80.19 | 0.843 | 0.41 | 15015.5 | 85.77 | TAG |

B

| Peak | Start Rf | Start Height | Max Rf | Max Height | Max % | End Rf | End Height | Area | Area % | Assigned substance |
|---|---|---|---|---|---|---|---|---|---|---|
| 1 | 0.047 | 0.5 | 0.064 | 22.4 | 15.54 | 0.083 | 0.3 | 267.7 | 8.05 | MAG |
| 2 | 0.216 | 4.1 | 0.249 | 63.7 | 44.13 | 0.283 | 5.8 | 1148.2 | 34.53 | 1,2- DAG |
| 3 | 0.413 | 4.5 | 0.456 | 38.6 | 26.72 | 0.500 | 0.7 | 1098.2 | 33.03 | FFA |
| 4 | 0.726 | 3.1 | 0.776 | 19.6 | 13.61 | 0.827 | 0.5 | 810.7 | 24.38 | TAG |

**FIGURE 33**

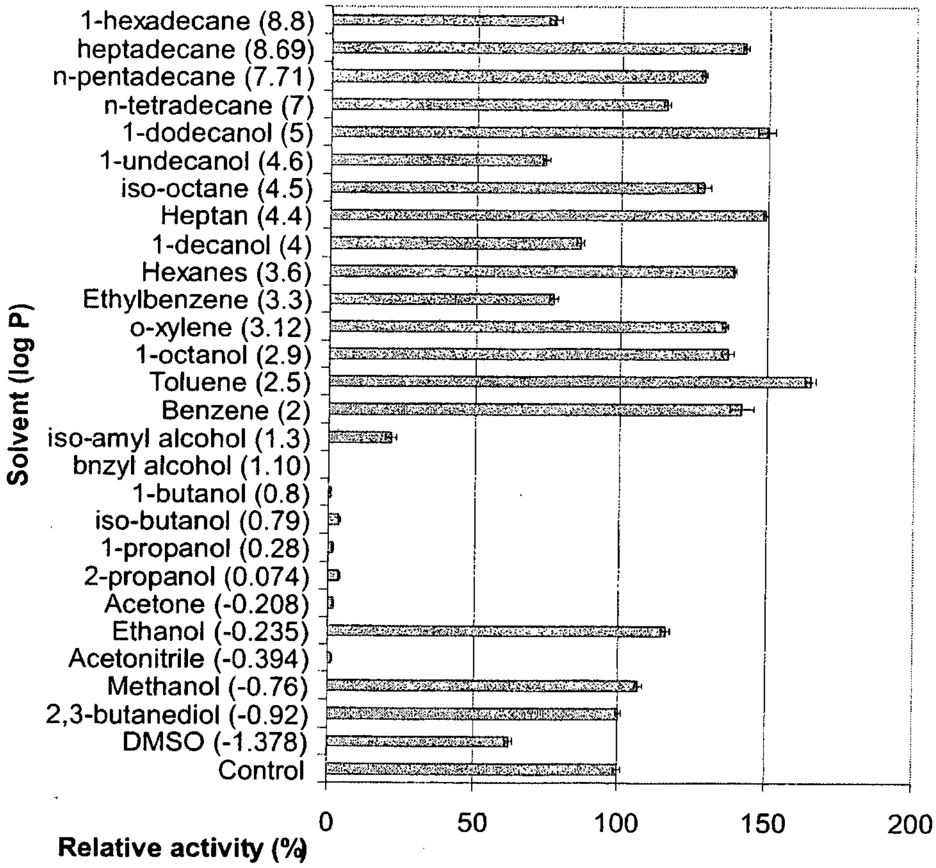
1-hexadecane (8.8)
heptadecane (8.69)
n-pentadecane (7.71)
n-tetradecane (7)
1-dodecanol (5)
1-undecanol (4.6)
iso-octane (4.5)
Heptan (4.4)
1-decanol (4)
Hexanes (3.6)
Ethylbenzene (3.3)
o-xylene (3.12)
1-octanol (2.9)
Toluene (2.5)
Benzene (2)
iso-amyl alcohol (1.3)
bnzyl alcohol (1.10)
1-butanol (0.8)
iso-butanol (0.79)
1-propanol (0.28)
2-propanol (0.074)
Acetone (-0.208)
Ethanol (-0.235)
Acetonitrile (-0.394)
Methanol (-0.76)
2,3-butanediol (-0.92)
DMSO (-1.378)
Control
Solvent (log P)
0
50
100
150
200
Relative activity (%)

FIGURE 34

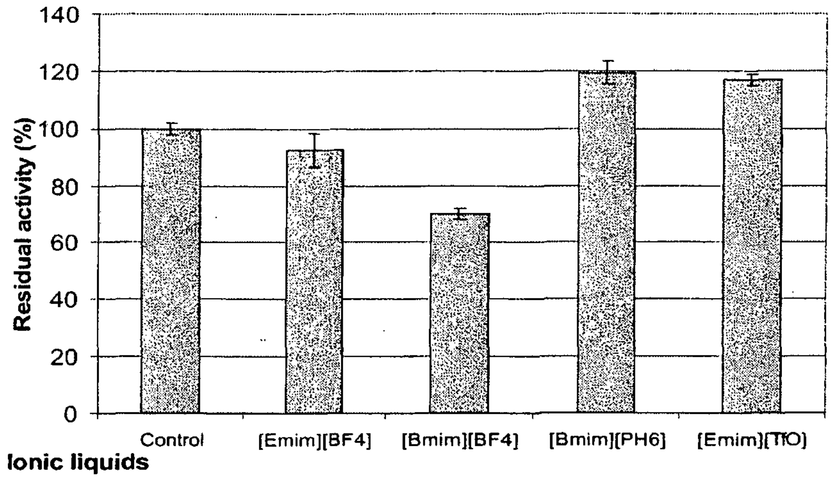


FIGURE 35

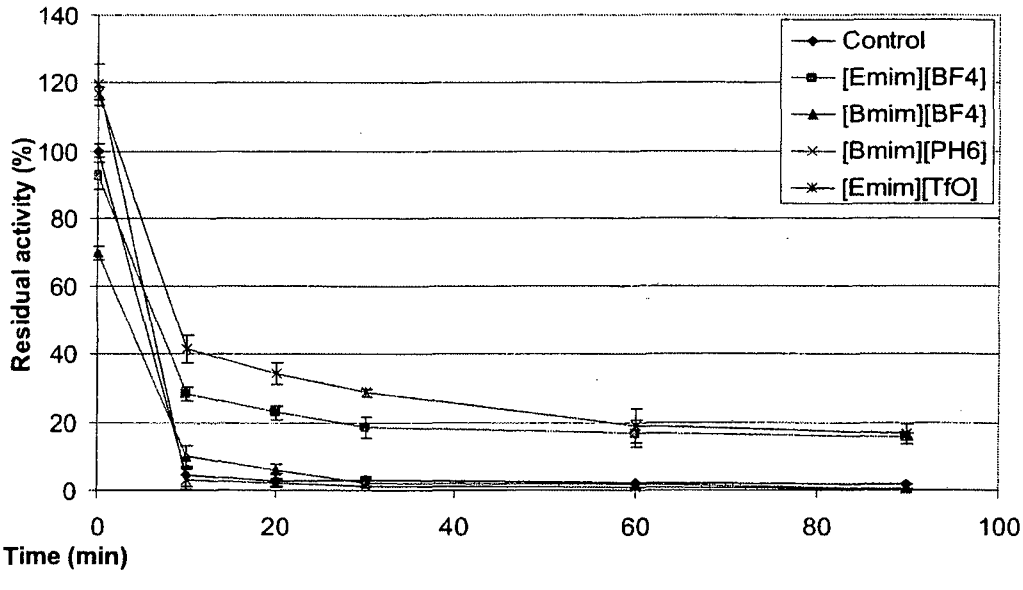


FIGURE 36

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- MY 2010700066